# EUROPÄISCHE PATENTSCHRIFT

(11) **EP 0 609 734 B1**
(45) Veröffentlichungstag und Bekanntmachung des Hinweises auf die Patenterteilung: **27.08.1997**
(21) Anmeldenummer: 94100938.3
(22) Anmeldetag: 24.01.1994
(51) Int. Cl.: C07D 249/12, C07D 417/12, C07D 413/12, C07D 409/12, A01N 43/653, C07C 255/50, C07C 311/00, C07C 307/10, C07C 307/02

(54) **Substituierte Triazolinone sowie ihre Verwendung als Herbizide**
Substituted triazolinones and their use as herbicides
Triazolinones substituées et leur utilisation comme herbicides

(30) Priorität: 05.02.1993 DE 4303376
(43) Veröffentlichungstag der Anmeldung: 10.08.1994
(73) Patentinhaber: BAYER AG, 51368 Leverkusen (DE)
(72) Erfinder: Haas, Wilhelm, Dr., D-50259 Pulheim (DE); Linker, Karl-Heinz, D-51377 Leverkusen (DE); Schallner, Otto, Dr., D-40789 Monheim (DE); Findeisen, Kurt, Prof. Dr., D-51375 Leverkusen (DE); Santel, Hans-Joachim, Dr., D-51371 Leverkusen (DE); Dollinger, Markus, Dr., D-42799 Leichlingen (DE)

(56) Entgegenhaltungen:
- EP-A- 0 011 693
- EP-A- 0 370 332
- EP-A- 0 558 999
- EP-A- 0 597 360
- WO-A-87/03782
- GB-A- 2 230 261
- CHEMICAL ABSTRACTS, vol. 118, no. 17, 26. April 1993, Columbus, Ohio, US; abstract no. 168823z, Seite 850 ;

## Beschreibung

Die Erfindung betrifft neue substituierte Triazolinone, mehrere Verfahren zu ihrer Herstellung und ihre Verwendung als Herbizide.

Es ist bekannt, daß bestimmte substituierte Triazolinone wie beispielsweise die Verbindung 3 -Methyl-4-propargyl-1-(2,5-difluor-4-cyano-phenyl)-1,2,4-triazolin-5-on herbizide Eigenschaften besitzen (vergl. z.B. DE 38 39 480).

GB 2 230 261 und WO 87/03782 offenbaren meta-Sulfonylaminophenyltriazolinone, sowie deren Verwendung als Herbizide.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden nun neue substituierte Triazolinone der allgemeinen Formel (I), 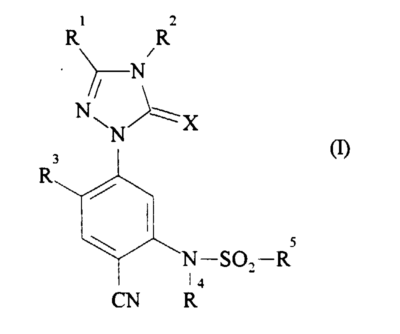 in welcher
- R¹: für Wasserstoff, Halogen, Cyano, Hydroxy oder für einen der Reste -R⁶, -O-R⁶, -O-NR⁶R⁷, -S-R⁶, -S(O)-R⁶ oder -SO₂-R⁶ steht,
- R²: für Wasserstoff, Hydroxy, Amino, Cyano oder für einen der Reste -R⁶, -O-R⁶ oder -N=CR⁶R⁷ steht,
- R³: für Wasserstoff, Halogen, Alkyl oder Halogenalkyl steht,
- R⁴: für Wasserstoff, für einen der Reste -R⁶, -O-R⁶ oder -SO₂-R⁶ oder für ein anorganisches oder organisches Kation steht und
- R⁵: für Amino, Hydroxy oder für einen der Reste -R⁶ oder -NR⁶R⁷ steht, oder
- R⁴ und R⁵: gemeinsam für einen zweifach verknüpften Alkandiylrest stehen und
- X: für Sauerstoff oder Schwefel steht, wobei
- R⁶: für jeweils gegebenenfalls substituiertes Alkyl, Alkenyl, Alkinyl, Cycloalkyl, Aryl oder Heterocyclyl steht und
- R⁷: für Wasserstoff oder für jeweils gegebenenfalls substituiertes Alkyl, Alkenyl, Alkinyl, Cycloalkyl oder Aryl steht,
gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I), 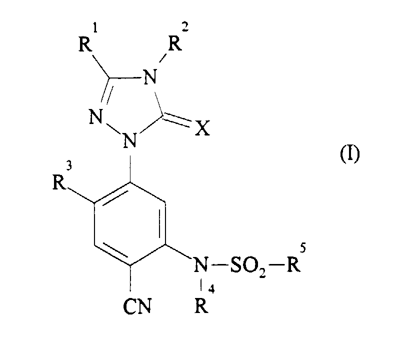 in welcher
R¹, R², R³, R⁴, R⁵ und X die oben angegebenen Bedeutungen haben, erhält, wenn man
a) 1H-Triazolinone der Formel (II), 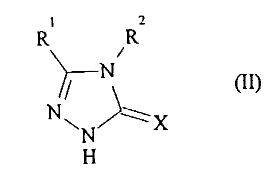 in welcher
   R¹, R² und X die oben angegebenen Bedeutungen haben,
   mit Halogenbenzol-Derivaten der Formel (III), 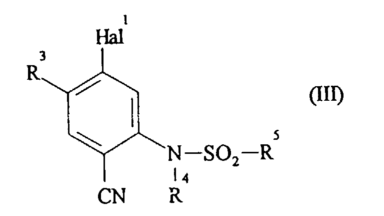 in welcher
   R³, R⁴ und R⁵ die oben angegebenen Bedeutungen haben und
   Hal¹ für Halogen, insbesondere Fluor, Chlor, Brom und Iod steht,
   gegebenenfalls in Gegenwart eines Verdünnungsmittels und gegebebenfalls in Gegenwart eines Reaktionshilfsmittels umsetzt, oder wenn man
b) substituierte Triazolinone der Formel (IV), 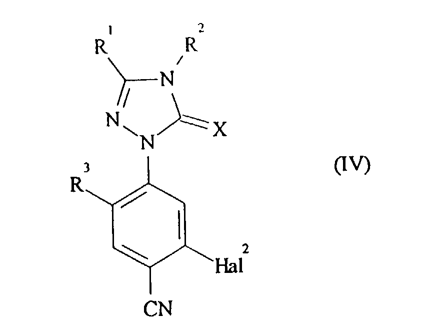 in welcher
   R¹, R², R³ und X die oben angegebenen Bedeutungen haben und
   Hal² für Halogen steht,
   mit Sulfonamiden der Formel (V),

   R⁴―NH―SO₂―R⁵ (V)

   in welcher
   R⁴ und R⁵ die oben angegebenen Bedeutungen haben,
   gegebenenfalls in Gegenwart eines Verdünnungsmittels und gegebenenfalls in Gegenwart eines Reaktionshilfsmittels umsetzt, oder wenn man
c) substituierte Triazolinone der Formel (Ia), 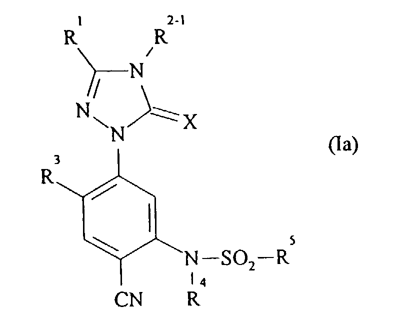 in welcher
   R¹, R³, R⁴, R⁵ und X die oben angegebenen Bedeutungen haben und
   R²⁻¹ für Amino steht,
   mit Natriumnitrit in Gegenwart einer Säure und gegebenenfalls in Gegenwart eines Verdünnungsmittels umsetzt, oder wenn man
d) substituierte Triazolinone der Formel (Ib), 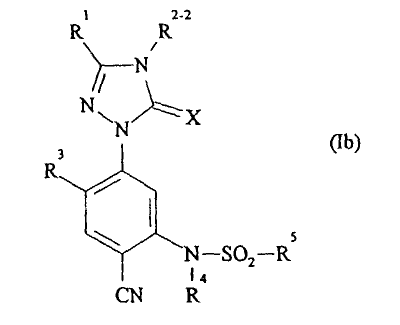 in welcher
   R¹, R³, R⁴, R⁵ und X die oben angegebenen Bedeutungen haben und
   R²⁻² für Wasserstoff steht,
   mit Alkylierungsmitteln der Formel (VI),

   R²⁻³-E¹ (VI)

   in welcher
   - R²⁻³: für jeweils gegebenenfalls substituiertes Alkyl, Alkenyl, Alkinyl oder Cycloalkyl steht und
   - E¹: für eine elektronenanziehende Abgangsgruppe steht,
   gegebenenfalls in Gegenwart eines Verdünnungsmittels und gegebenenfalls in Gegenwart eines Reaktionshilfsmittels umsetzt, oder wenn man
e) substituierte Triazolinone der Formel (Ic), 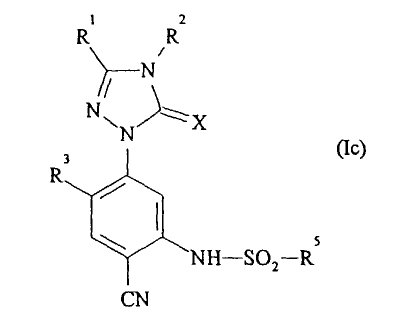 in welcher
   R¹, R², R³, R⁵ und X die oben angegebenen Bedeutungen haben,
   mit Alkylierungsmitteln der Formel (VII),

   R⁴⁻¹-E² (VII)

   in welcher
   - R⁴⁻¹: für Alkyl, Alkenyl, Alkinyl, Halogenalkyl, Halogenalkenyl, Halogenalkinyl, Alkoxyalkyl oder für gegebenenfalls substituiertes Cycloalkyl steht und
   - E²: für eine elektronenanziehende Abgangsgruppe steht,
   gegebenenfalls in Gegenwart eines Verdünnungsmittels und gegebenenfalls in Gegenwart eines Reaktionshilfsmittels umsetzt, oder wenn man
f) substituierte Triazolinone der Formel (VIII), 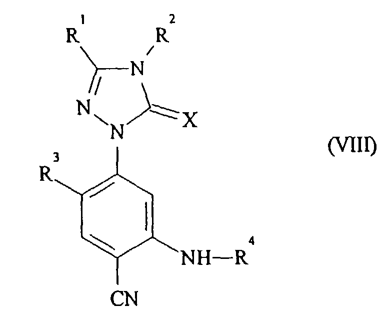 in welcher
   R¹, R², R³, R⁴ und X die oben angegebenen Bedeutungen haben,
   mit Sulfonsäurehalogeniden der Formel (IX),

   R⁵-SO₂-Hal³ (IX)

   in welcher
   R⁵ die oben angegebene Bedeutung hat und
   Hal³ für Halogen steht,
   gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich bessere herbizide Wirksamkeit gegenüber Problemunkräutern und gleichzeitig eine verbesserte Verträglichkeit gegenüber Nutzpflanzen im Vergleich zu den aus dem Stand der Technik bekannten substituierten Triazolinonen, wie beispielsweise die Verbindung 3-Methyl-4-propargyl-1-(2,5-difluor-4-cyano-phenyl)-1,2,4-triazolin-5-on, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen
- R¹: für Wasserstoff, Fluor, Chlor, Brom, Iod, Cyano, Hydroxy oder für einen der Reste -R⁶, -O-R⁶, -O-NR⁶R⁷, -S-R⁶, -S(O)-R⁶ oder -SO₂-R⁶ steht,
- R²: für Wasserstoff Hydroxy, Amino, Cyano oder für einen der Reste -R⁶, -O-R⁶ oder -N=CR⁶R⁷ steht,
- R³: für Wasserstoff Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen - insbesondere Fluor, Chlor, Brom oder Iod - steht,
- R⁴: für Wasserstof für einen der Reste -R⁶, -O-R⁶ oder -SO₂-R⁶, für ein Äquivalent eines Alkali- oder Erdalkalimetallkations oder für ein gegebenenfalls einfach oder mehrfach, gleich oder verschieden durch Alkyl mit 1 bis 16 Kohlenstoffatomen substituiertes Ammoniumkation steht und
- R⁵: für Amino, Hydroxy oder für einen der Reste -R⁶ oder -NR⁶R⁷ steht, oder
- R⁴ und R⁵: gemeinsam für einen zweifach verknüpften Alkandiylrest mit 2 bis 7 Kohlenstoffatomen stehen und
- X: für Sauerstoff oder Schwefel steht, wobei
- R⁶: für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder lod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
- R⁶: außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
- R⁶: außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
- R⁶: außerdem für jeweils gegebenenfalls im Arylteil einfach oder mehrfach gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen und/oder methylsubstituiertes 1,2,4-oxadiazol-2-yl substituiertes Phenyl;
- R⁷: für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
- R⁷: außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
- R⁷: außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht, oder
- R⁷: für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl.

Besonders bevorzugt sind Verbindungen der Formel (I), bei welchen
- R¹: für Wasserstoff, Fluor, Chlor, Brom, Cyano, Hydroxy oder für einen der Reste -R⁶, -O-R⁶, -O-NR⁶R⁷, -S-R⁶, -S(O)-R⁶ oder -SO₂-R⁶ steht,
- R²: für Wasserstoff, Hydroxy, Amino, Cyano oder für einen der Reste -R⁶, -O-R⁶ oder -N=CR⁶R⁷ steht,
- R³: für Wasserstoff Fluor, Chlor, Brom, für geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen - insbesondere Fluor, Chlor oder Brom - steht,
- R⁴: für Wasserstoff, für einen der Reste -R⁶, -O-R⁶ oder -SO₂-R⁶, für ein Äquivalent eines Alkali- oder Erdalkalimetallkations oder für ein gegebenenfalls einfach bis vierfach, gleich oder verschieden durch Alkyl mit 1 bis 12 Kohlenstoffatomen substituiertes Ammoniumkation steht und
- R⁵: für Amino, Hydroxy oder für einen der Reste -R⁶ oder -NR⁶R⁷ steht, oder
- R⁴ und R⁵: gemeinsam für einen zweifach verknüpften Alkandiylrest mit 2 bis 6 Kohlenstoffatomen stehen und
- X: für Sauerstoff oder Schwefel steht, wobei
- R⁶: für gegebenenfalls einfach substituiertes geradkettiges oder verzweigtes Alkyl mit 1 bis 12 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl, Trialkylsilyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
- R⁶: außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor oder Brom - steht;
- R⁶: außerdem für jeweils gegebenenfalls einfach bis dreifach, gleich oder verschieden durch Halogen - insbesondere Fluor, Chlor oder Brom - substituiertes Alkenyl oder Alkinyl mit jeweils 2 bis 6 Kohlenstoffatomen steht;
- R⁶: außerdem für gegebenenfalls einfach bis vierfach, gleich oder verschieden durch Halogen - insbesondere Fluor, Chlor oder Brom - und/oder geradkettiges oder verzweigtes Alkyl mit 1 bis 3 Kohlenstoffatomen substituiertes Cycloalkyl mit 3 bis 6 Kohlenstoffatomen steht;
- R⁶: außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht oder für einen gegebenenfalls einfach bis dreifach, gleich oder verschieden substituierten und/oder benzannellierten, gesättigten oder ungesättigten, fünf- bis sechsgliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen: Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen und/oder 3-methyl-1,2,4-oxadiazol-2-yl-substituiertes Phenyl;
- R⁷: für Wasserstoff oder für gegebenenfalls einfach substituiertes geradkettiges oder verzweigtes Alkyl mit 1 bis 6 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl, Trialkylsilyl oder Akylsulfonylaminocarbonyl mit jeweils 1 bis 6 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
- R⁷: außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor oder Brom - steht;
- R⁷: außerdem für jeweils gegebenenfalls einfach bis dreifach, gleich oder verschieden durch Halogen - insbesondere Fluor, Chlor oder Brom - substituiertes Alkenyl oder Alkinyl mit jeweils 2 bis 6 Kohlenstoffatomen steht;
- R⁷: außerdem für gegebenenfalls einfach bis vierfach, gleich oder verschieden durch Halogen - insbesondere Fluor, Chlor oder Brom - und/oder geradkettiges oder verzweigtes Alkyl mit 1 bis 3 Kohlenstoffatomen substituiertes Cycloalkyl mit 3 bis 6 Kohlenstoffatomen steht oder
- R⁷: für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht, wobei als Phenylsubstituenten jeweils infrage kommen:
Fluor, Chlor, Brom, Iod, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl.

Ganz besonders bevorzugt sind Verbindungen der Formel (I), bei welchen
- R¹: für Wasserstoff, Fluor, Chlor, Brom, Cyano, Hydroxy oder für einen der Reste -R⁶, -O-R⁶, -O-NR⁶R⁷, -S-R⁶, -S(O)-R⁶ oder -SO₂-R⁶ steht,
- R²: für Wasserstoff, Hydroxy, Amino, Cyano oder für einen der Reste -R⁶, -O-R⁶ oder -N=CR⁶R7 steht,
- R³: für Wasserstoff, Fluor, Chlor, Brom, für geradkettiges oder verzweigtes Alkyl mit 1 bis 4 Kohlenstoffatomen oder für Halogenalkyl mit 1 bis 2 Kohlenstoffatomen und 1 bis 5 gleichen oder verschiedenen Halogenatomen - insbesondere Fluor, Chlor oder Brom - steht,
- R⁴: für Wasserstoff, für einen der Reste -R⁶, -O-R⁶ oder -SO₂-R⁶, für ein Äquivalent eines Natrium- oder Kaliumkations oder für ein gegebenenfalls einfach bis vierfach, gleich oder verschieden durch Alkyl mit 1 bis 8 Kohlenstoffatomen substituiertes Ammoniumkation steht und
- R⁵: für Amino, Hydroxy oder für einen der Reste -R⁶ oder -NR⁶R⁷ steht, oder
- R⁴ und R⁵: gemeinsam für einen zweifach verknüpften Alkandiylrest mit 2 bis 5 Kohlenstoffatomen stehen und
- X: für Sauerstoff oder Schwefel steht, wobei
- R⁶: für gegebenenfalls einfach substituiertes geradkettiges oder verzweigtes Alkyl mit 1 bis 10 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Al-kylaminocarbonyl, N,N-Dialkylaminocarbonyl, Trialkylsilyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
- R⁶: außerdem für Halogenalkyl mit 1 oder 2 Kohlenstoffatomen und 1 bis 5 gleichen oder verschiedenen Halogenatomen - insbesondere Fluor oder Chlor - steht;
- R⁶: außerdem für jeweils gegebenenfalls einfach durch Halogen - insbesondere Fluor oder Chlor - substituiertes Alkenyl oder Alkinyl mit jeweils 2 bis 5 Kohlenstoffatomen steht;
- R⁶: außerdem für gegebenenfalls einfach oder zweifach, gleich oder verschieden durch Fluor, Chlor, Methyl und/oder Ethyl substituiertes Cyclopropyl oder Cyclohexyl steht oder
- R⁶: für jeweils gegebenenfalls im Phenylteil einfach, zweifach oder dreifach, gleich oder verschieden substituiertes Phenylalkyl oder Phenyl mit gegebenenfalls 1 oder 2 Kohlenstoffatomen im Alkylteil steht oder für einen gegebenenfalls einfach oder zweifach, gleich oder verschieden substituierten und/oder benzannellierten, fünf- oder sechsgliedrigen Heteroarylrest mit 1 bis 3 gleichen oder verschiedenen Heteroatomen - insbesondere Stickstoff, Sauerstoff und/oder Schwefel - steht, wobei als Phenyl- bzw. Heteroarylsubstituenten jeweils infrage kommen:
Fluor, Chlor, Brom, Cyano, Nitro, Amino, N-Acetylamino, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl oder gegebenenfalls ein- bis zweifach, gleich oder verschieden durch Fluor, Chlor, Brom, Methyl, Ethyl, Methoxy, Ethoxy, Trifluormethyl und/oder Trifluormethoxy substituiertes Phenyl und
- R⁷: für Wasserstoff oder für gegebenenfalls einfach substituiertes geradkettiges oder verzweigtes Alkyl mit 1 bis 4 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl, Trialkylsilyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff Sauerstoff und/oder Schwefel - steht;
- R⁷: außerdem für Halogenalkyl mit 1 oder 2 Kohlenstoffatomen und 1 bis 5 gleichen oder verschiedenen Halogenatomen - insbesondere Fluor oder Chlor - steht;
- R⁷: außerdem für jeweils gegebenenfalls einfach durch Halogen - insbesondere Fluor oder Chlor - substituiertes Alkenyl oder Alkinyl mit jeweils 2 bis 5 Kohlenstoffatomen steht;
- R⁷: außerdem für gegebenenfalls einfach oder zweifach, gleich oder verschieden durch Fluor, Chlor, Methyl und/oder Ethyl substituiertes Cyclopropyl oder Cyclohexyl steht, oder
- R⁷: für jeweils gegebenenfalls im Phenylteil einfach oder zweifach, gleich oder verschieden substituiertes Phenylalkyl oder Phenyl mit gegebenenfalls 1 oder 2 Kohlenstoffatomen Alkylteil steht, wobei als Phenylsubstituenten jeweils infrage kommen:
Fluor, Chlor, Brom, Cyano, Nitro, Methyl, Ethyl, n- oder i-Propyl, n-, i-, s- oder oder t-Butyl, Methoxy, Ethoxy, n- i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl oder gegebenenfalls ein- bis zweifach, gleich oder verschieden durch Fluor, Chlor, Brom, Methyl, Ethyl, Methoxy, Ethoxy, Trifluormethyl und/oder Trifluormethoxy substituiertes Phenyl.

Im einzelnen seien außer den bei den Herstellungsbeispielen aufgeführten Verbindungen die folgenden substituierten Triazolinone der allgemeinen Formel (I) genannt:

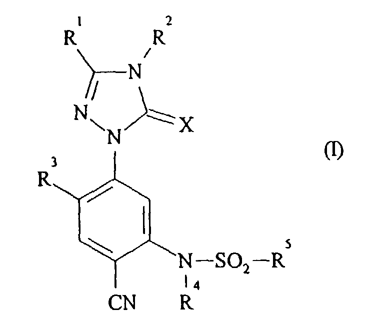

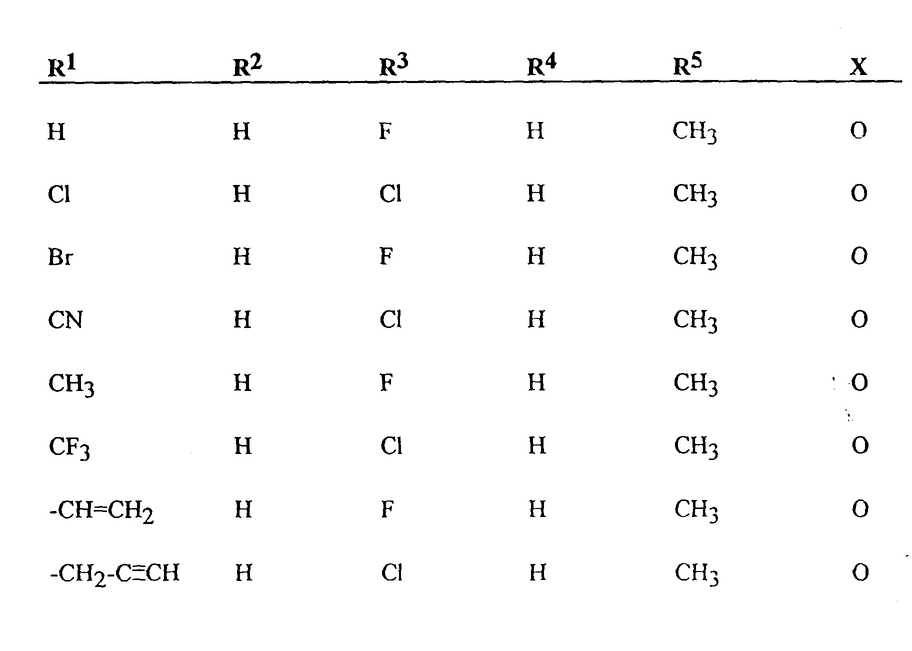

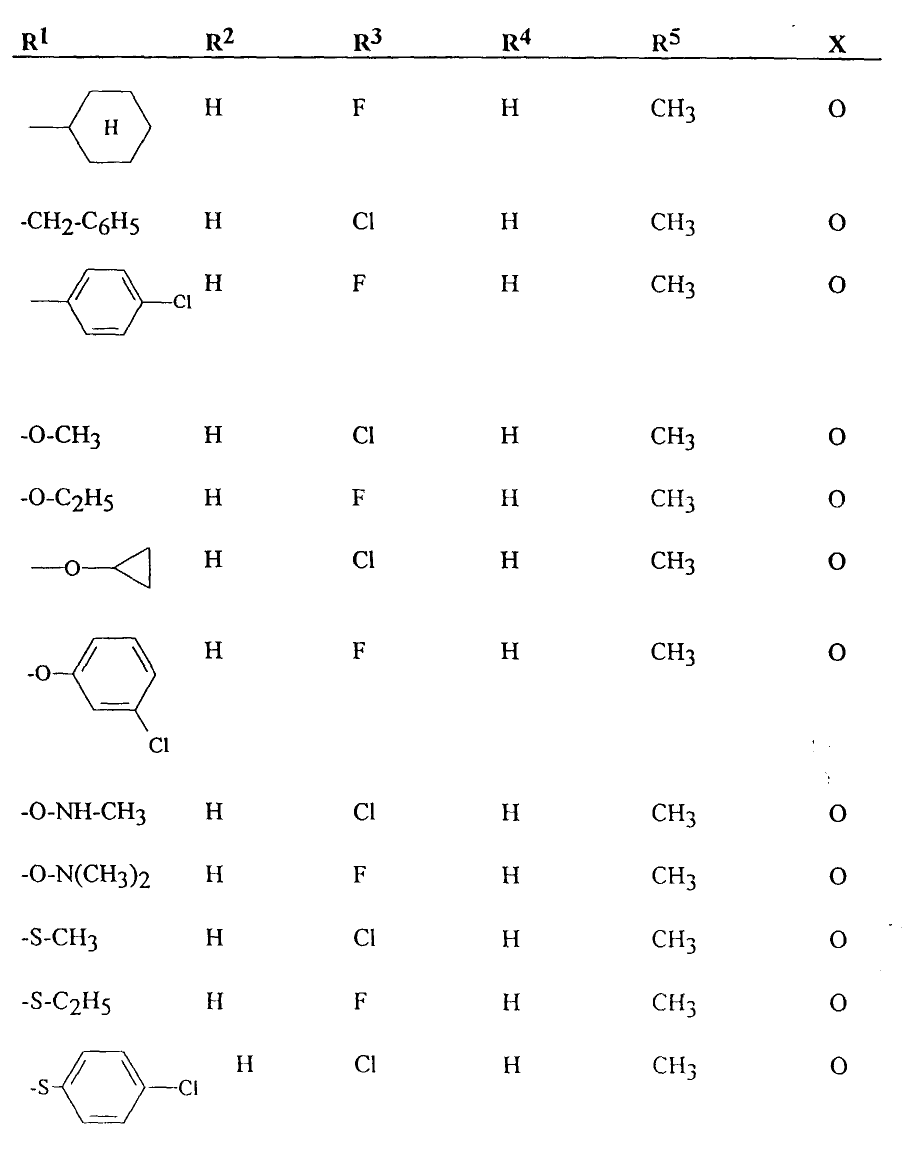

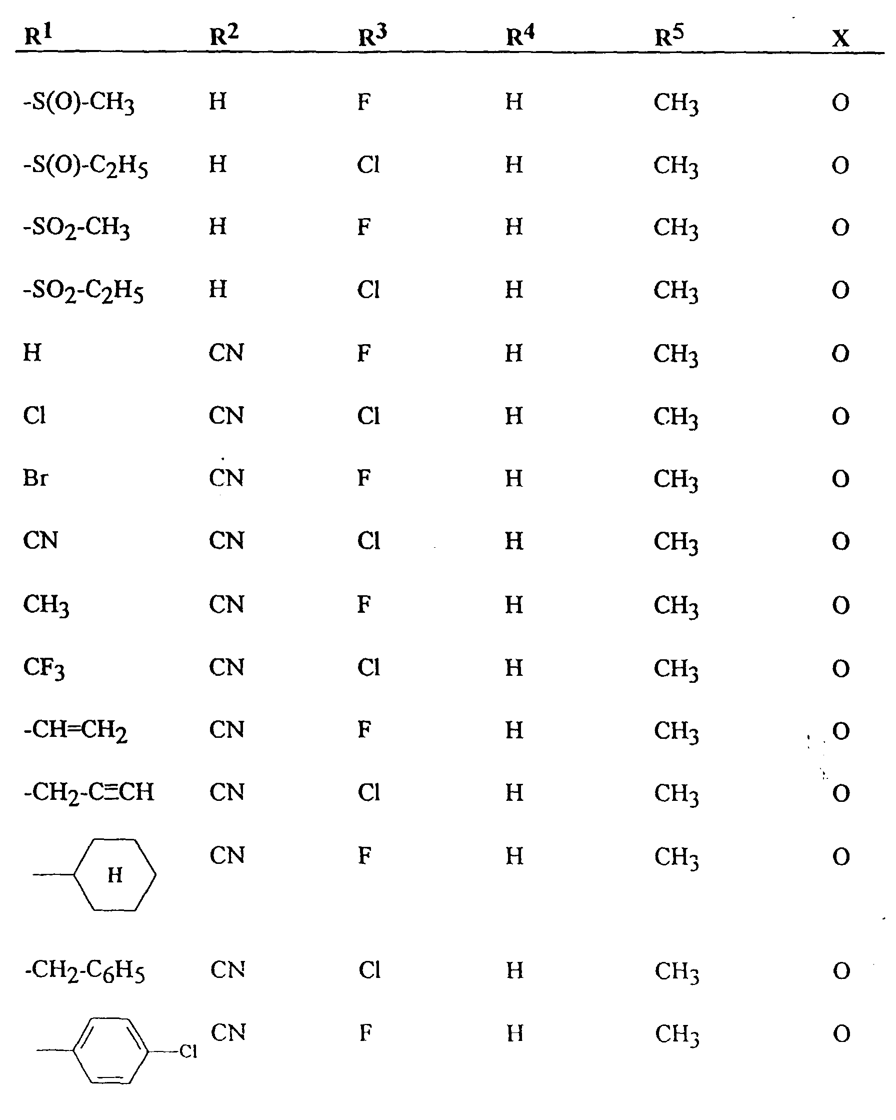

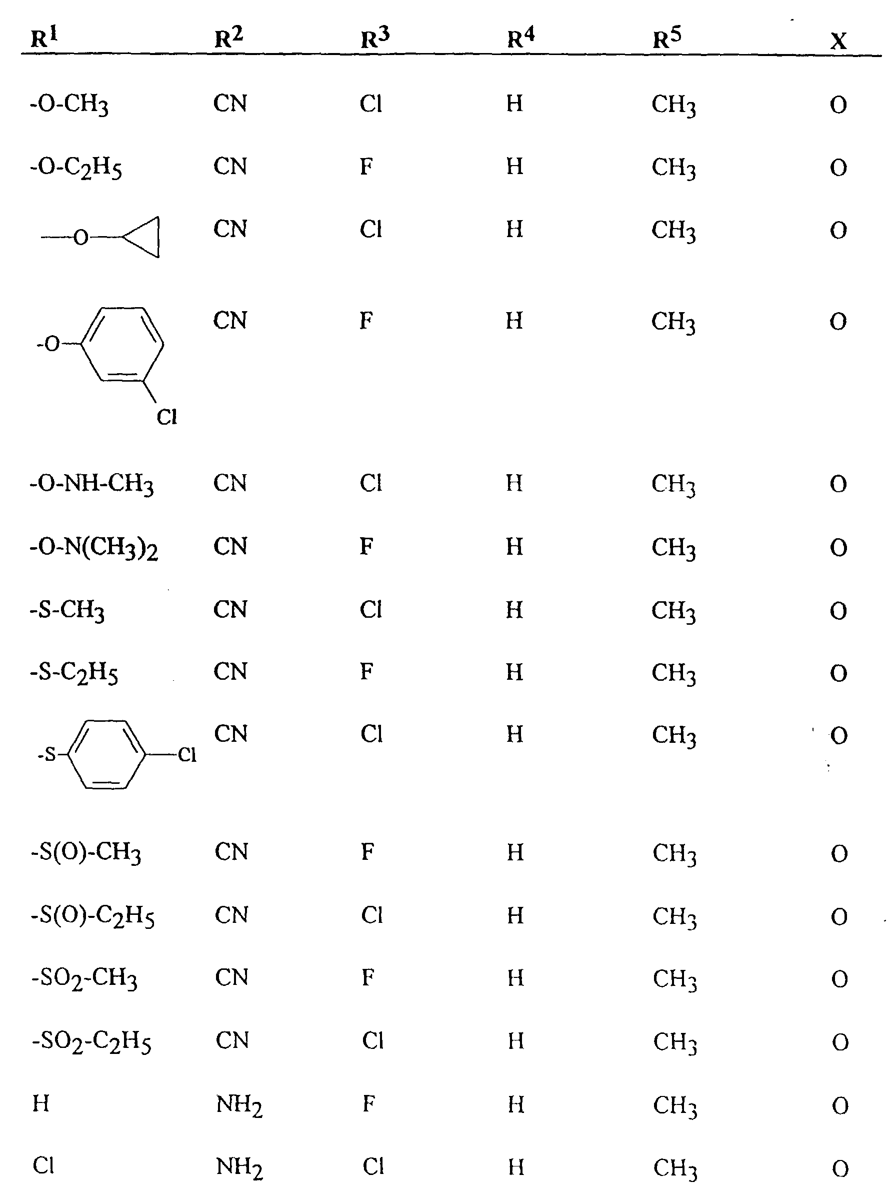

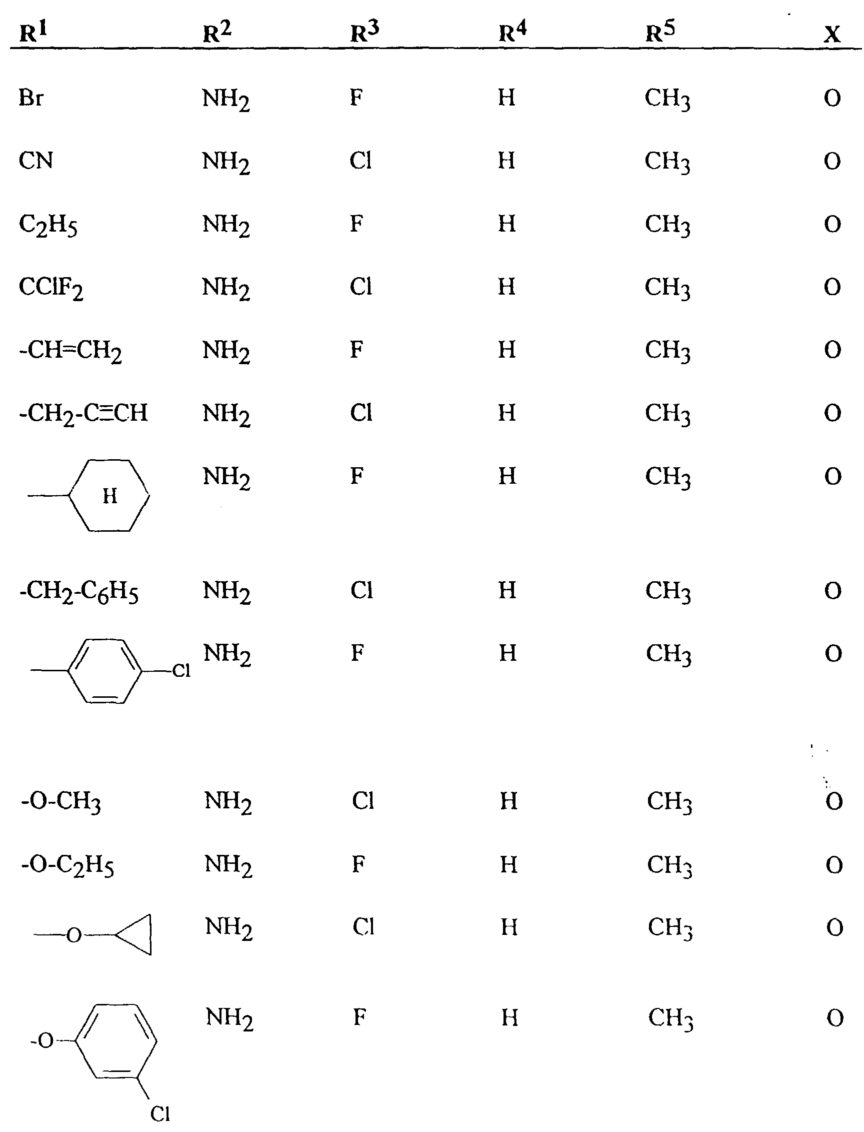

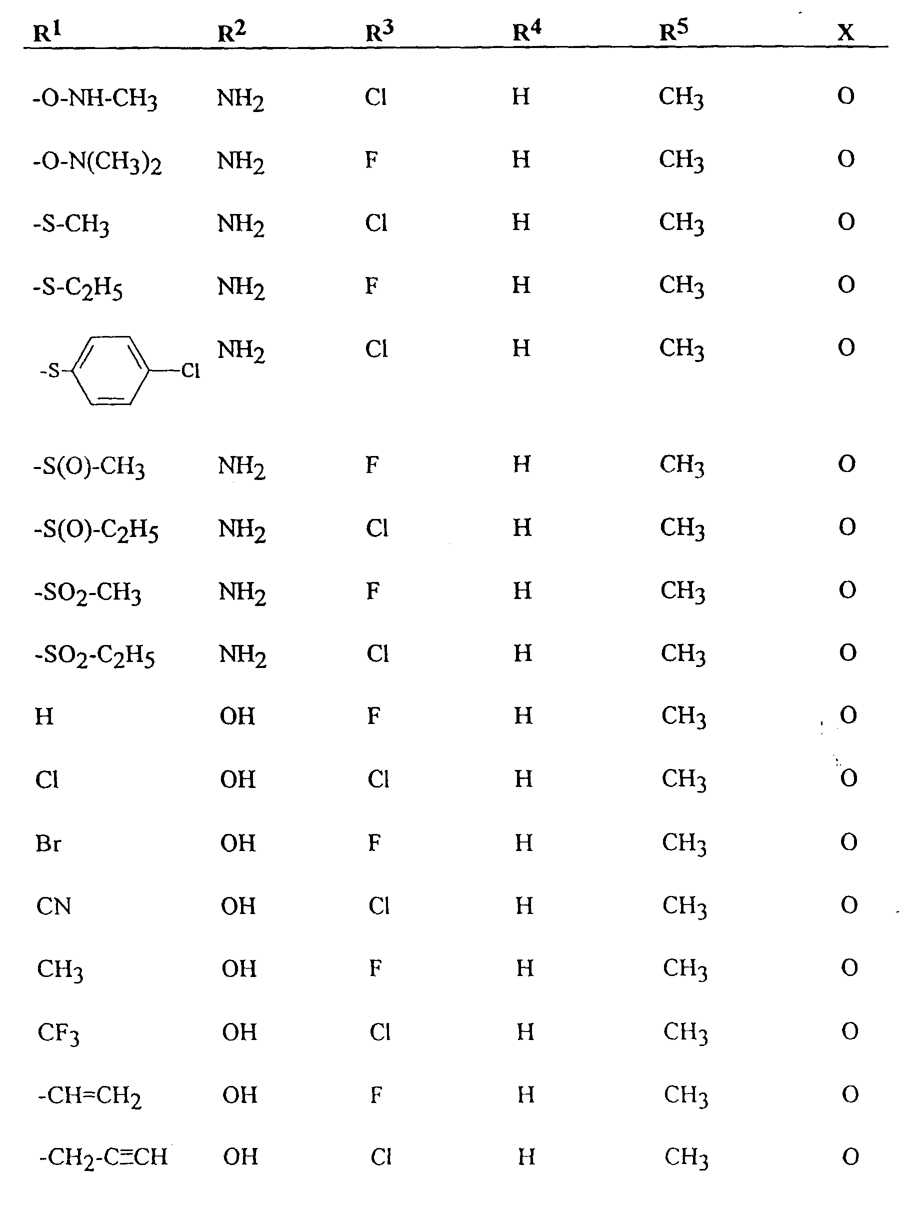

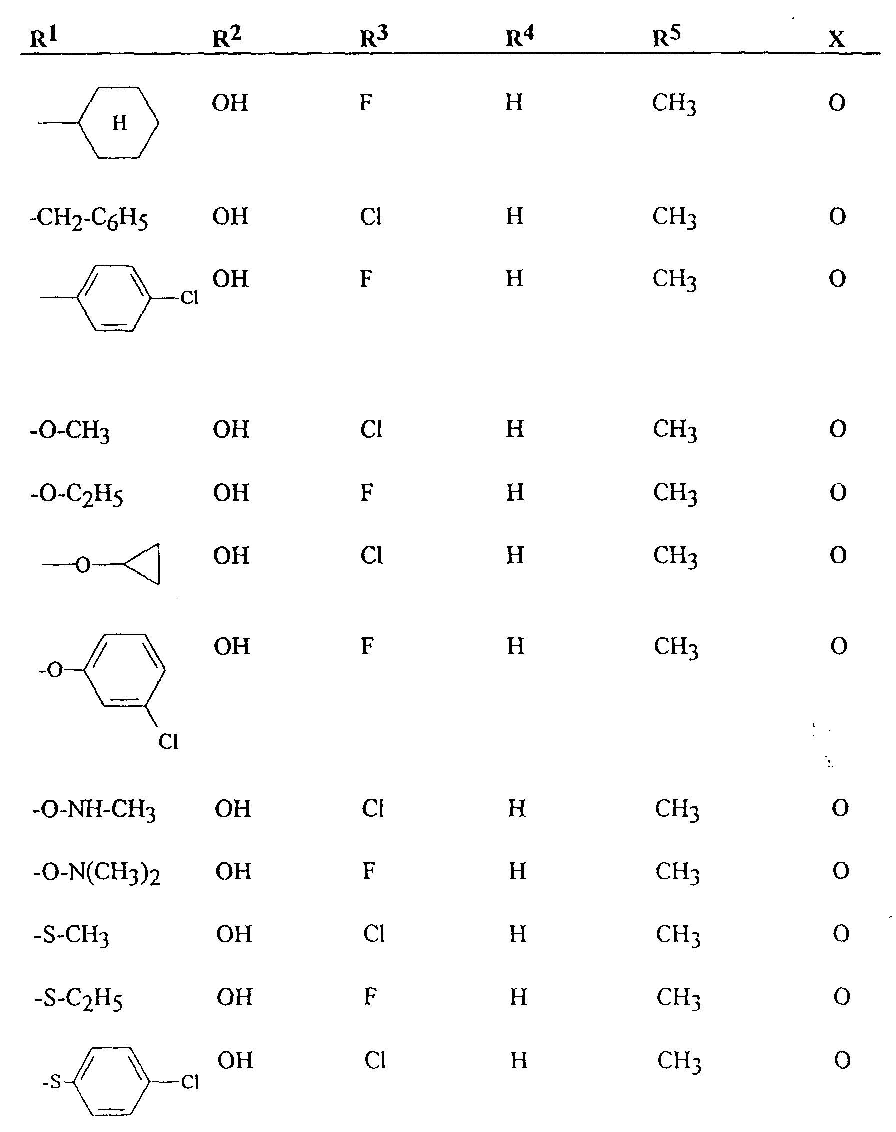

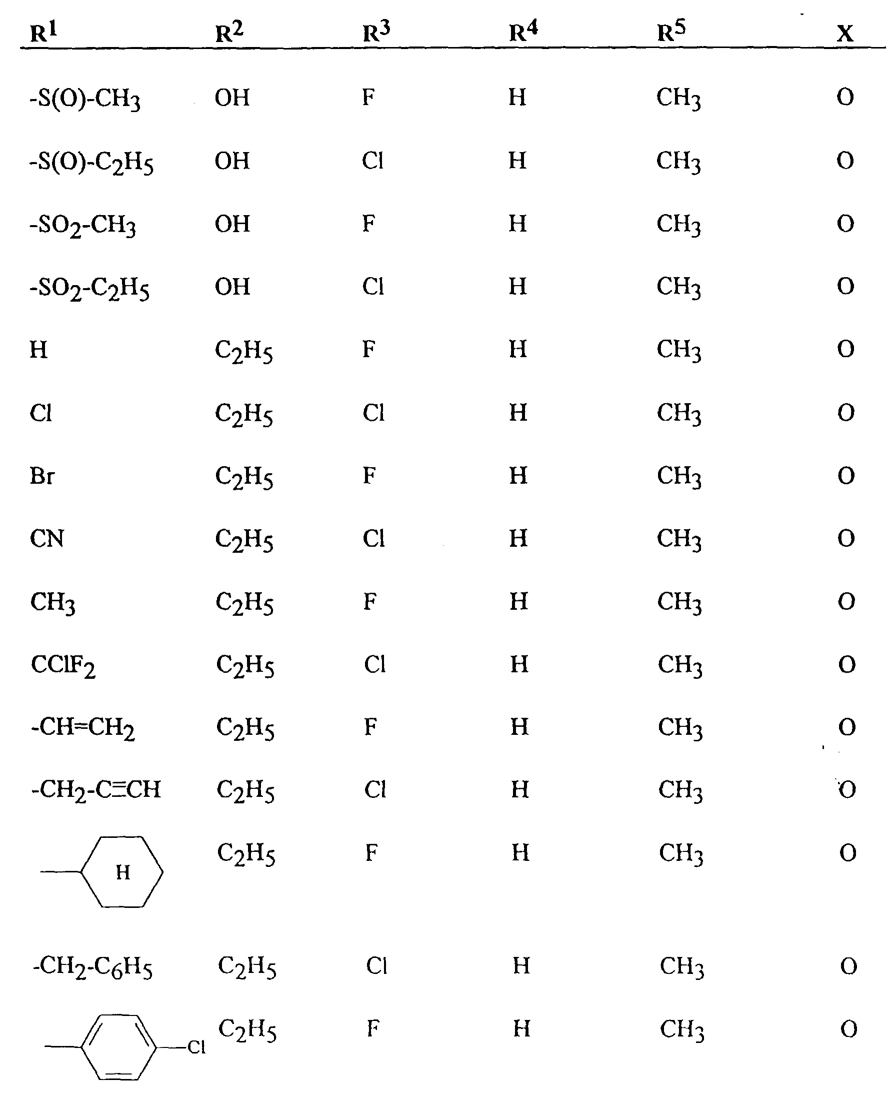

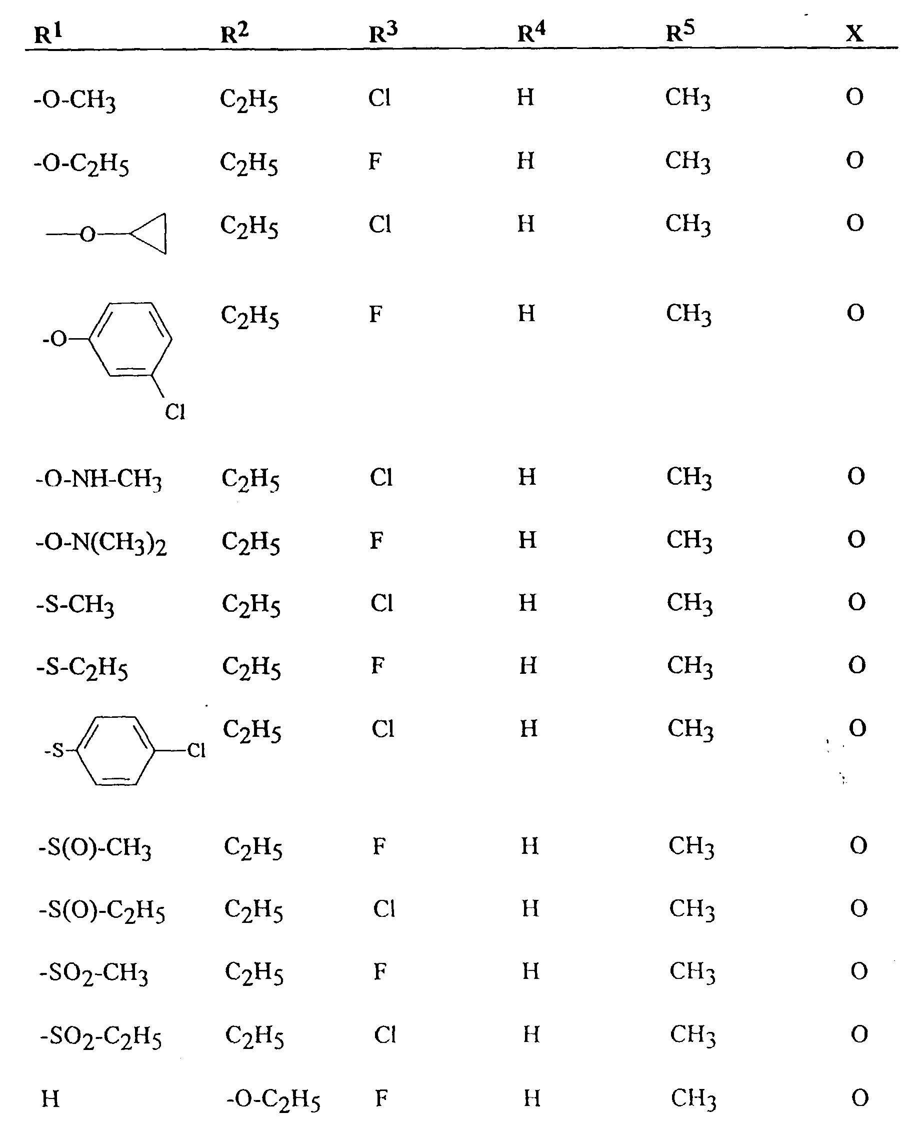

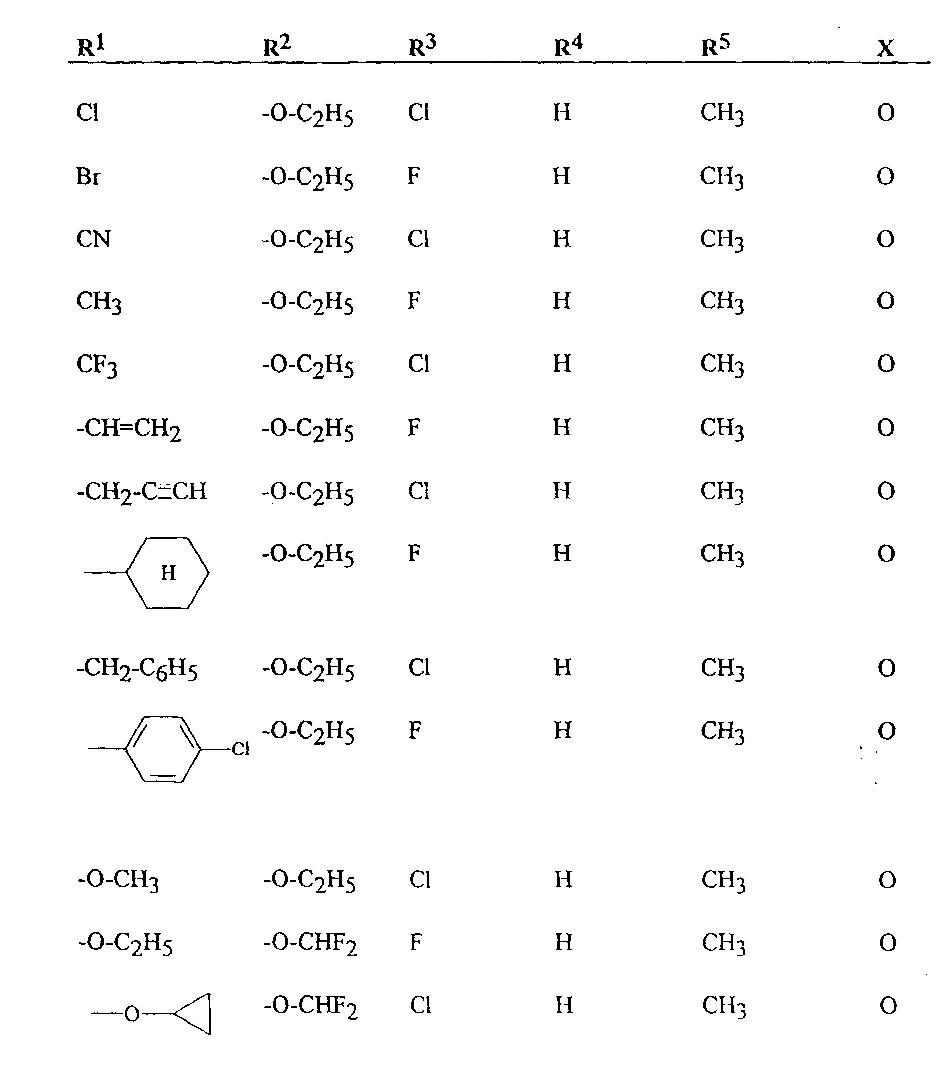

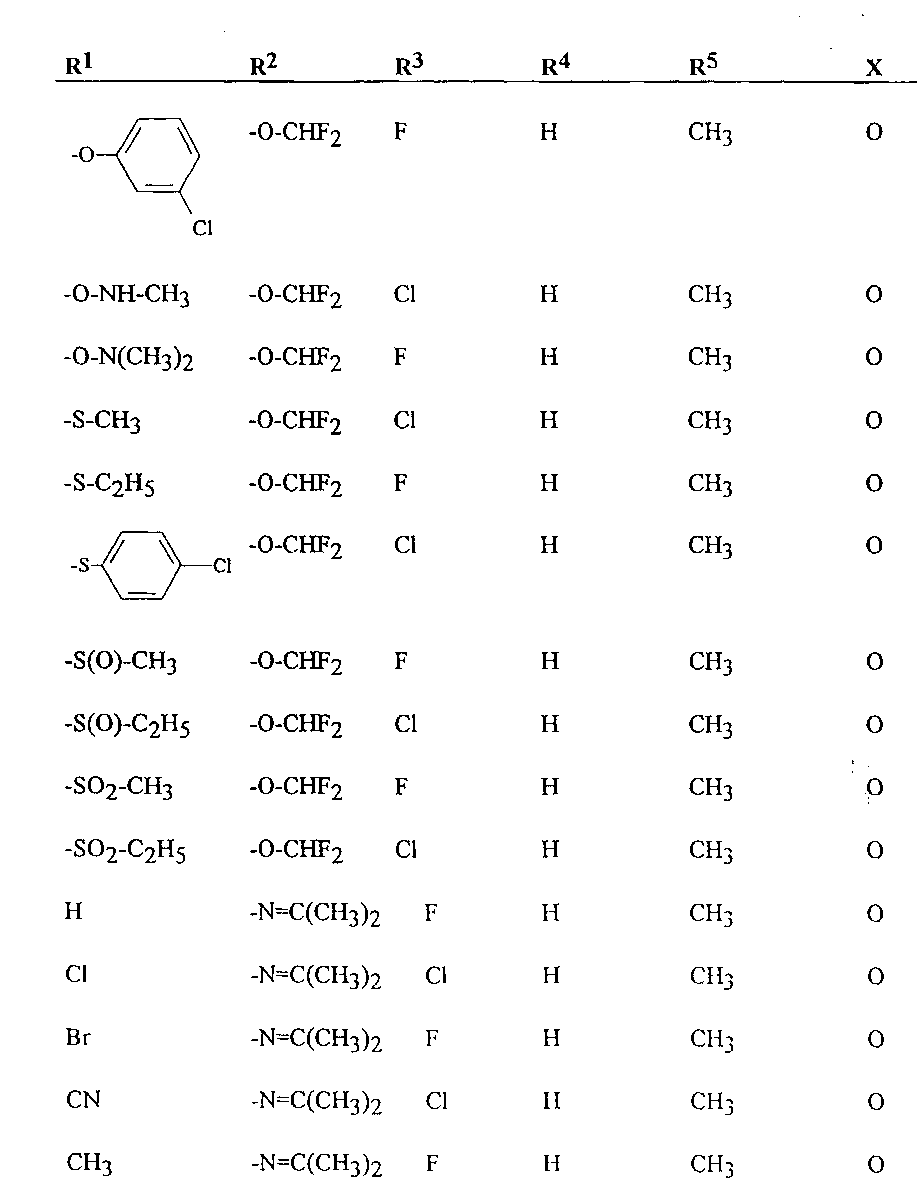

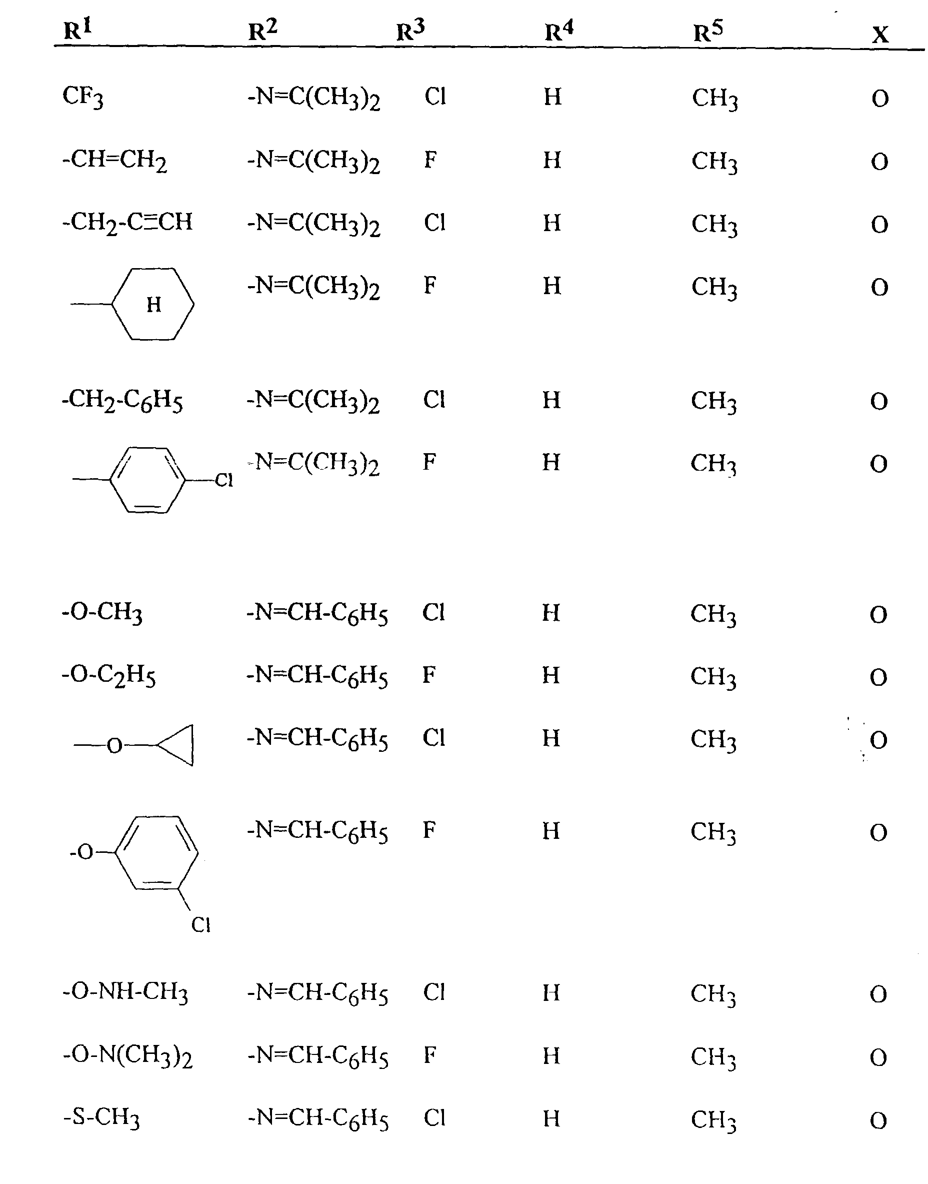

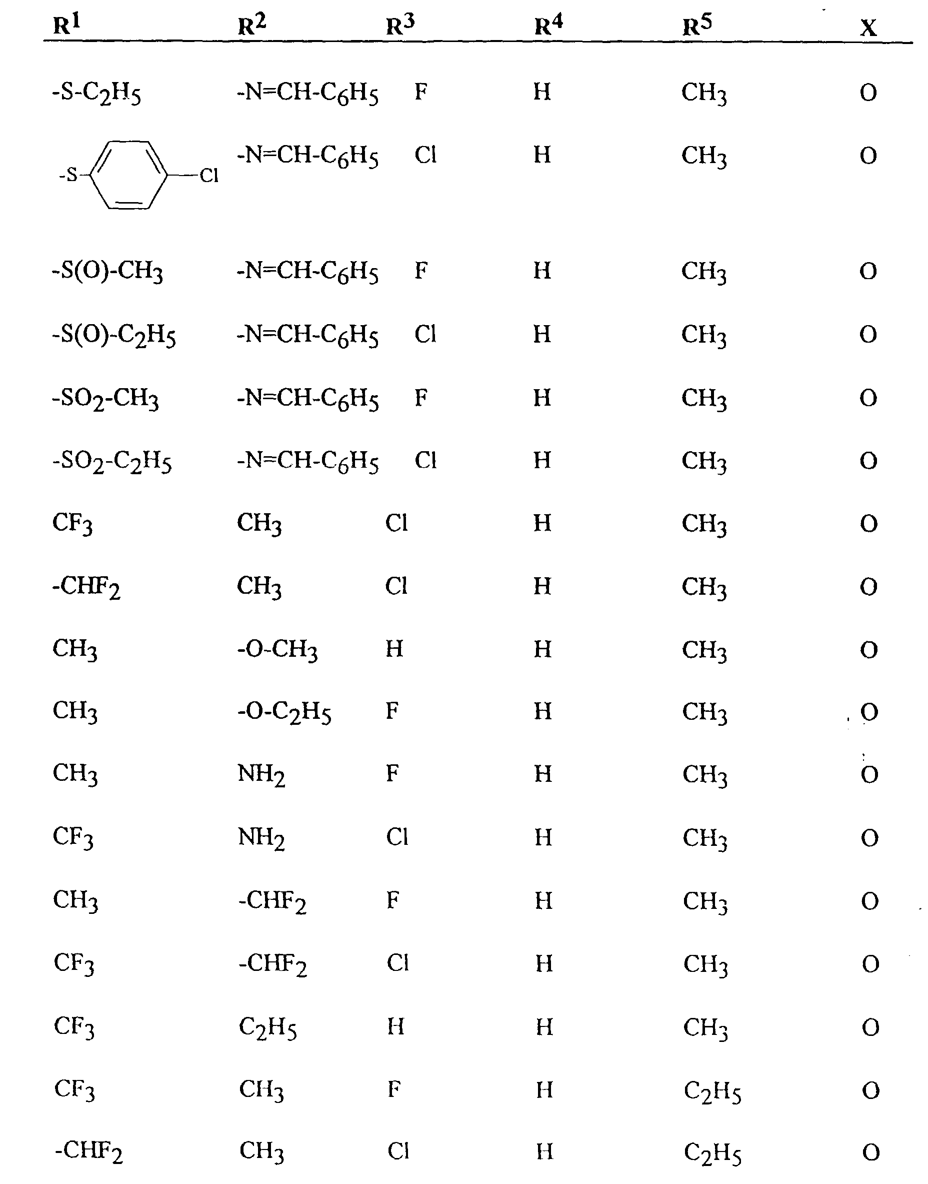

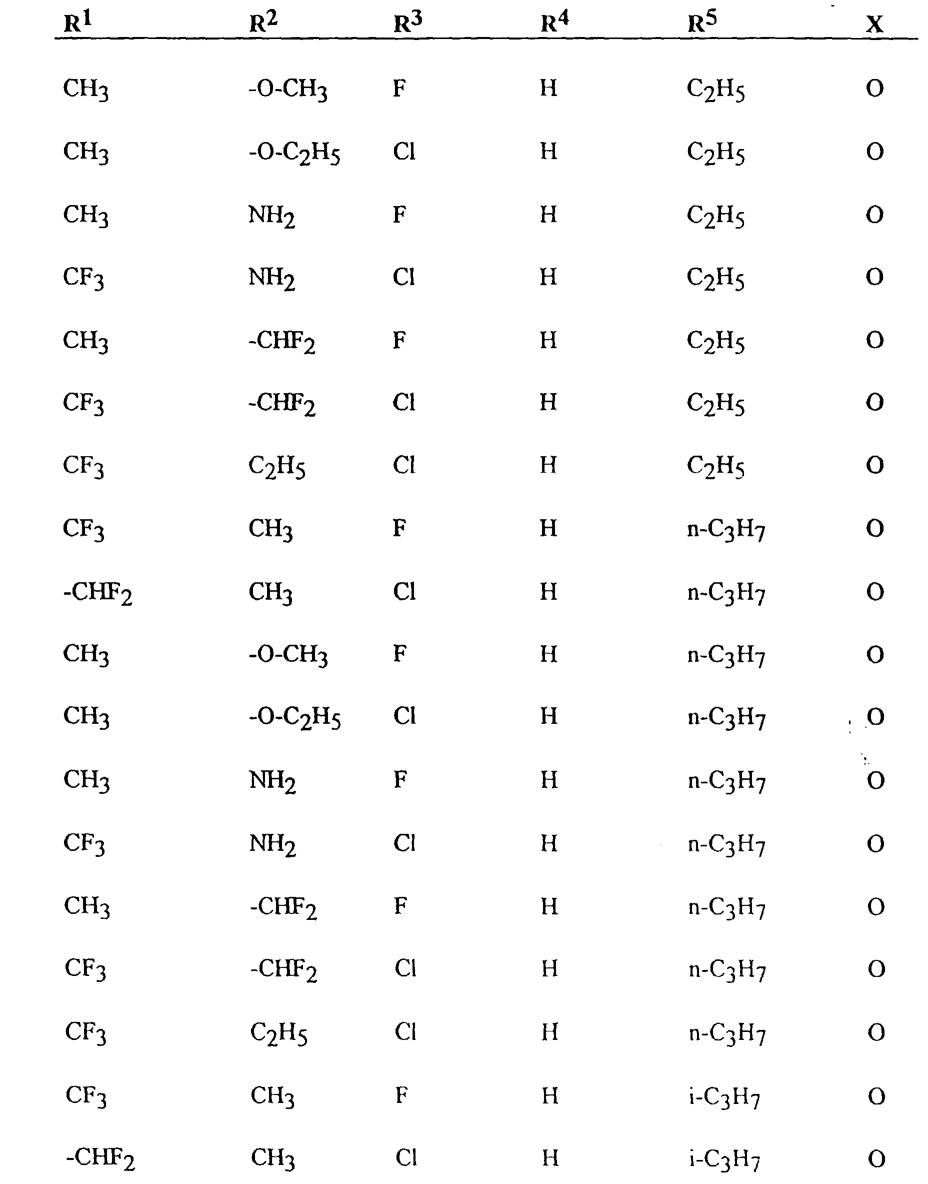

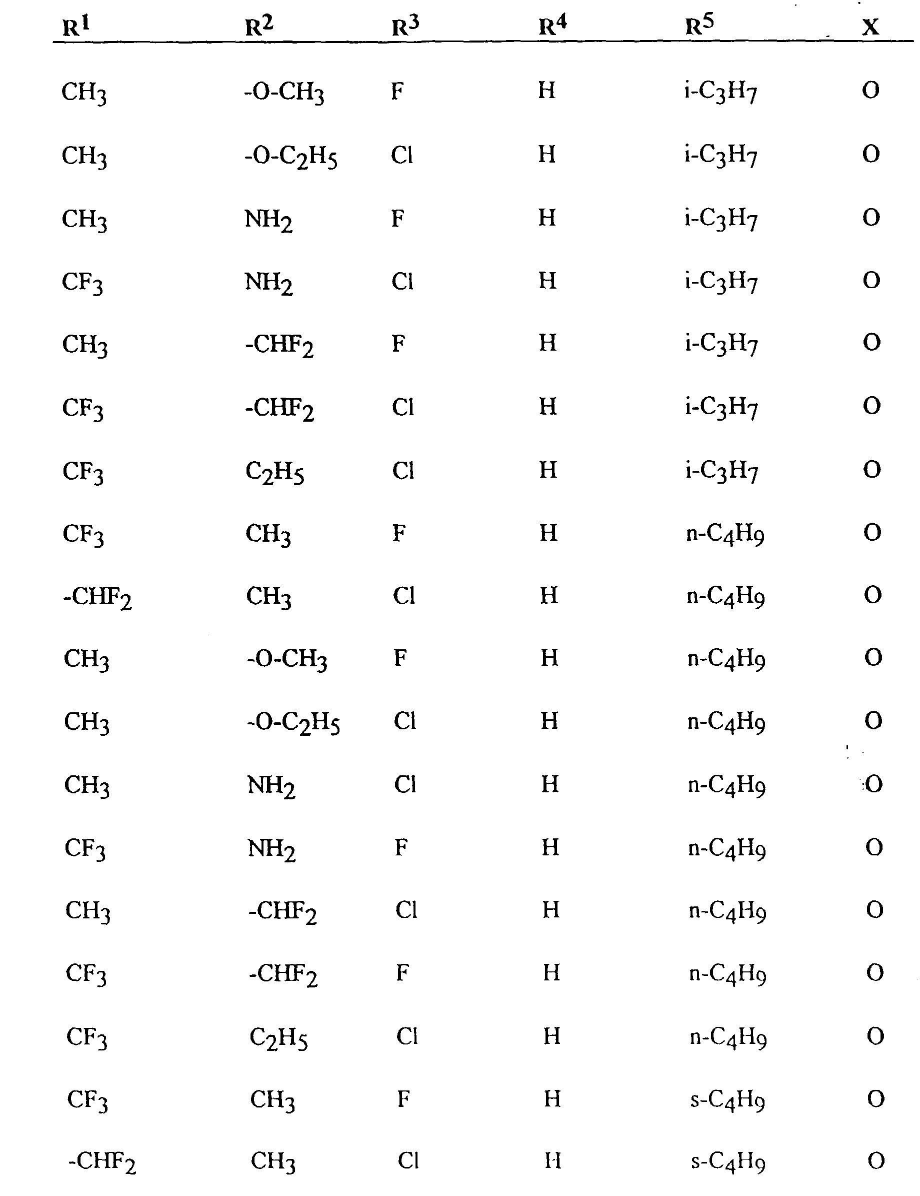

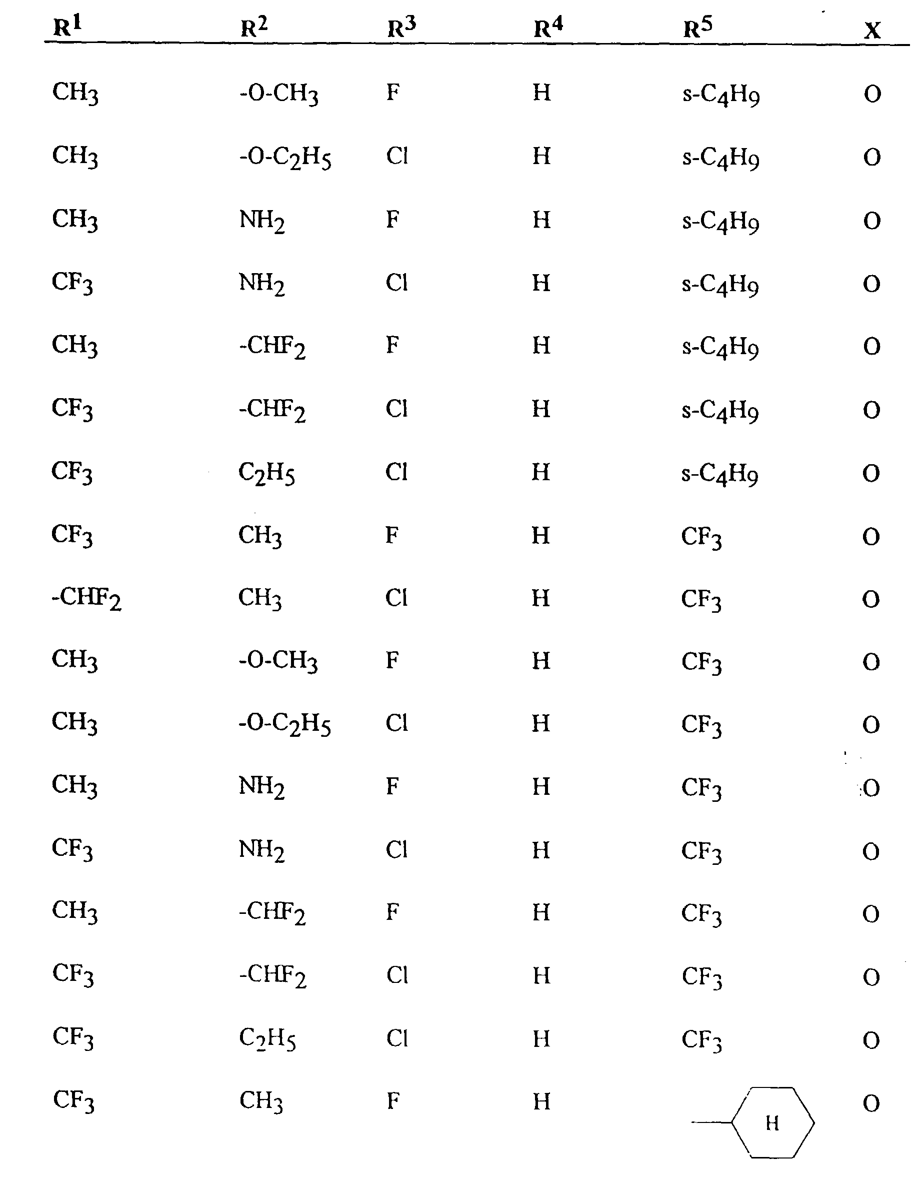

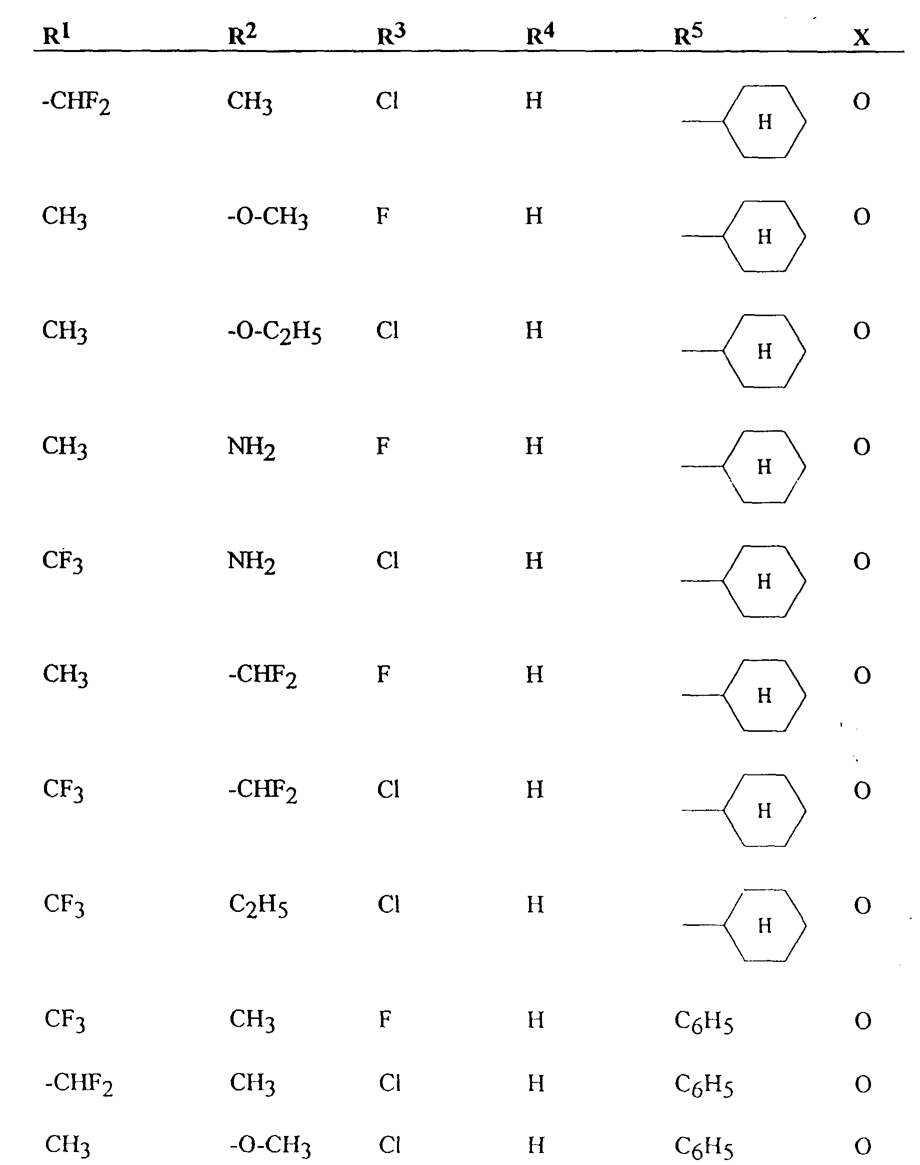

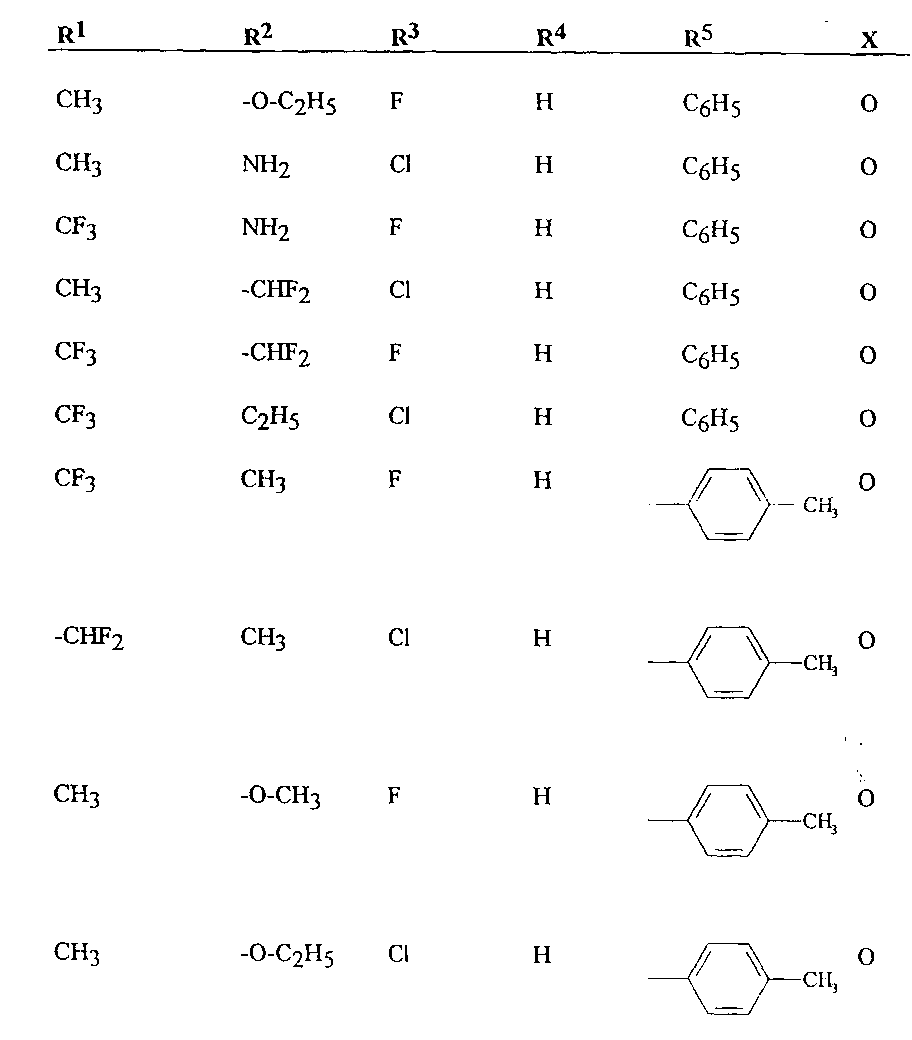

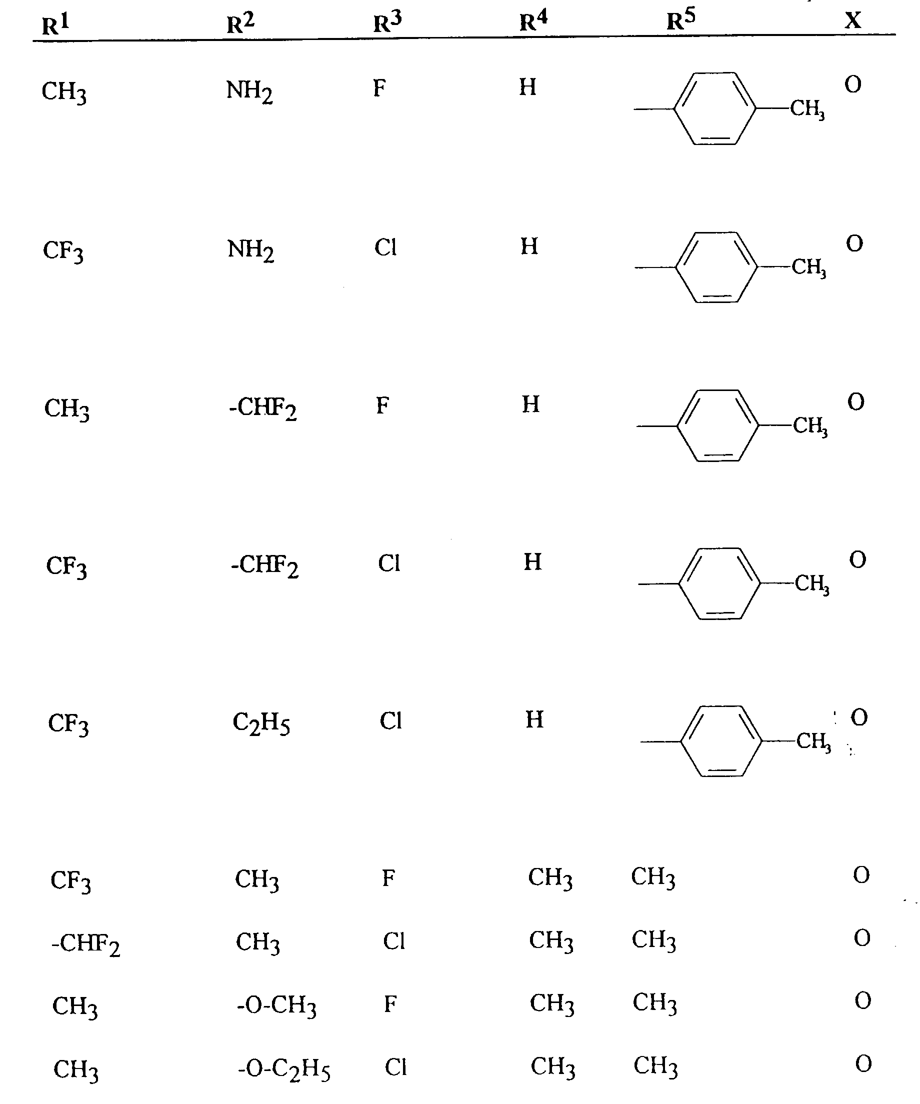

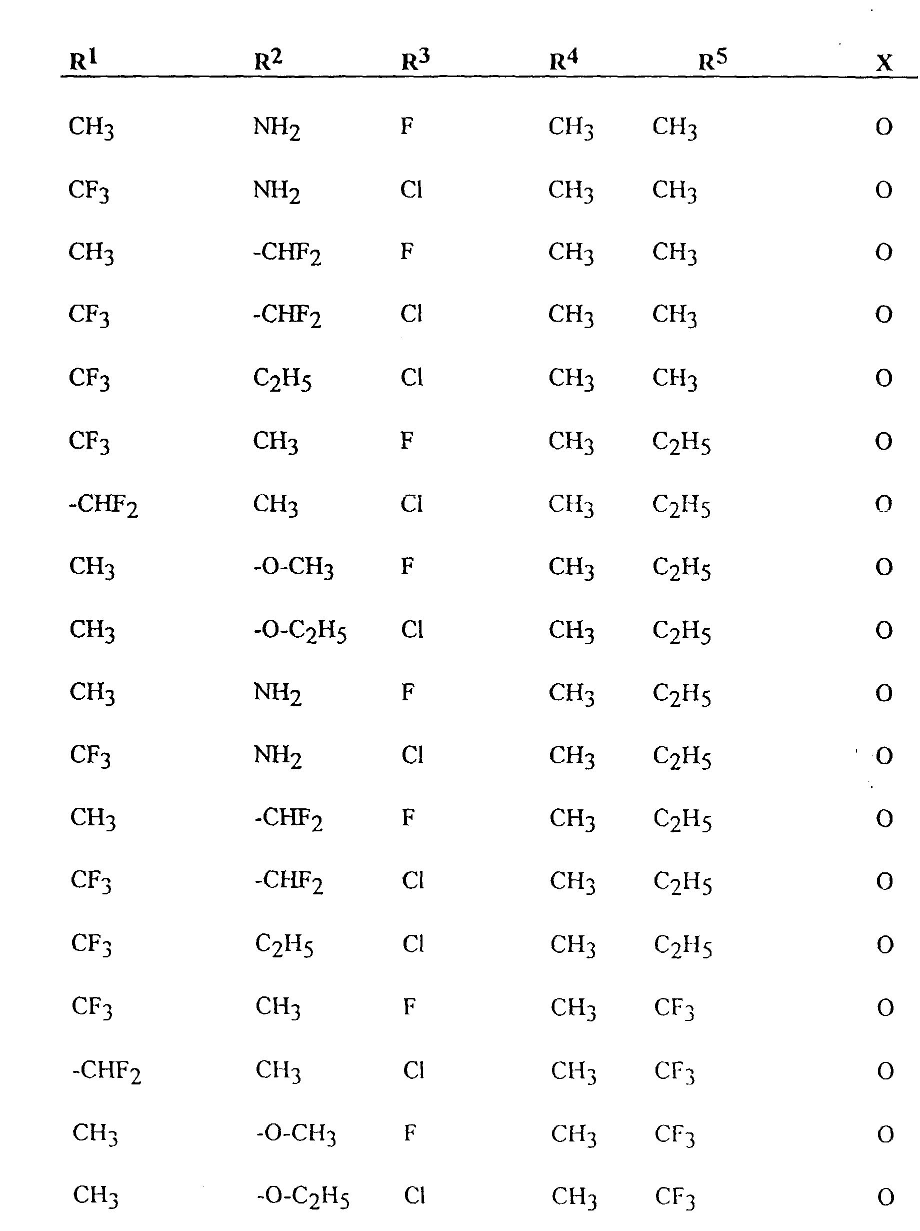

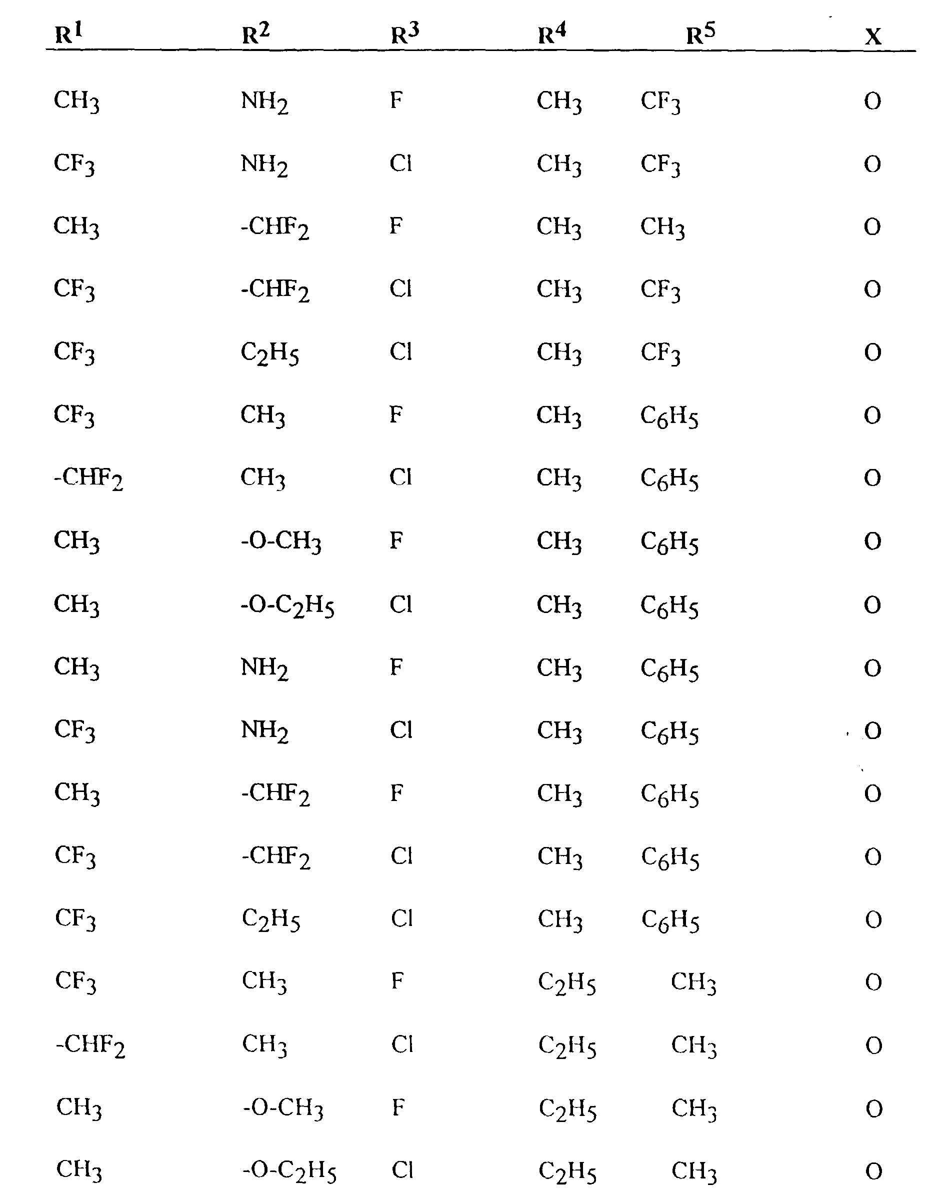

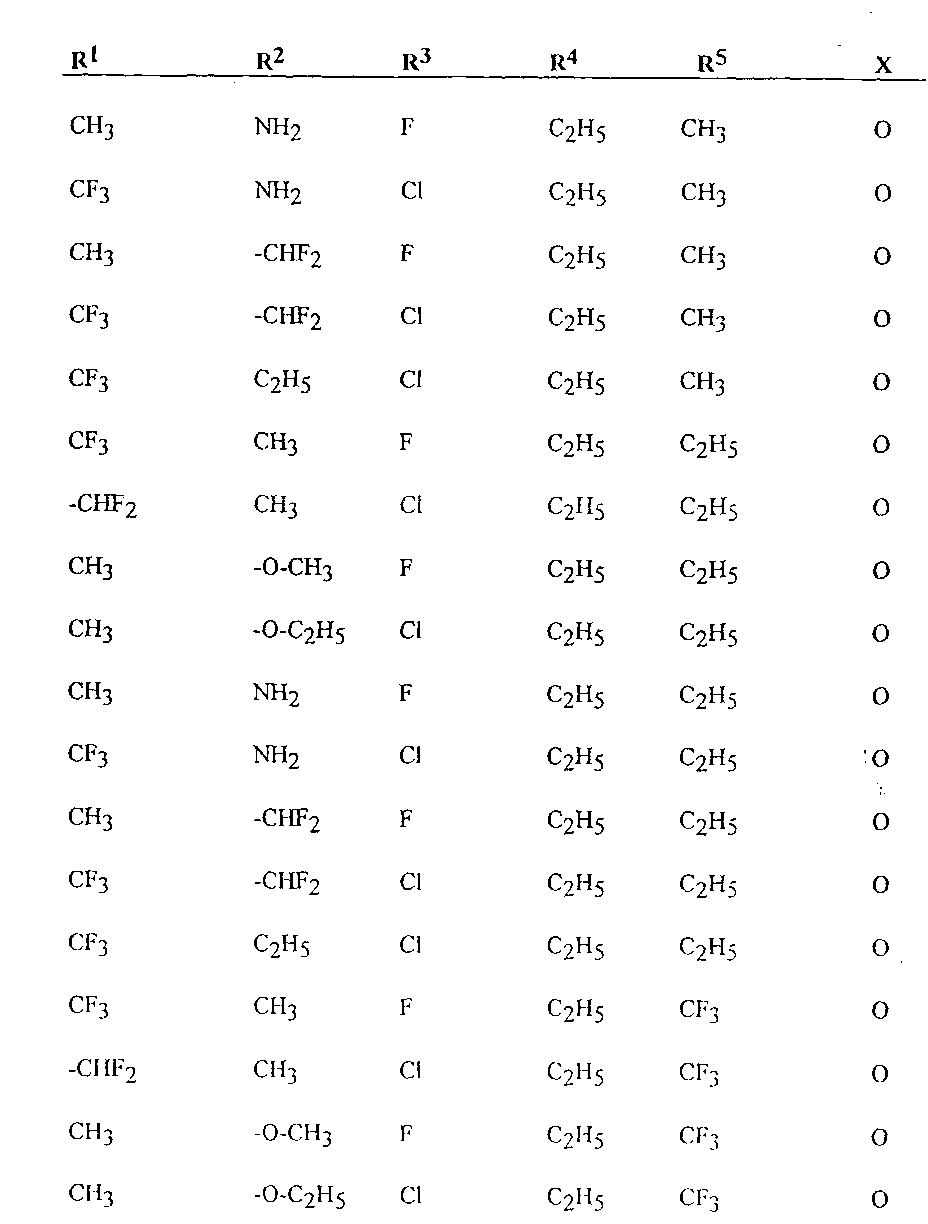

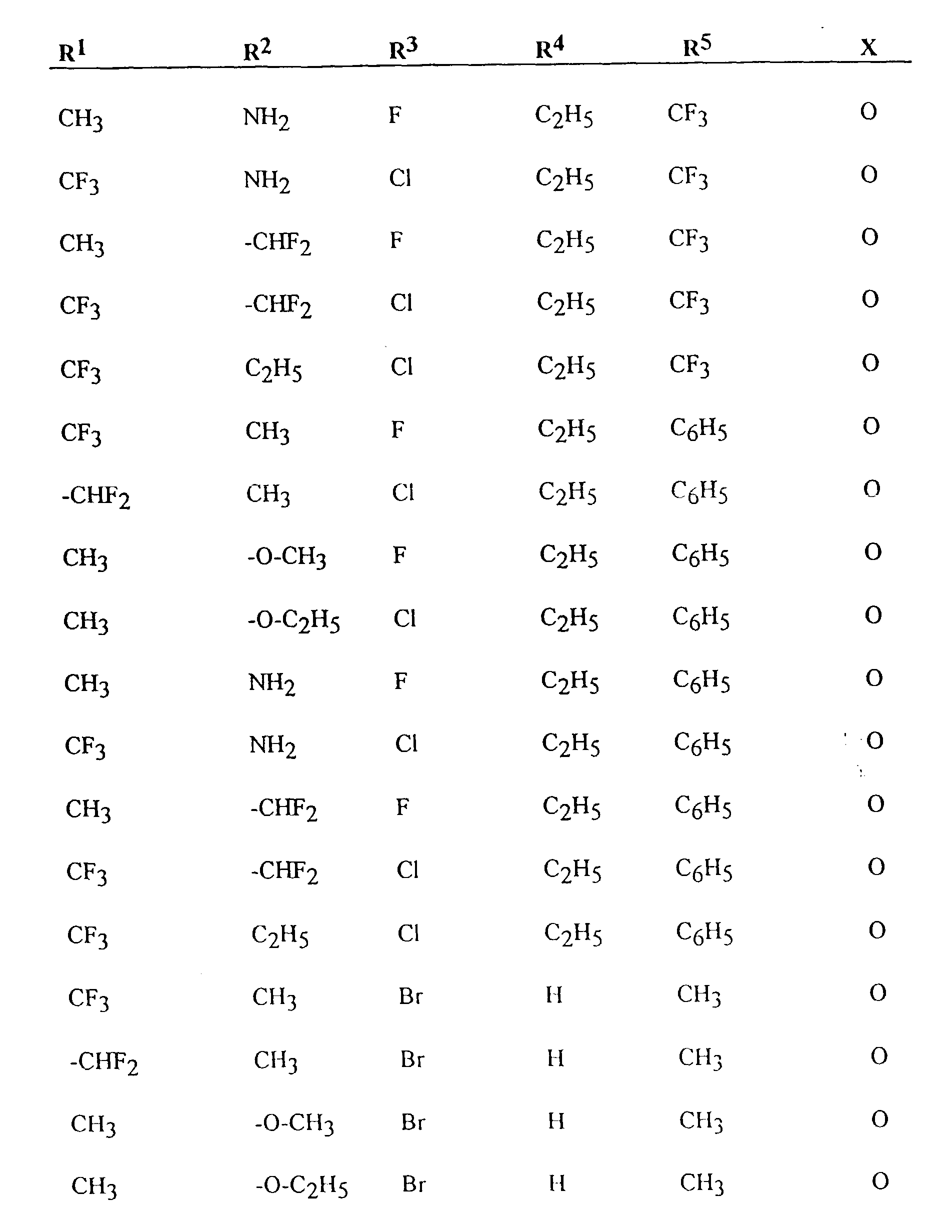

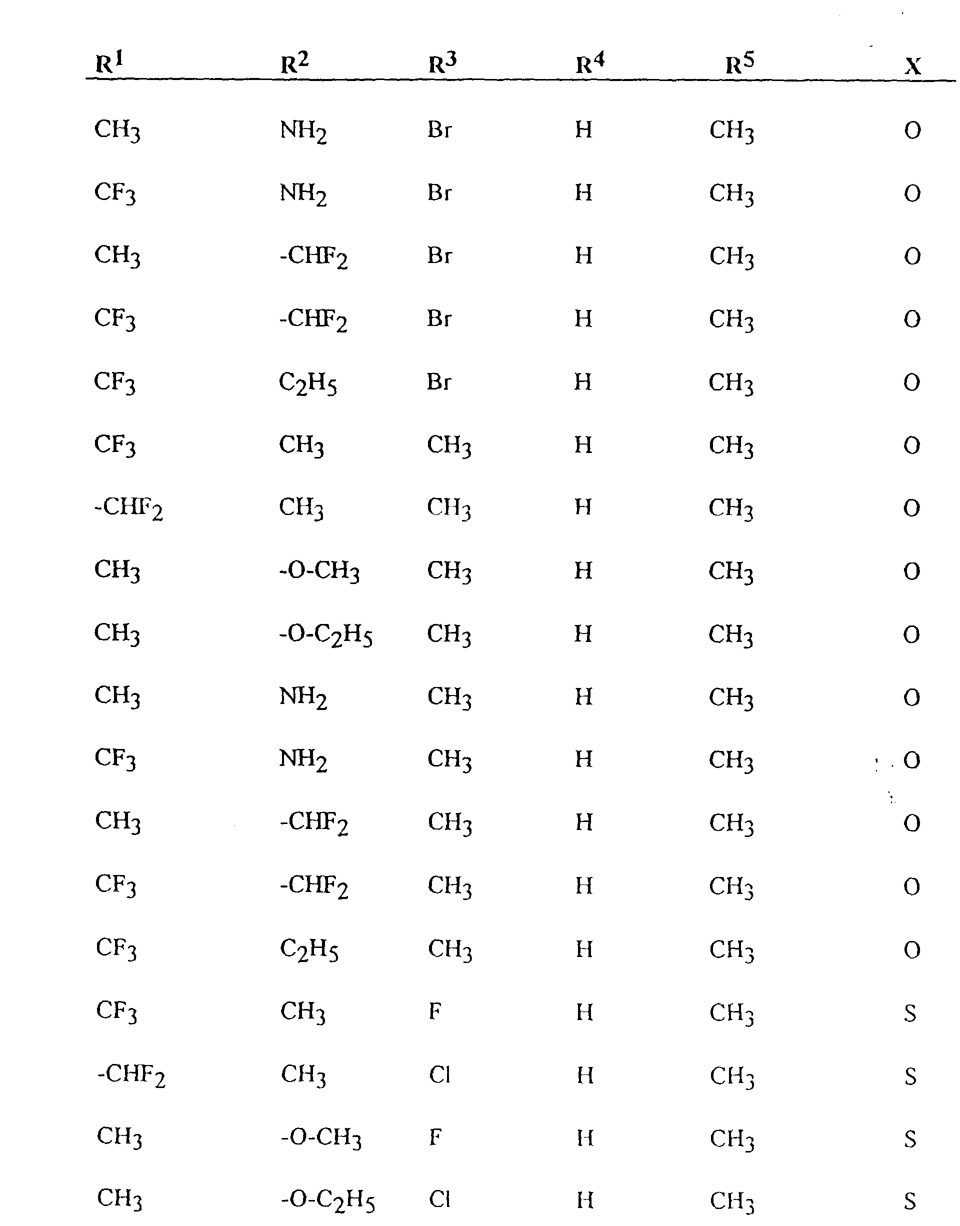

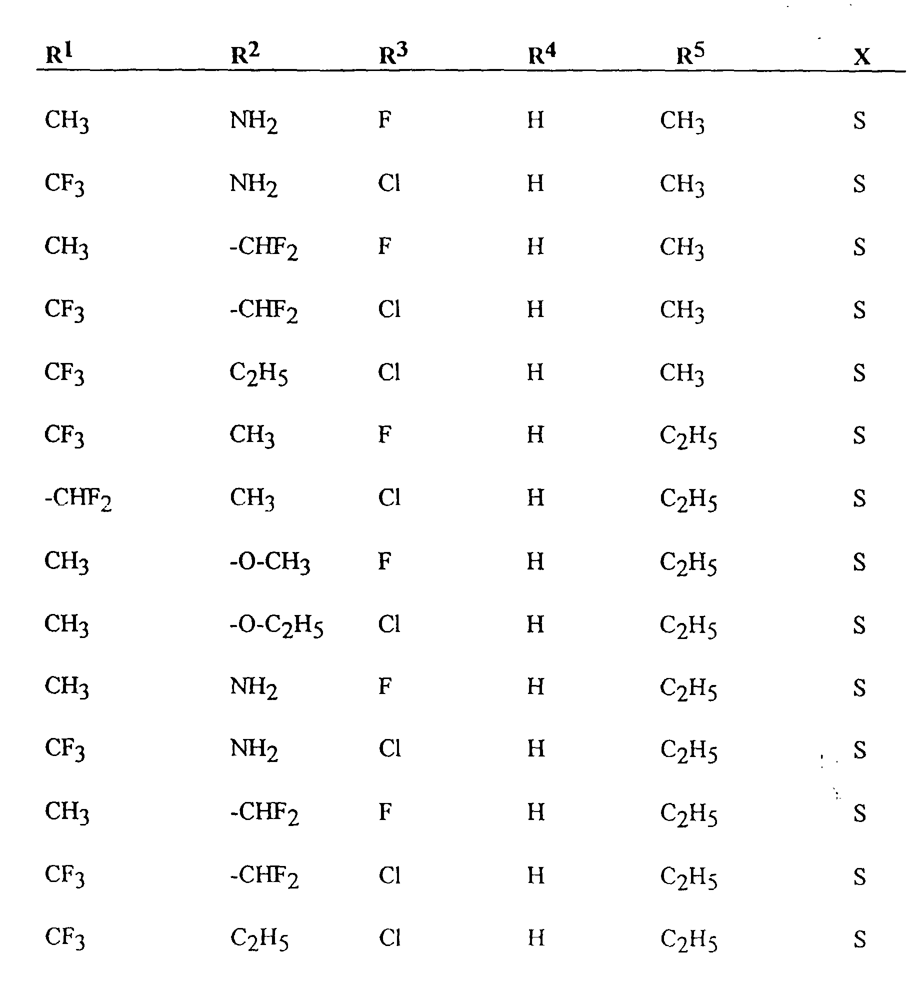

Verwendet man beispielsweise 4-Methyl-3-trifluormethyl-1,2,4-triazolin-5-on und 2-Methylsulfonamido-4,5-difluorbenzonitril als Ausgangsstoffe, so läßt sich der Reaktionsablauf des erfindungsgemäßen Verfahrens (a) durch das folgende Formelschema darstellen:

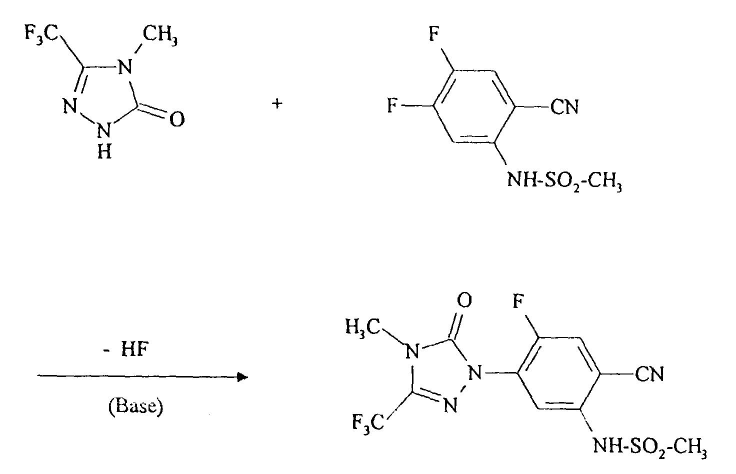

Verwendet man beispielsweise 1-(4-Cyano-2,5-difluorphenyl)-4-methyl-3-trifluormethyl-1,2,4-triazolin-5-on und N-Methyl-methansulfonsäureamid als Ausgangsstoffe, so läßt sich der Reaktionsablauf des erfindungsgemäßen Verfahrens (b) durch das folgende Formelschema darstellen:

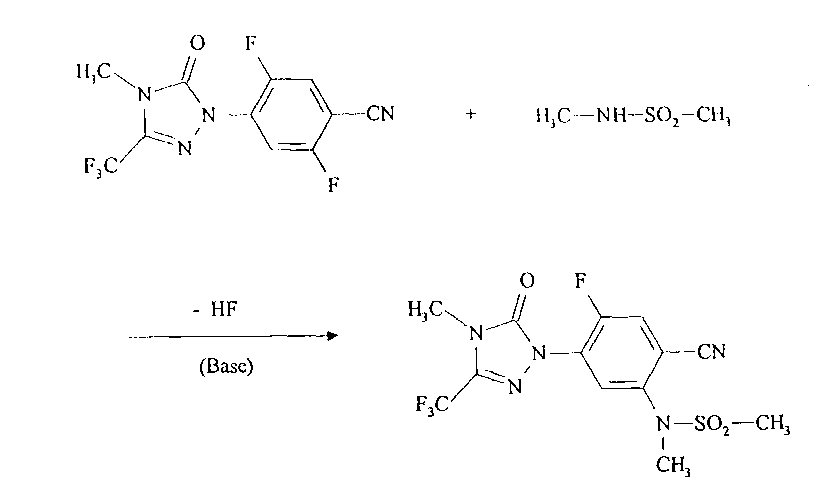

Verwendet man beispielsweise 1-(4-Cyano-2-fluor-5-methansulfonamido-phenyl)-4-amino-3-trifluormethyl-1,2,4-triazolin-5-on und Natriumnitrit als Ausgangsstoffe, so läßt sich der Reaktionsablauf des erfindungsgemäßen Verfahrens (c) durch das folgende Formelschema darstellen:

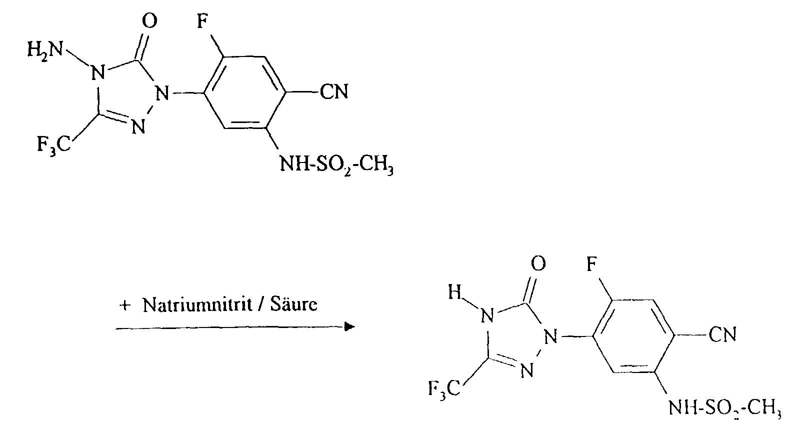

Verwendet man beispielsweise 1-[4-Cyano-2-fluor-5-(N-methyl-methansulfonamido)-phenyl]-3-trifluormethyl-(4H)-1,2,4-triazolin-5-on und Chlordifluormethan als Ausgangsstoffe, so läßt sich der Reaktionsablauf des erfindungsgemäßen Verfahrens (d) durch das folgende Formelschema darstellen:

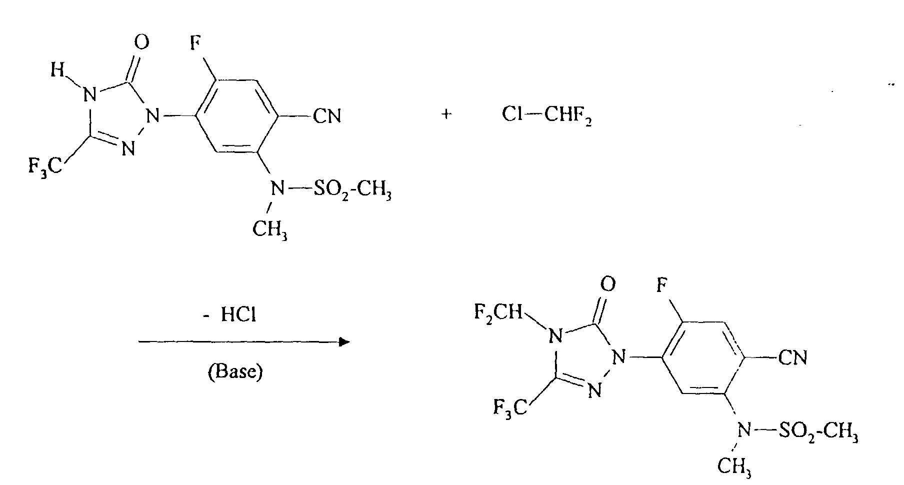

Verwendet man beispielsweise 1-[4-Cyano-2-fluor-5-methansulfonamido-phenyl]-4-methyl-3-trifluormethyl-1,2,4-triazolin-5-on und Iodmethan als Ausgangsstoffe, so läßt sich der Reaktionsablauf des erfindungsgemäßen Verfahrens (e) durch das folgende Formelschema darstellen:

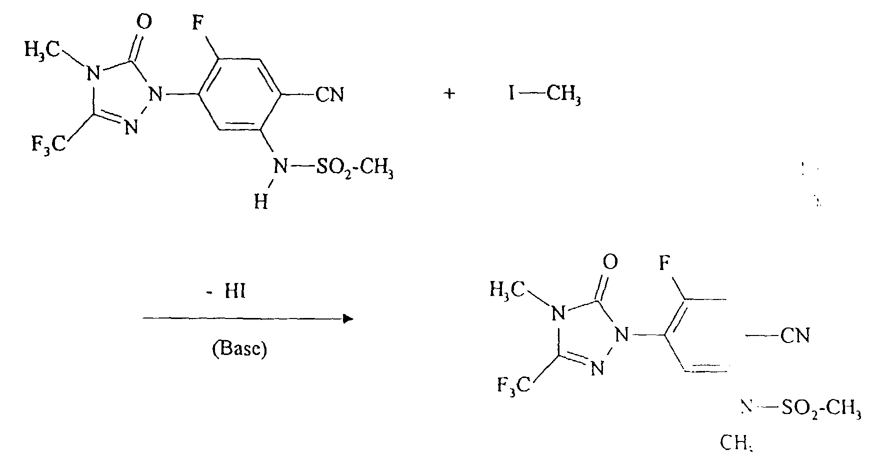

Verwendet man beispielsweise 1-(4-Cyano-2-fluor-5-methylamino-phenyl)-4-methyl-3-trifluormethyl-1,2,4-triazolin-5-on und Methansulfonsäurechlorid als Ausgangsstoffe, so läßt sich der Reaktionsablauf des erfindungsgemäßen Verfahrens (f) durch das folgende Formelschema darstellen:

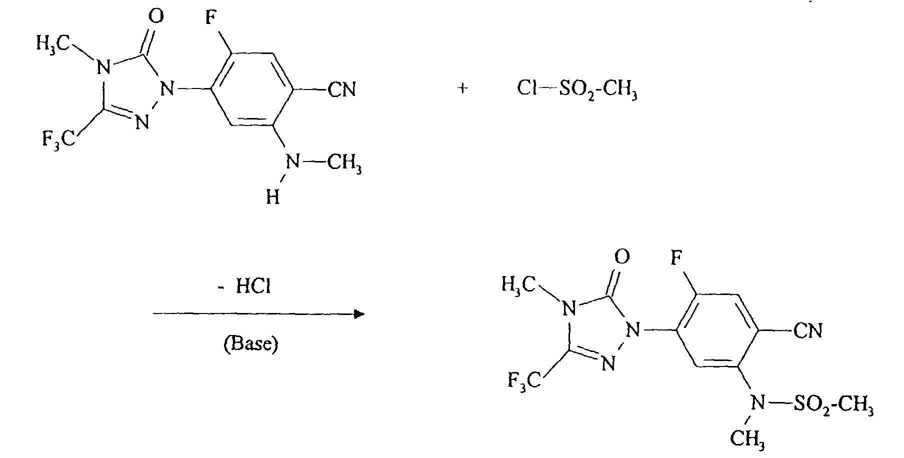

Die zur Durchführung des erfindungsgemäßen Verfahrens (a) als Ausgangsstoffe benötigten 1H-Triazolinone sind durch die Formel (II) allgemein definiert. In dieser Formel (II) stehen R¹, R² und X vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden.

Die 1H-Triazolinone der Formel (II) sind bekannt oder erhältlich in Analogie zu bekannten Verfahren (vergl. z.B. EP 399 294; US 4.477.459; DE 27 16 707; US 3.780.052; J. Med. Chem. 14, 335-338 [1971]; DE 20 29 375). Noch nicht bekannt, jedoch Gegenstand der parallel angemeldeten EP-A-0 597 360, ist die Verbindung 4-Amino-3-trifluormethyl-1H-1,2,4-triazolin-5-on. Man erhält sie, wenn man Hydrazinhydrat zunächst mit Diphenylcarbonat und anschließend mit Trifluoressigsäure bei Temperaturen zwischen -20 C und +200 C umsetzt (vergl. hierzu auch die Herstellungsbeispiele).

Die zur Durchführung des erfindungsgemäßen Verfahrens (a) weiterhin als Ausgangsstoffe benötigten Halogenbenzol-Derivate sind durch die Formel (III) allgemein definiert. In dieser Formel (III) stehen R³, R⁴ und R⁵ vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden. Hal¹ steht vorzugsweise für Fluor, Chlor oder Brom, insbesondere für Fluor oder Chlor.

Die Halogenbenzol-Derivate der Formel (III) sind noch nicht bekannt und ebenfalls Gegenstand der Erfindung. Man erhält sie, wenn man 2-Halogenbenzonitrile der Formel (X), 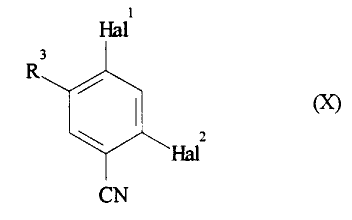 in welcher
Hal¹ und R³ die oben angegebene Bedeutung haben und
Hal² für Halogen steht,
mit Sulfonamiden der Formel (V),

R⁴-NH-SO₂-R⁵ (V)

in welcher
R⁴ und R⁵ die oben angegebene Bedeutung haben,
in Analogie zur Durchführung des erfindungsgemäßen Verfahrens (b) gegebenenfalls in Gegenwart eines Verdünnungsmittels wie beispielsweise Acetonitril und gegebenenfalls in Gegenwart eines Reaktionshilfsmittels wie beispielsweise Kaliumcarbonat bei Temperaturen zwischen -20°C und +120°C umsetzt.

2-Halogenbenzonitrile der Formel (X) sind bekannt oder erhältlich in Analogie zu bekannten Verfahren (vergl. z.B. EP 441 004; EP 431 373). Man erhält 5-Chlor-2,4-difluorbenzonitril, wenn man die bekannte Verbindung 2,4,5-Trichlorbenzonitril (vergl. z.B. EP 441 004) mit Kaliumfluorid gegebenenfalls in Gegenwart eines Verdünnungsmittels, wie beispielsweise Tetramethylensulfon bei Temperaturen zwischen 100°C und 200°C umsetzt (vergl. hierzu auch die Herstellungsbeispiele).

Die zur Durchführung des erfindungsgemäßen Verfahren (b) als Ausgangsprodukte benötigten substituierten Triazolinone sind durch die Formel (IV) allgemein definiert. In dieser Formel (IV) stehen R¹, R², R³ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. Hal² steht vorzugsweise für Fluor, Chlor oder Brom, insbesondere für Fluor oder Chlor.

Die substituierten Triazolinone der Formel (IV) sind noch nicht bekannt. Sie sind jedoch Gegenstand der parallel angemeldeten EP-A-0 597 360 und erhältlich mit Hilfe der dort beschriebenen Verfahren, beispielsweise wenn man 1H-Triazolinone der Formel (II), 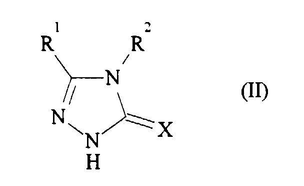 in welcher
R¹, R² und X die oben angegebene Bedeutung haben,
mit 2-Halogenbenzonitrilen der Formel (X), 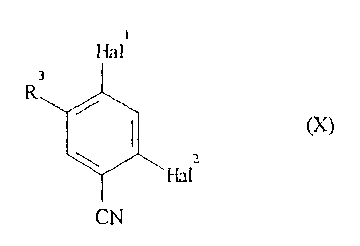 in welcher
Hal¹ und R³ die oben angegebene Bedeutung haben und
Hal² für Halogen steht,
in Analogie zur Durchführung des erfindungsgemäßen Verfahrens (a) gegebenenfalls in Gegenwart eines Verdünnungsmittels wie beispielsweise Acetonitril und gegebenenfalls in Gegenwart eines Reaktionshilfsmittels wie beispielsweise Kaliumcarbonat bei Temperaturen zwischen -20 C und +120 C umsetzt.

Die zur Durchführung des erfindungsgemäßen Verfahrens (b) weiterhin als Edukte benötigten Sulfonamide sind durch die Formel (V) allgemein definiert. In dieser Formel (V) stehen R⁴ und R⁵ vorzugsweise für diejenigen Reste, die bereits im Zusammenhang mit der Beschreibung der erfindungsgemäßen Stoffe der Formel (I) als bevorzugt und besonders bevorzugt für diese Substituenten genannt wurden.

Die Sulfonamide der Formel (V) sind allgemein bekannte Verbindungen der organischen Chemie.

Die zur Durchführung des erfindungsgemäßen Verfahrens (c) als Edukte benötigten substituierten Triazolinone sind durch die Formel (Ia) allgemein definiert. In dieser Formel (Ia) stehen R¹, R³, R⁴, R⁵ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. R²⁻¹ steht vorzugsweise für Amino.

Die substituierten Triazolinone der Formel (1a) sind erfindungsgemäße Verbindungen und erhältlich mit Hilfe der erfindungsgemäßen Verfahren (a), (b), (e) und/oder (f).

Die zur Durchführung des erfindungsgemäßen Verfahrens (d) als Edukte benötigten substituierten Triazolinone sind durch die Formel (Ib) allgemein definiert. In dieser Formel (Ib) stehen R¹, R³, R⁴, R⁵ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. R²⁻² steht vorzugsweise für Wasserstoff.

Die substituierten Triazolinone der Formel (Ib) sind erfindungsgemäße Verbindungen und erhältlich mit Hilfe der erfindungsgemäßen Verfahren (a), (b), (c), (e) und/oder (f).

Die zur Durchführung des erfindungsgemäßen Verfahrens (d) weiterhin als Edukte benötigten Alkylierungsmittel sind durch die Formel (VI) allgemein definiert. In dieser Formel (VI) steht R²⁻³ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für den Substituenten R⁶ genannt wurden mit Ausnahme der gegebenenfalls substituierten Arylreste. E¹ steht vorzugsweise für einen bei Alkylierungsmitteln üblichen Abgangsrest, wie beispielsweise Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (VI) sind allgemein bekannte Verbindungen der organischen Chemie.

Die zur Durchführung des erfindungsgemäßen Verfahrens (e) als Edukte benötigten substituierten Triazolinone sind durch die Formel (Ic) allgemein definiert. In dieser Formel (Ic) stehen R¹, R², R³, R⁵ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. Die substituierten Triazolinone der Formel (Ic) sind erfindungsgemäße Verbindungen und erhältlich mit Hilfe der erfindungsgemäßen Verfahren (a), (b), (c), (d) und/oder (f).

Die zur Durchführung des erfindungsgemäßen Verfahrens (e) weiterhin als Edukte benötigten Alkylierungsmittel sind durch die Formel (VII) allgemein definiert. In dieser Formel (VII) steht R⁴⁻¹ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für den Substituenten R⁶ genannt wurden mit Ausnahme der gegebenenfalls substituierten Arylreste. E² steht vorzugsweise für einen bei Alkylierungsmitteln üblichen Abgangsrest, wie beispielsweise Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (VII) sind allgemein bekannte Verbindungen der organischen Chemie.

Die zur Durchführung des erfindungsgemäßen Verfahrens (f) als Edukte benötigten substituierten Triazolinone sind durch die Formel (VIII) allgemein definiert. In dieser Formel (VIII) stehen R¹, R², R³, R⁴ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden.

Die substituierten Triazolinone der Formel (VIII) sind noch nicht bekannt. Sie sind jedoch teilweise Gegenstand der parallel angemeldeten EP-A-0 597 360 und erhältlich mit Hilfe der dort beschriebenen Verfahren, beispielsweise wenn man substituierte Triazolinone der Formel (IV), 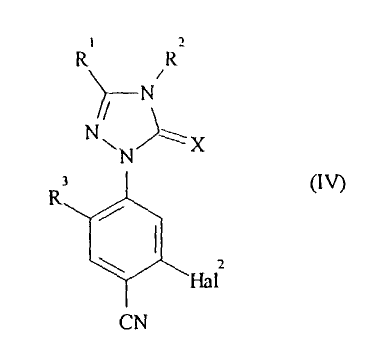 in welcher
R¹, R², R³ und X die oben angegebene Bedeutung haben und
Hal² für Halogen steht,
mit Aminen der Formel (XI),

R⁴-NH₂ (XI)

in welcher
- R⁴: die oben angegebene Bedeutung hat,
gegebenenfalls in Gegenwart eines Verdünnungsmittels wie beispielsweise Acetonitril und gegebenenfalls in Gegenwart eines Reaktionshilfsmittels wie beispielsweise Kaliumcarbonat bei Temperaturen zwischen -20 C und +120 C umsetzt.

Amine der Formel (XI) sind allgemein bekannte Verbindungen der organischen Chemie.

Die zur Durchführung des erfindungsgemäßen Verfahrens (f) weiterhin als Edukte benötigten Sulfonsäurehalogenide sind durch die Formel (IX) allgemein definiert. In dieser Formel (IX) steht R⁵ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diesen Substituenten genannt wurden. Hal³ steht vorzugsweise für Fluor, Chlor oder Brom, insbesondere für Chlor oder Brom.

Die Sulfonsäurehalogenide der Formel (IX) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder azabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 C und + 180 C, vorzugsweise bei Temperaturen zwischen +20 C und +120 C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol 1H-Triazolinon der Formel (II) im allgemeinen 1,0 bis 3,0 Mol, vorzugsweise 1,0 bis 1,5 Mol Halogenbenzol-Derivat der Formel (III) und gegebenenfalls 1,0 bis 3,0 Mol, vorzugsweise 1,0 bis 1,5 Mol Base als Reaktionshilfsmittel ein. Die Reaktionsdurchführung, Aufarbeitung und Isolierung der Reaktionsprodukte erfolgt nach allgemein üblichen, bekannten Verfahren.

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Das erfindungsgemäße Verfahren (b) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20 C und + 150 C, vorzugsweise bei Temperaturen zwischen 0 C und +120 C.

Das erfindungsgemäße Verfahren (b) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (b) setzt man pro Mol substituiertem Triazolinon der Formel (IV) im allgemeinen 1,0 bis 3,0 Mol, vorzugsweise 1,0 bis 1,5 Mol Sulfonamid der Formel (V) und gegebenenfalls 0,1 bis 3,0 Mol, vorzugsweise 1,0 bis 1,5 Mol Base als Reaktionshilfsmittel ein.

Die Reaktionsdurchführung, Aufarbeitung und Isolierung der Reaktionsprodukte erfolgt nach allgemein üblichen, bekannten Verfahren (vergl. hierzu auch die Herstellungsbeispiele).

Das erfindungsgemäße Verfahren (c) wird üblicherweise in Gegenwart einer geeigneten Säure durchgeführt. Als solche kommen insbesondere wässrige Mineralsäuren infrage. Mit besonderem Vorzug verwendet man verdünnte Salzsäure.

Als Verdünnungsmittel zur Durchführung des erfindungsgemäßen Verfahrens (c) kommen alle für derartigen Diazotierungsreaktionen üblichen Verdünnungsmittel in Betracht. Mit besonderem Vorzug verwendet man die als Reagenzien eingesetzten wässrigen Mineralsäuren, wie beispielsweise Salzsäure in entsprechendem Überschuß gleichzeitig als Verdünnungsmittel.

Die Reaktionstemperaturen können bei der Durchführung des erfindungsgemäßen Verfahrens (c) in einem größeren Bereich variiert werden. Im allgemeinen arbeitet man bei Temperaturen zwischen -20 C und + 100 C, vorzugsweise bei Temperaturen zwischen -10 C und +80 C.

Das erfindungsgemäße Verfahren (c) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (c) setzt man pro Mol substituiertem Triazolinon der Formel (Ia) im allgemeinen 1,0 bis 3,0 Mol, vorzugsweise 1,0 bis 2,0 Mol Natriumnitrit und 1,0 bis 10,0 Mol, vorzugsweise 1,0 bis 5,0 Mol Säure ein.

Die Reaktionsdurchführung, Aufarbeitung und Isolierung der Reaktionsprodukte erfolgt nach allgemein üblichen, bekannten Verfahren.

Als Verdünnungsmittel zur Durchführung der erfindungsgemäßen Verfahren (d) und (e) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Diisopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Die erfindungsgemäßen Verfahren (d) und (e) können gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die erfindungsgemäßen Verfahren (d) und (e) werden vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder cycloundecen (DBU).

Die Reaktionstemperaturen können bei der Durchführung der erfindungsgemäßen Verfahren (d) und (e) in einem größeren Bereich variiert werden. Im allgemeinen arbeitet man bei Temperaturen zwischen -20 C und +150 C, vorzugsweise bei Temperaturen zwischen 0 C und +120 C.

Die erfindungsgemäßen Verfahren (d) und (e) werden üblicherweise unter Normaldruck durchgeführt. Es ist jedoch auch möglich, unter erhöhtem oder vermindertem Druck zu arbeiten.

Zur Durchführung des erfindungsgemäßen Verfahrens (d) setzt man pro Mol substituiertem Triazolinon der Formel (Ib) im allgemeinen 1,0 bis 3,0 Mol, vorzugsweise 1,0 bis 2,0 Mol Alkylierungsmittel der Formel (VI) und gegebenenfalls 1,0 bis 3,0 Mol, vorzugsweise 1,0 bis 2,0 Mol Base als Reaktionshilfsmittel ein.

Zur Durchführung des erfindungsgemäßen Verfahrens (e) setzt man pro Mol an substituiertem Triazolinon der Formel (Ic) im allgemeinen 1,0 bis 3,0 Mol, vorzugsweise 1,0 bis 2,0 Mol an Alkylierungsmittel der Formel (VII) und gegebenenfalls 1,0 bis 3,0 Mol, vorzugsweise 1,0 bis 2,0 Mol an als Reaktionshilfsmittel verwendter Base ein.

Die Reaktionsdurchführung, Aufarbeitung und Isolierung der Reaktionsprodukte erfolgt in beiden Fällen nach allgemein üblichen, bekannten Verfahren.

Als Verdünnungsmittel zur Durchführung des erfindungsgemäßen Verfahrens (f) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester.

Das erfindungsgemäße Verfahren (f) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder azabicycloundecen (DBU).

Die Reaktionstemperaturen können bei der Durchführung des erfindungsgemäßen Verfahrens (f) in einem größeren Bereich variiert werden. Im allgemeinen arbeitet man bei Temperaturen zwischen 0 C und +180 C, vorzugsweise bei Temperaturen zwischen +20 C und +120 C.

Das erfindungsgemäße Verfahren (f) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (f) setzt man pro Mol substituiertem Triazolinon der Formel (VIII) im allgemeinen 1,0 bis 3,0 Mol, vorzugsweise 1,0 bis 1,5 Mol Sulfonsäurehalogenid der Formel (IX) und gegebenenfalls 1,0 bis 3,0 Mol, vorzugsweise 1,0 bis 1,5 Mol Base als Reaktionshilfsmittel ein. Die Reaktionsdurchführung, Aufarbeitung und Isolierung der Reaktionsprodukte erfolgt nach allgemein üblichen, bekannten Verfahren.

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe der Protonen-Kernresonanzspektroskopie (¹H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern einsetzen.

Die Wirkstoffe können in Abhängigkeit von ihren jeweiligen physikalischen und/oder chemischen Eigenschaften in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infragen: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können dabei als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

### Herstellungsbeispiele:

### Beispiel 1:

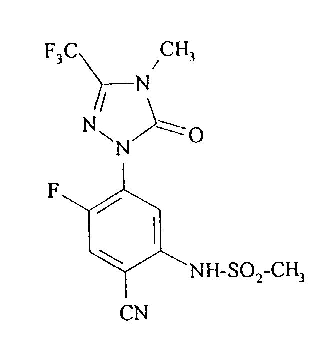

### (Verfahren b)

Zu 1,52 g (0,005 Mol) 1-(4-Cyano-2,5-difluorphenyl)-4-methyl-3-trifluormethyl-1,2,4-triazolin-5-on und 0,48 g (0,005 Mol) Methansulfonsäureamid in 50 ml Dimethylsulfoxid gibt man bei Raumtemperatur 0,83 g (0,006 Mol) Kaliumcarbonat und erwärmt anschließend für 12 Stunden auf 120 C. Zur Aufarbeitung wird die abgekühlte Reaktionsmischung in Wasser gegeben, mit verdünnter Salzsäure auf pH 2 gebracht und mehrfach mit Dichlormethan extrahiert. Die vereinigten organischen Phasen werden über Natriumsulfat getrocknet und im Vakuum eingeengt. Der Rückstand wird über Kieselgel (Laufmittel: Dichlormethan/Methanol 20:1) chromatographiert.

Man erhält 0,55 g (28 % der Theorie) 1-(4-Cyano-2-fluor-5-methylsulfonylaminophenyl)-4-methyl-3-trifluormethyl-1,2,4-triazolin-5-on mit Schmelzpunkt 67 C.

### Herstellung der Ausgangsverbindungen:

### Beispiel IV-1:

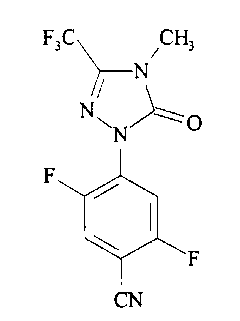

Zu 6,3 g (0,034 Mol) 4-Methyl-3-trifluormethyl-1,2,4-triazolin-5-on (vergl. z.B. US 3.780.052) und 5,4 g (0,034 Mol) 2,4,5-Trifluorbenzonitril in 150 ml Dimethylsulfoxid gibt man bei Raumtemperatur 5,8 g (0,042 Mol) Kaliumcarbonat und erwärmt anschließend für 14 Stunden auf 100°C. Zur Aufarbeitung wird die abgekühlte Reaktionsmischung in Wasser gegeben, mit verdünnter Salzsäure auf pH 2 gebracht und mehrfach mit Dichlormethan extrahiert. Die vereinigten organischen Phasen werden über Natriumsulfat getrocknet und im Vakuum eingeengt. Der Rückstand wird über Kieselgel (Laufmittel: Dichlormethan) chromatographiert.

Man erhält 6,2 g (60 % der Theorie) 1-(4-Cyano-2,5-difluorphenyl)-4-methyl-3-trifluormethyl-1,2,4-triazolin-5-on mit Schmelzpunkt 74°C.

### Beispiel X-1:

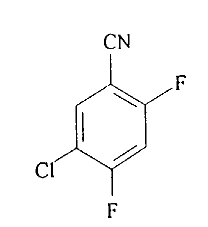

Zu 250 g (4,31 Mol) Kaliumfluorid in 400 ml destilliertem Tetramethylensulfon gibt man unter Rühren bei Raumtemperatur 220 g (1,06 Mol) 2,4,5-Trichlorbenzonitril (vergl. z.B. EP 441 004) und rührt anschließend 10 Stunden bei 195 C bis 200 C. Zur Aufarbeitung wird abgekühlt, 500 ml Wasser zugegeben und die Mischung einer Wasserdampfdestillation unterzogen. Der organische Anteil wird in Dichlormethan aufgenommen, über Natriumsulfat getrocknet, im Vakuum eingeengt und destilliert.

Man erhält 108 g (58 % der Theorie) 2,4-Difluor-5-chlorbenzonitril mit Siedepunkt 105-107 C bei 30 mbar und mit Schmelzpunkt 48-50 C.

In entsprechender Weise und gemäß den allgemeinen Angaben zur Herstellung erhält man die folgenden substituierten Triazolinone der allgemeinen Formel (I):

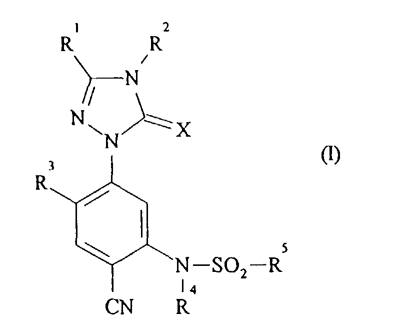

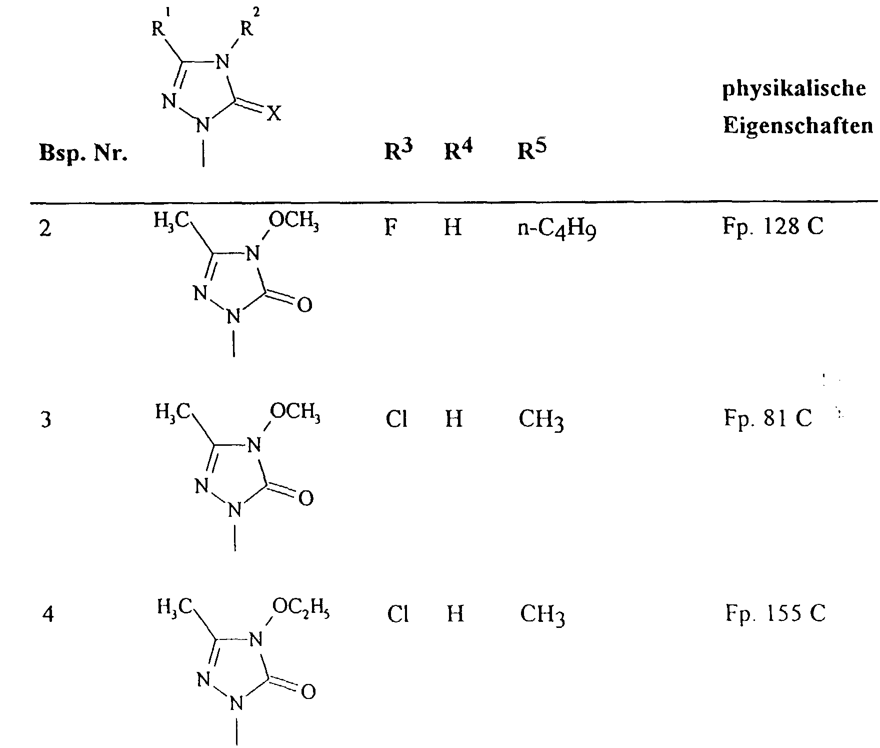

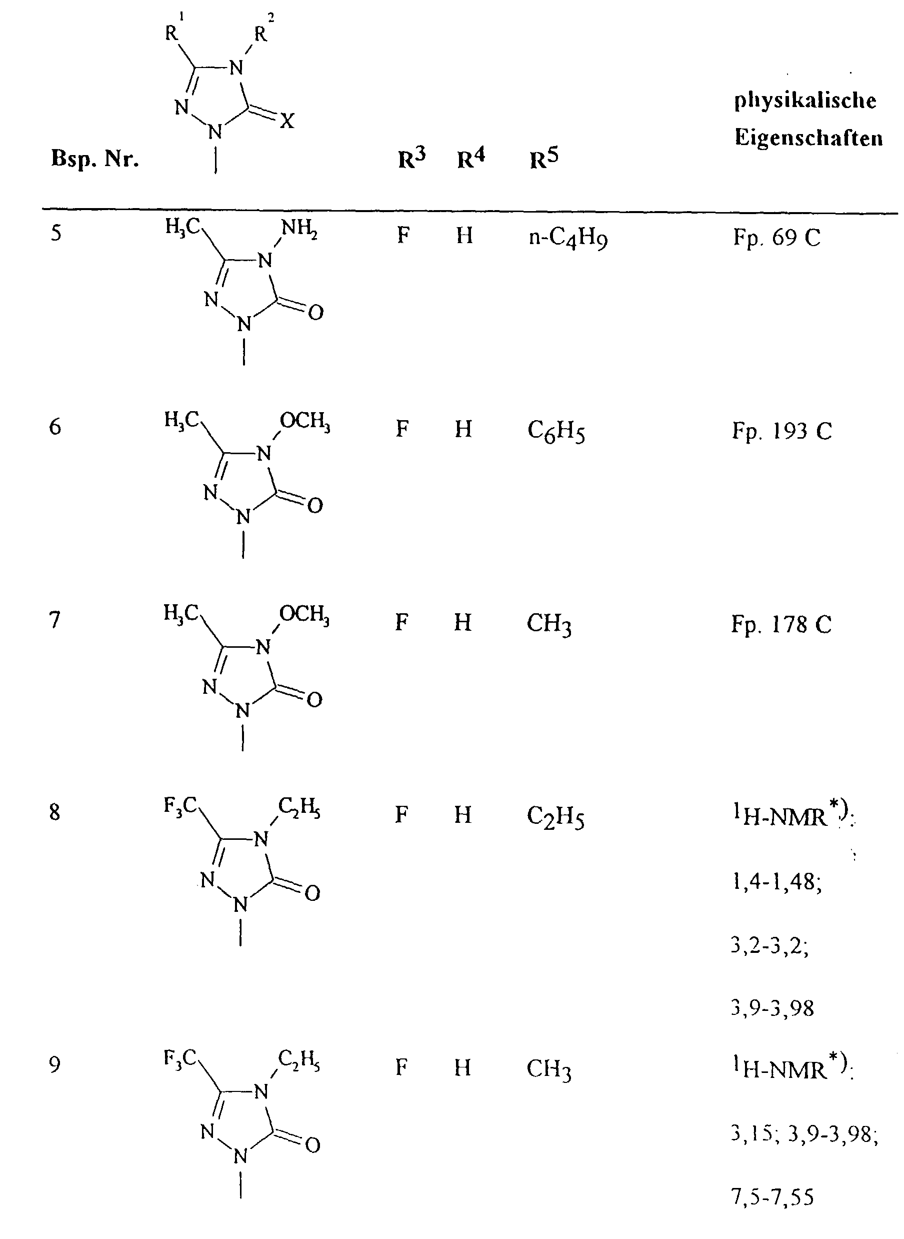

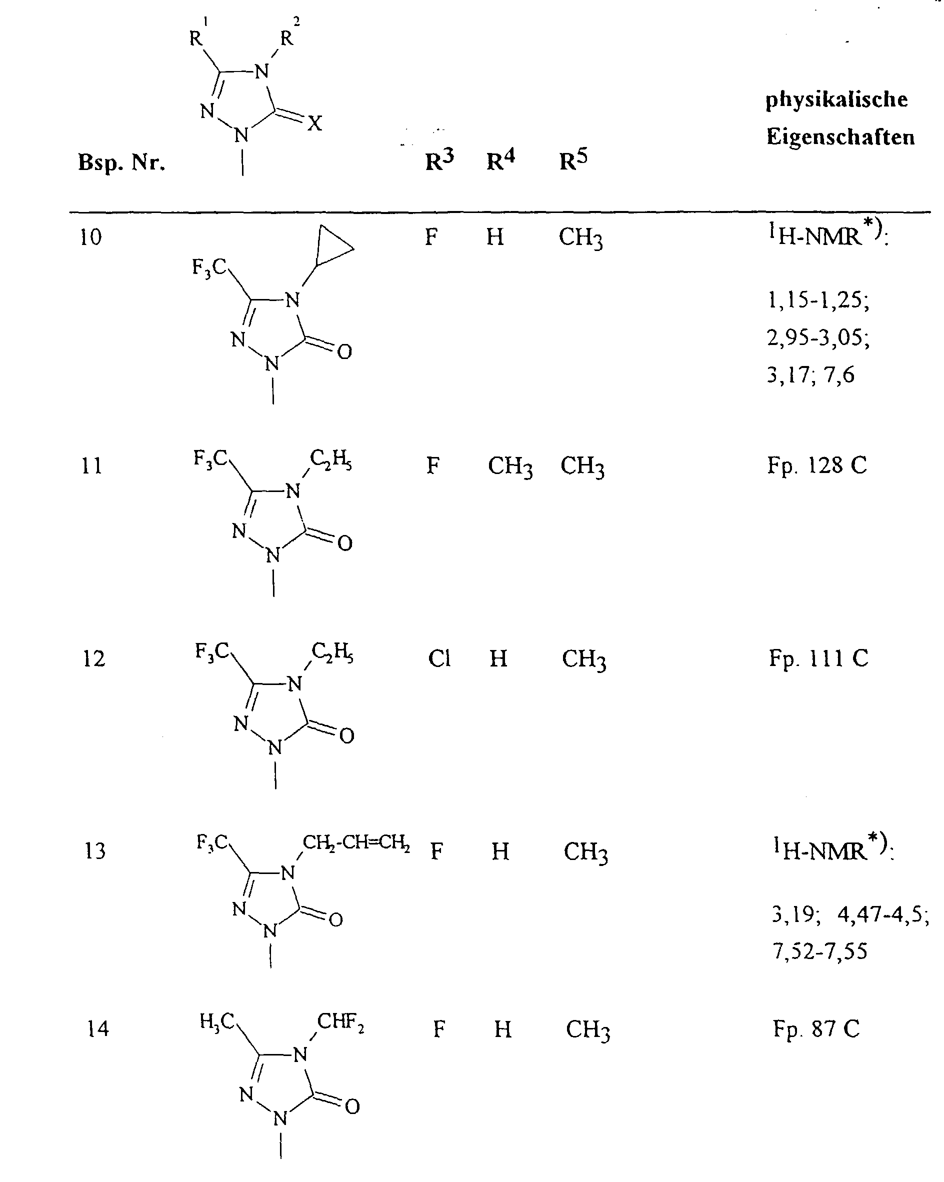

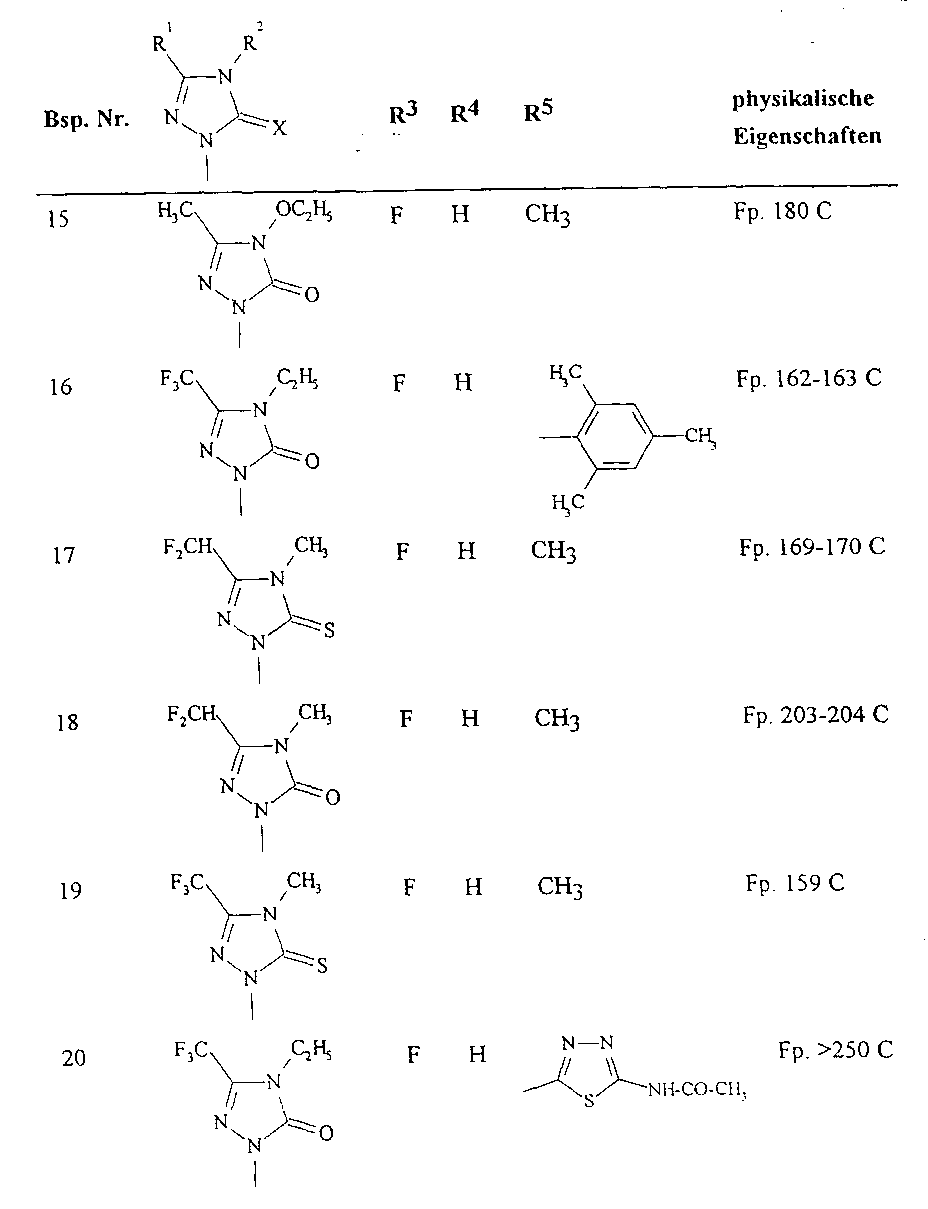

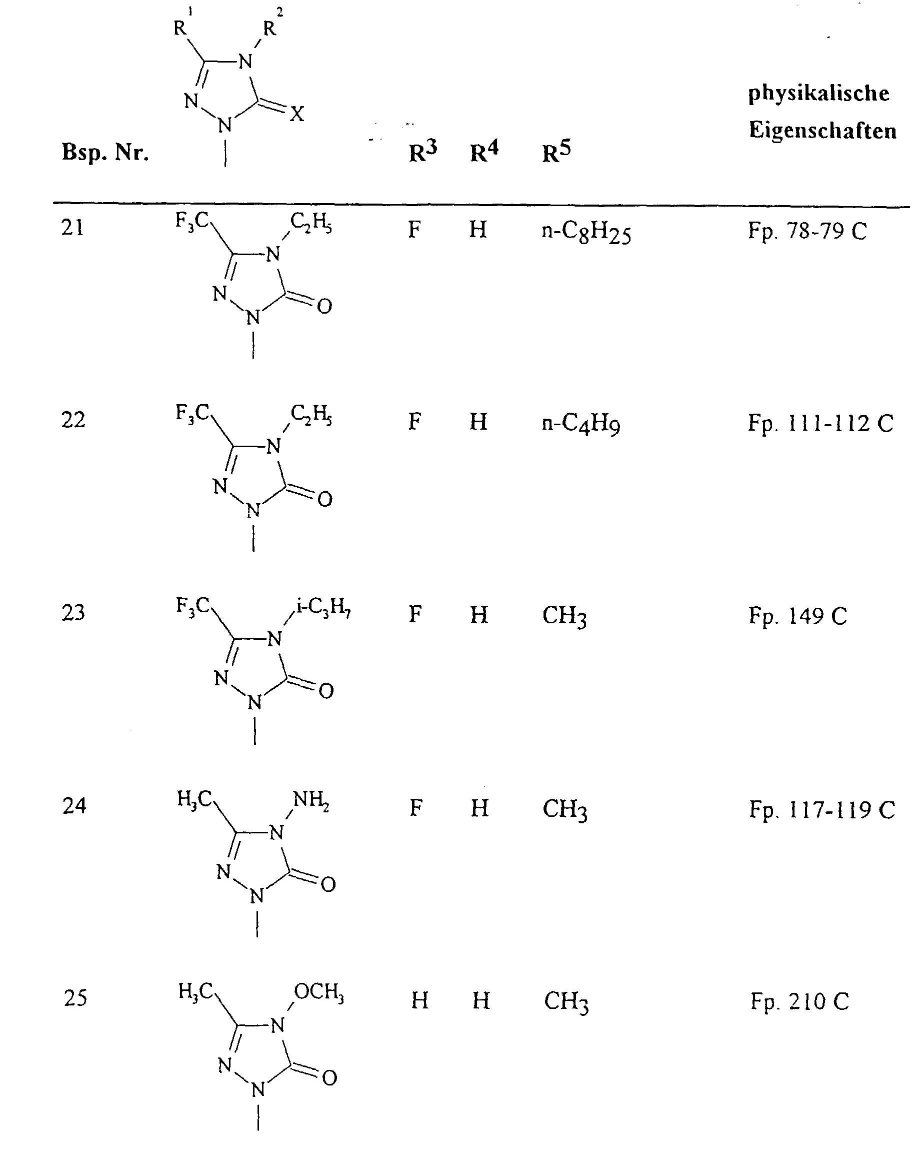

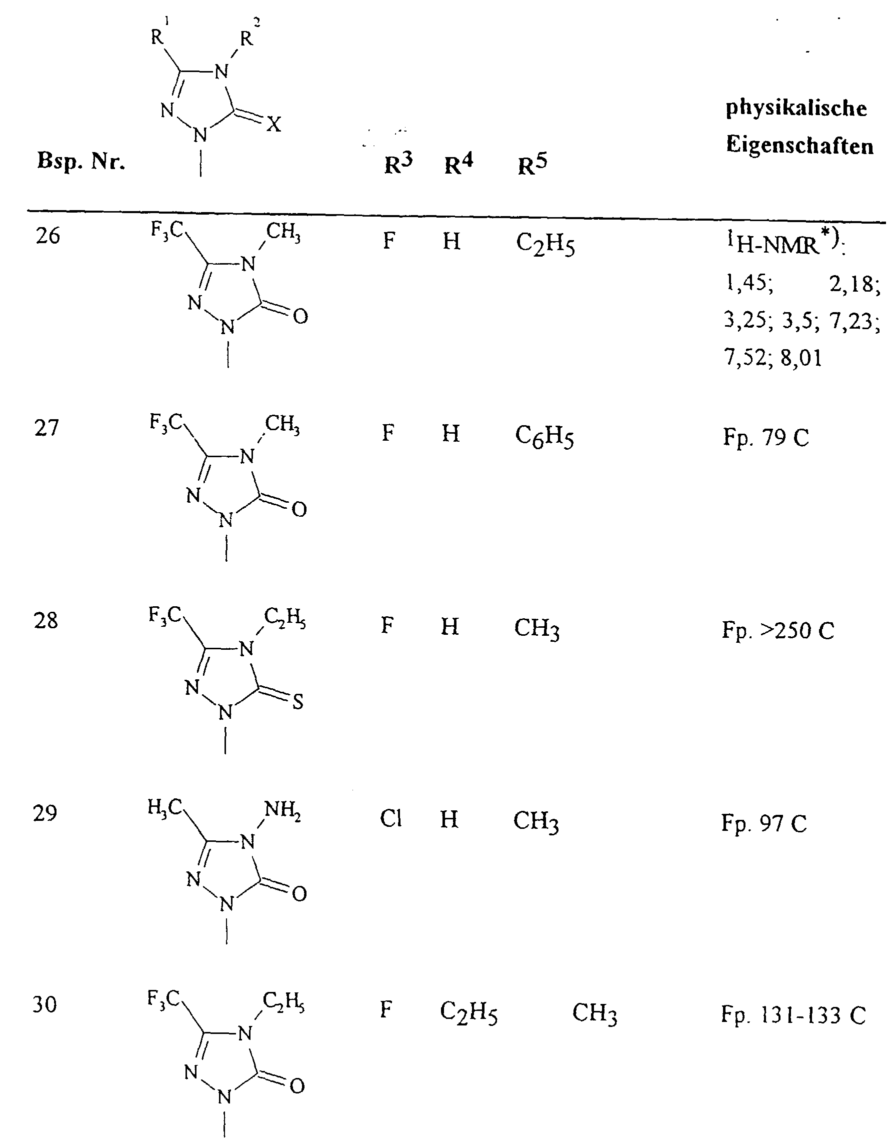

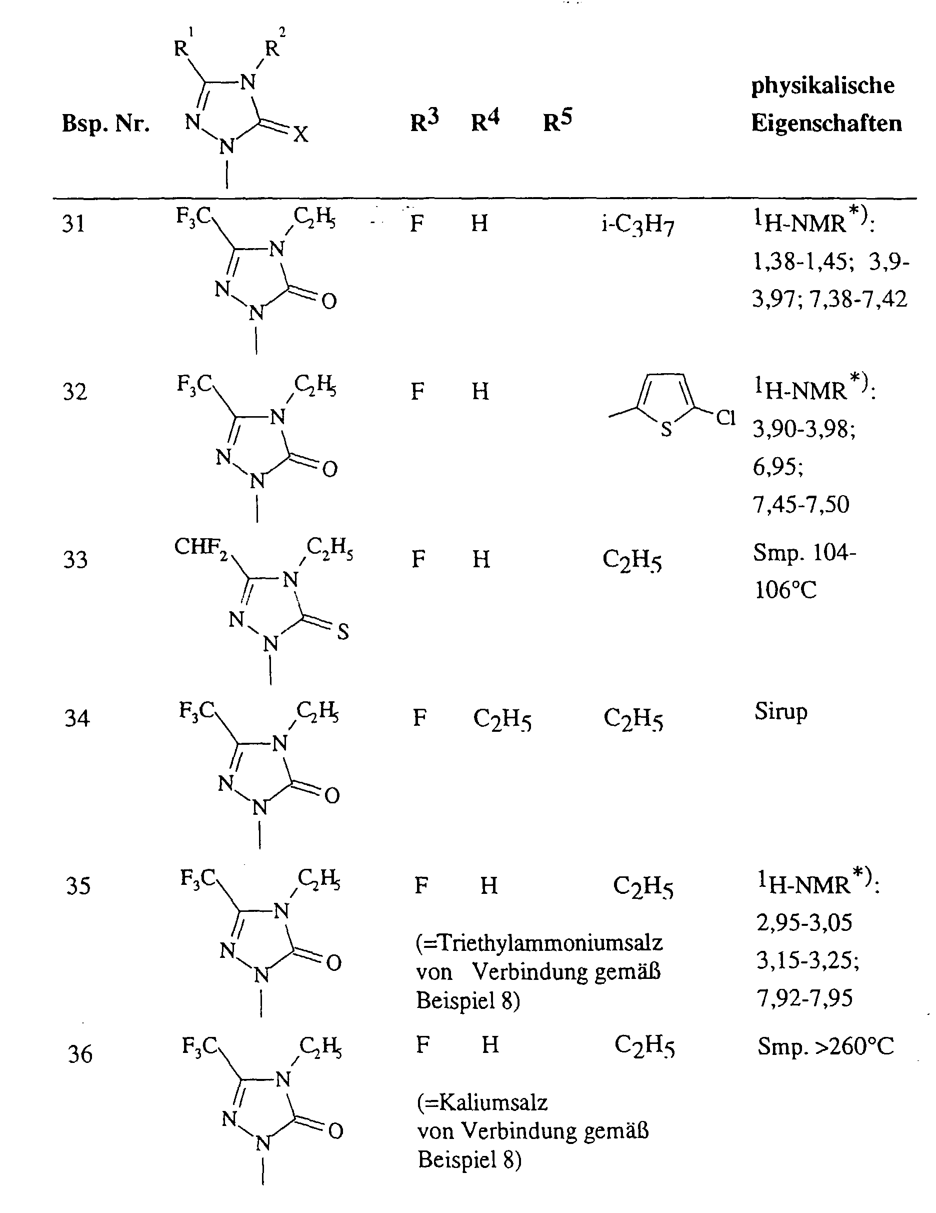

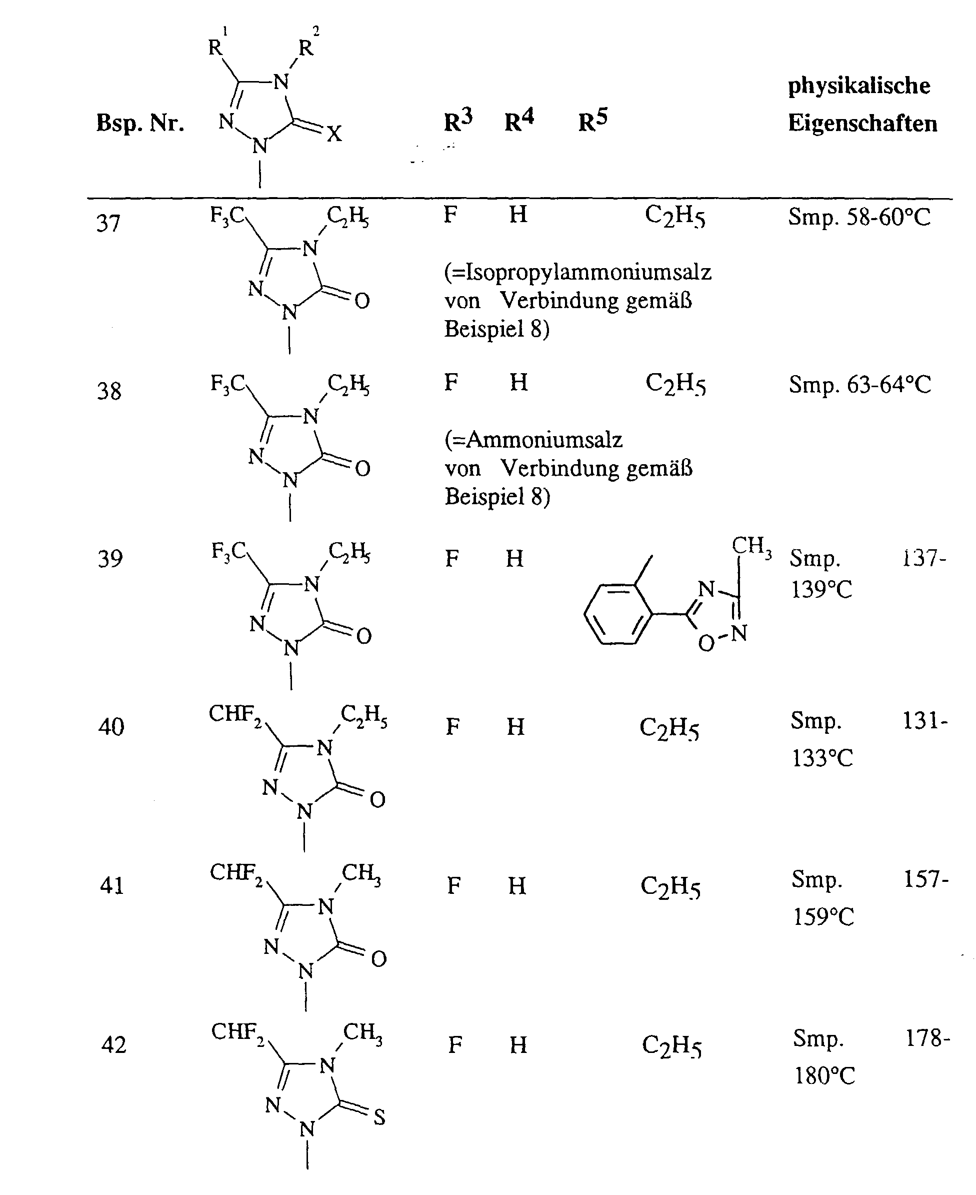

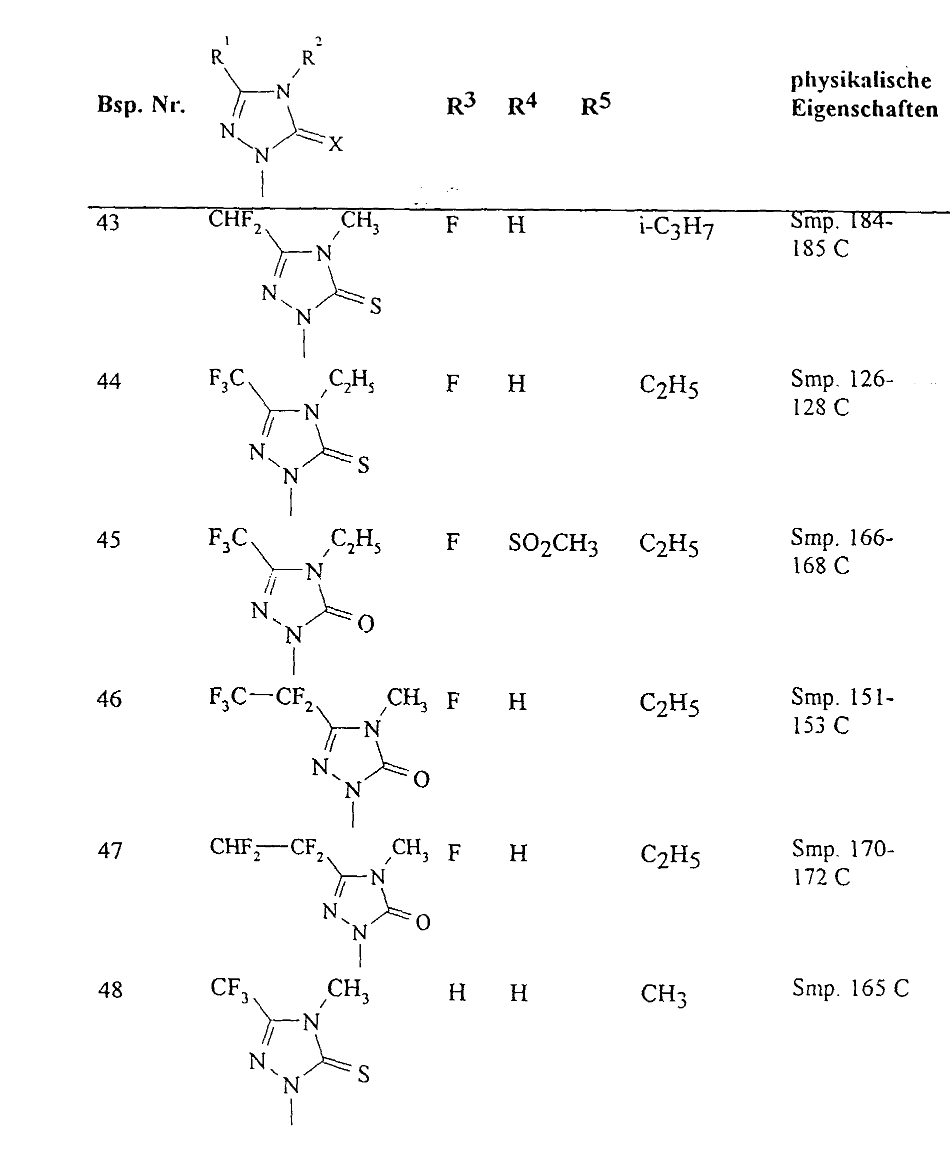

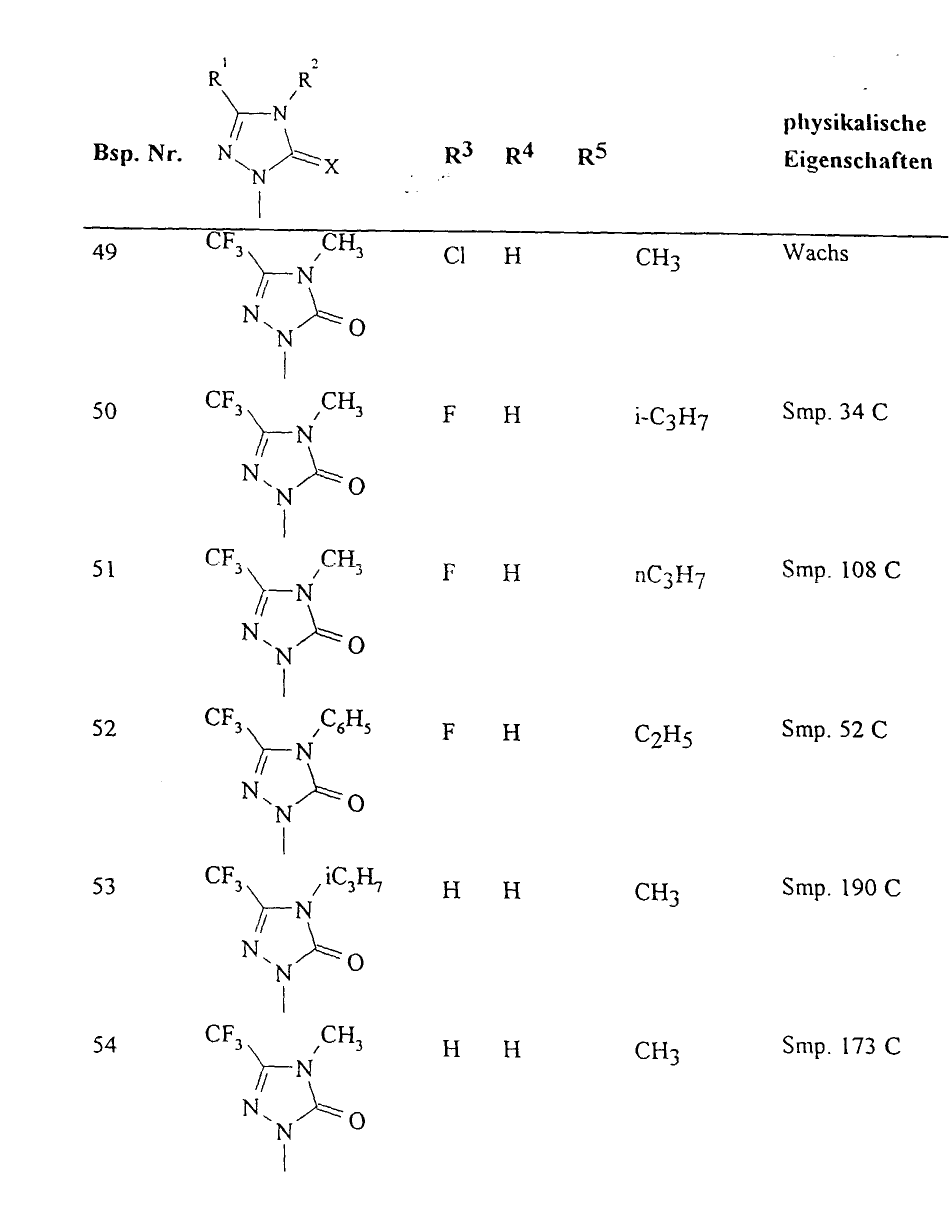

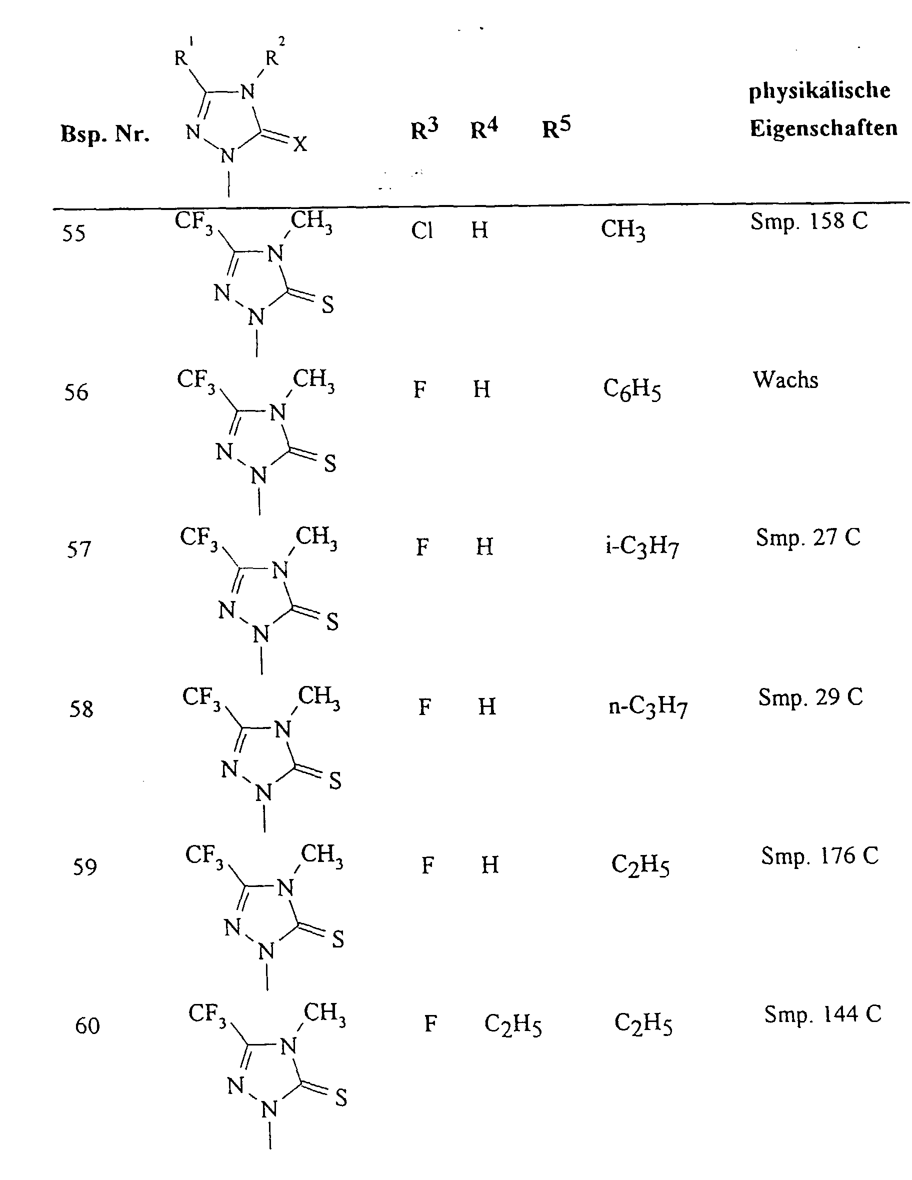

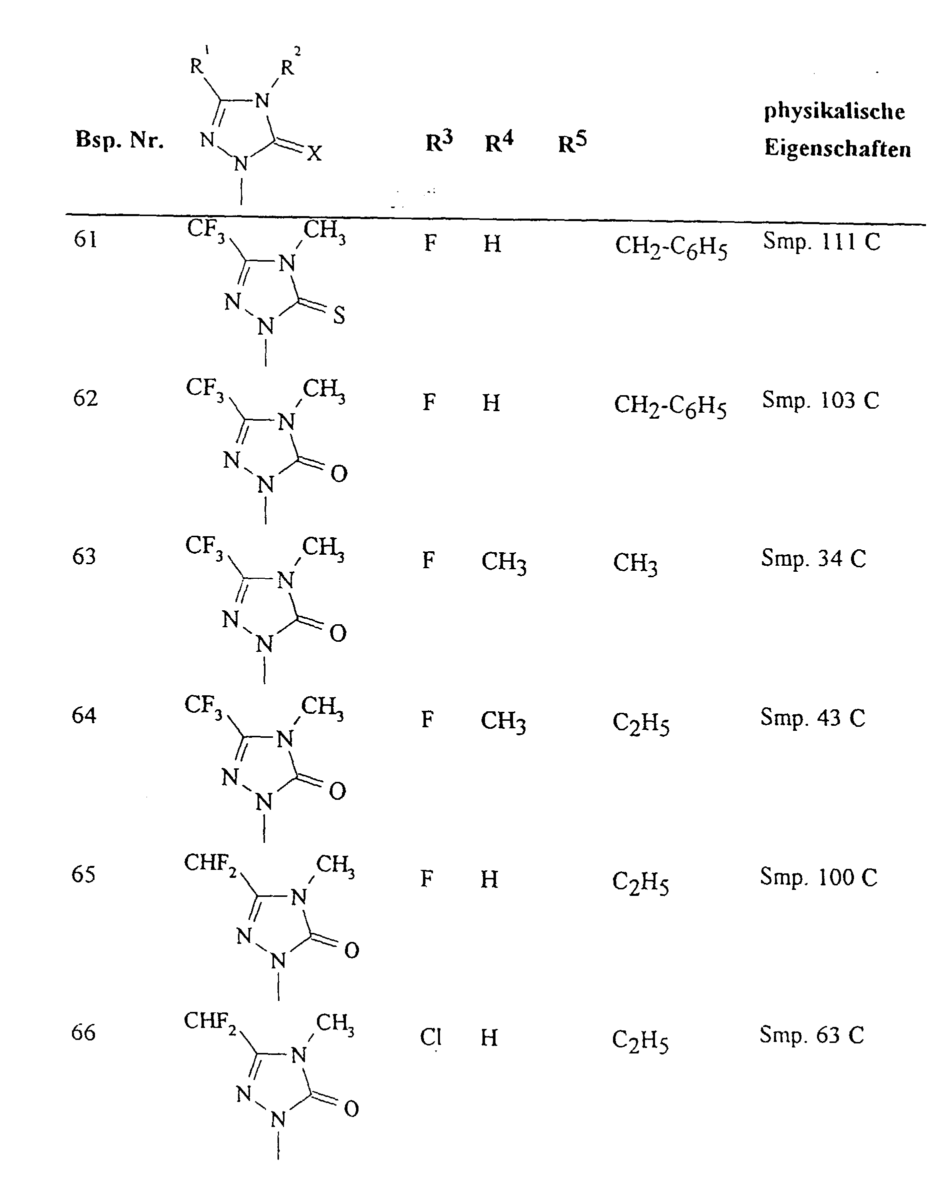

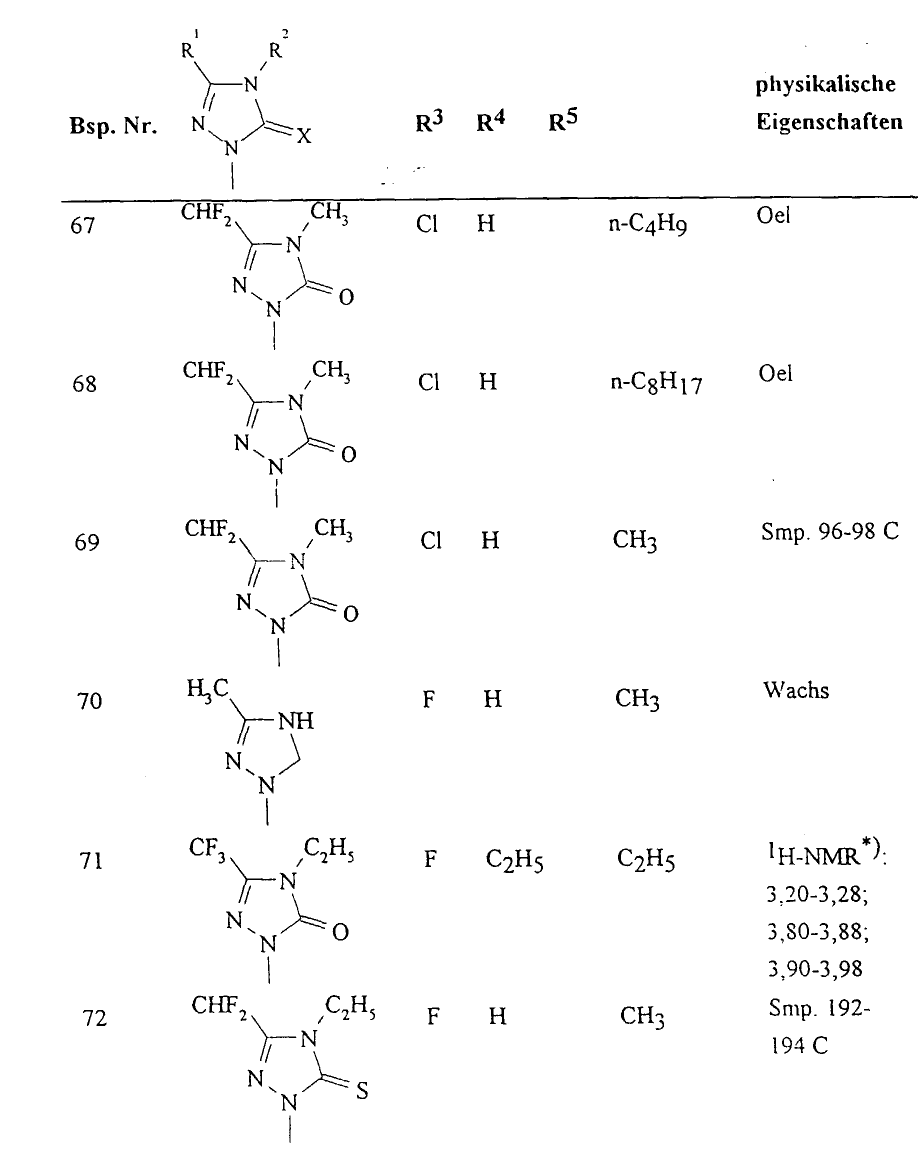

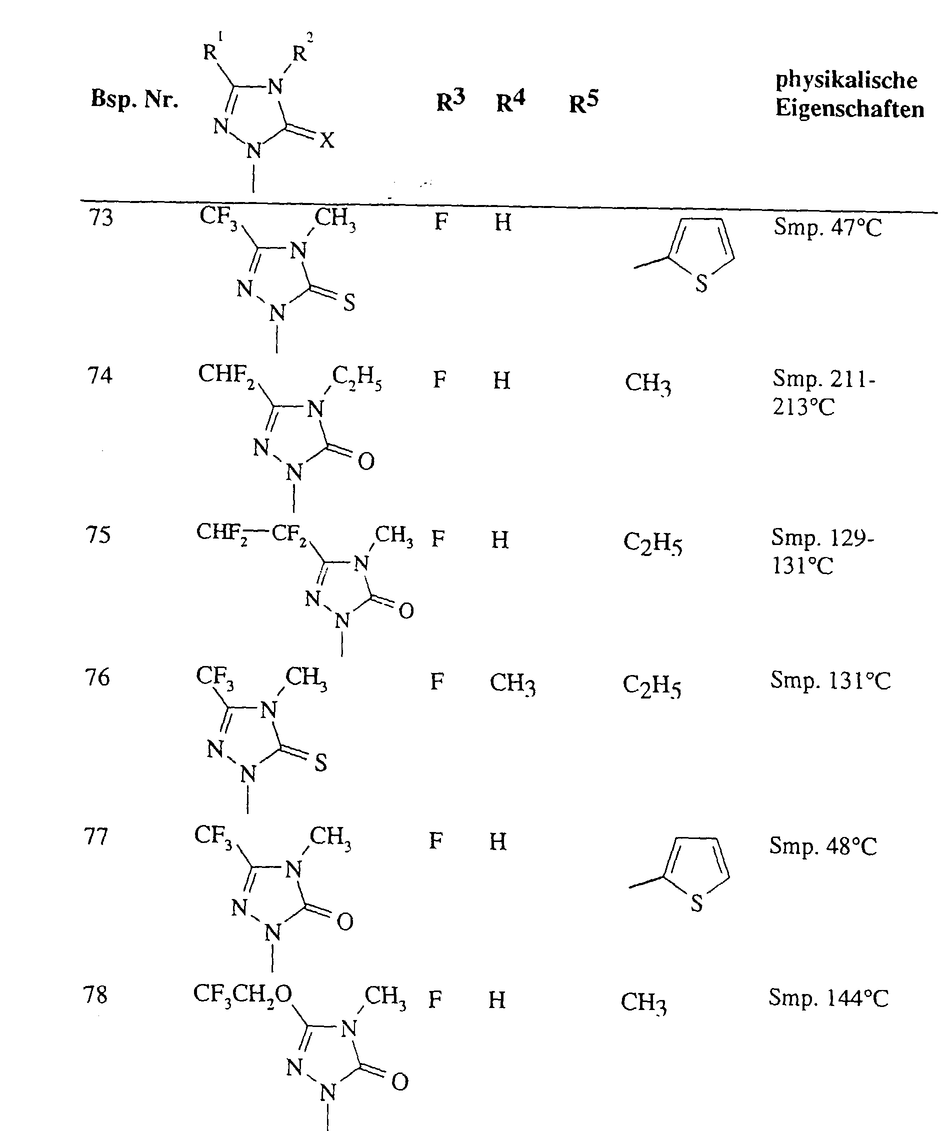

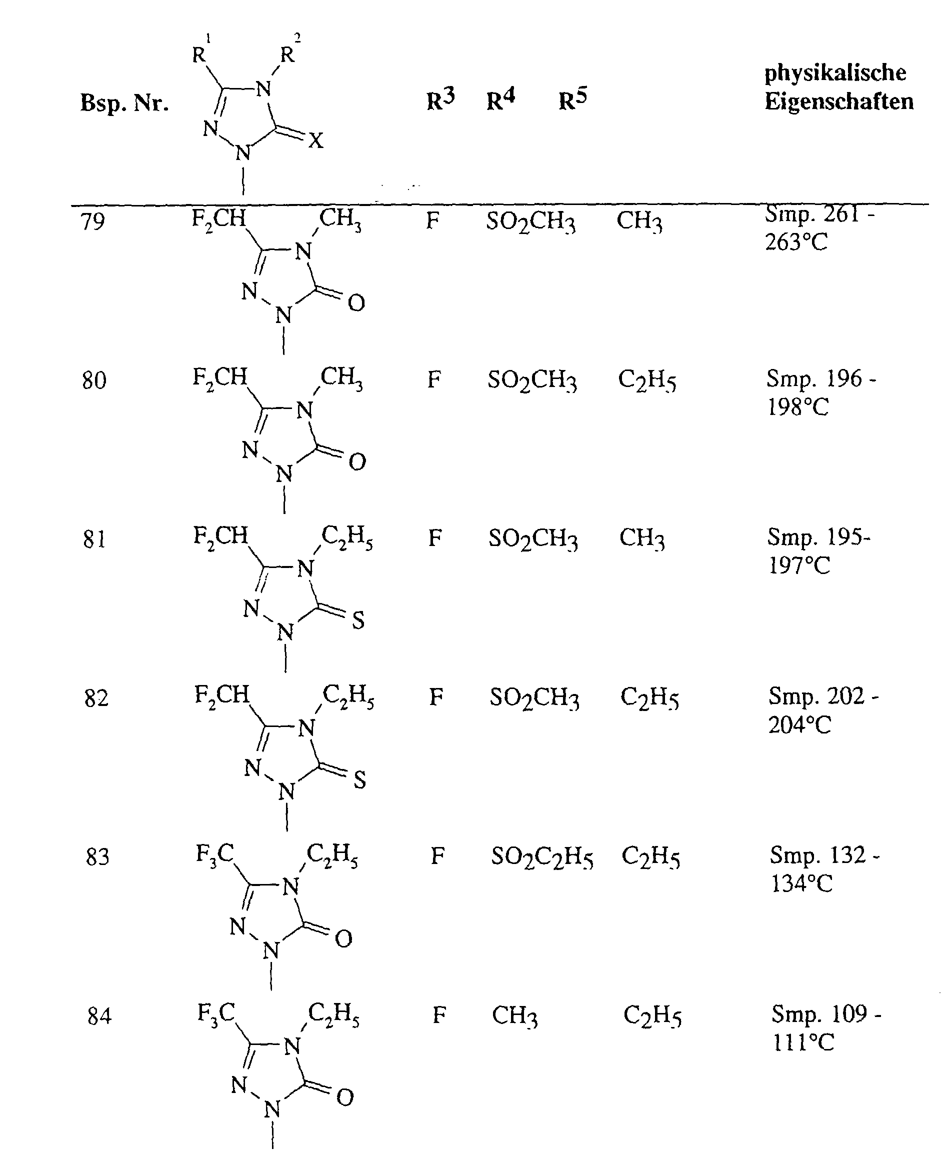

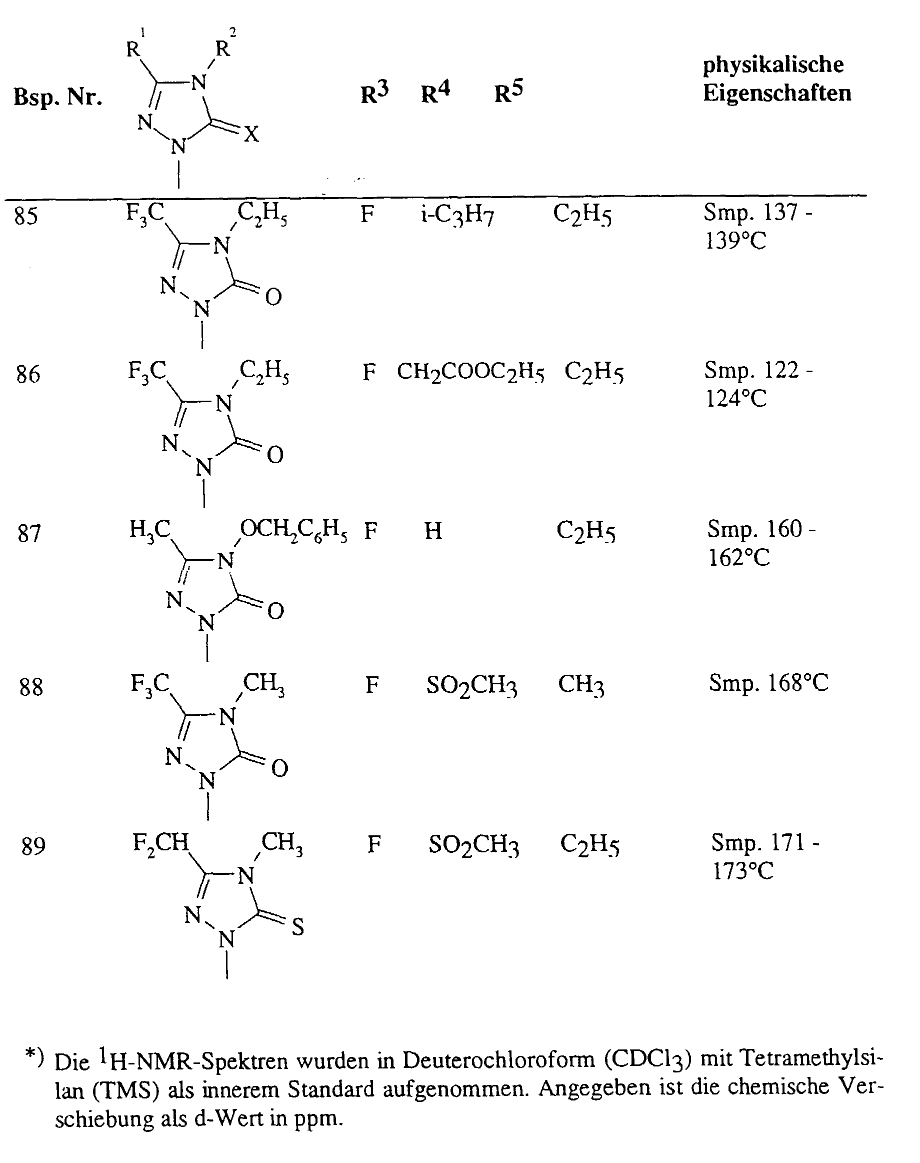

### Anwendungsbeispiele:

In dem folgenden Anwendungsbeispiel wurde die nachstehend aufgeführte Verbindung als Vergleichssubstanz eingesetzt: 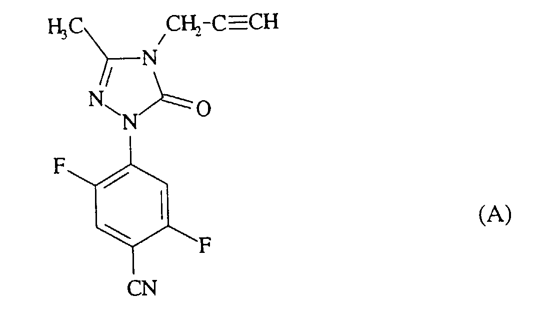 3-Methyl-4-propargyl-1-(2,5-difluor-4-cyano-phenyl)-1,2,4-triazolin-5-on (bekannt aus DE 38 39 480)

### Beispiel A:

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 3 und 8. Dabei zeigen diese Verbindung gegenüber Problemunkräutern wie Abuthilon, Cassia, Chenopodium, Galinsoga, Matricaria und Portulaca eine Wirksamkeit von 80 bis 100 % bei Aufwandmengen von 250 bis 500 g/ha, wohingegen der Stand der Technik in Form von Verbindung (A) aus der DE 3 839 480 bei einer Aufwandmenge von 500 g/ha zumeist gar keine und lediglich bei Galinsoga 70 % und bei Matricaria 20 % herbizide Wirksamkeit zeigt.

## Patentansprüche

1. Substituierte Triazolinone der allgemeinen Formel (I), 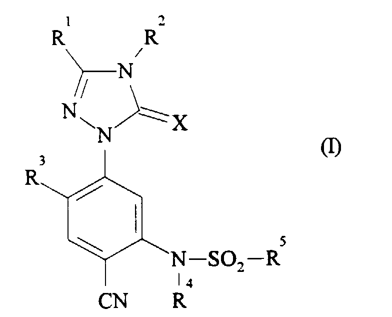 dadurch gekennzeichnet, daß
R1 für Wasserstoff, Halogen, Cyano, Hydroxy oder für einen der Reste -R⁶, -O-R⁶, -O-NR⁶R⁷, -S-R⁶, -S(O)-R⁶ oder -SO₂-R⁶ steht,
R² für Wasserstoff, Hydroxy, Amino, Cyano oder für einen der Reste -R⁶, -O-R⁶ oder -N=CR⁶R⁷ steht,
R³ für Wasserstoff, Halogen, Alkyl oder Halogenalkyl steht,
R⁴ für Wasserstoff, für einen der Reste -R⁶, -O-R⁶ oder -SO₂-R⁶ oder für ein anorganisches oder organisches Kation steht und
R⁵ für Amino, Hydroxy oder für einen der Reste -R⁶ oder -NR⁶R⁷ steht, oder
R⁴ und R⁵ gemeinsam für einen zweifach verknüpften Alkandiylrest stehen und
X für Sauerstoff oder Schwefel steht, wobei
R⁶ für jeweils gegebenenfalls substituiertes Alkyl, Alkenyl, Alkinyl, Cycloalkyl, Aryl oder Heterocyclyl steht und
R⁷ für Wasserstoff oder für jeweils gegebenenfalls substituiertes Alkyl, Alkenyl, Alkinyl, Cycloalkyl oder Aryl steht.

2. Substituierte Triazolinone der allgemeinen Formel (I) gemäß Anspruch 1, dadurch gekennzeichnet, daß
R¹ für Wasserstoff, Fluor, Chlor, Brom, Iod, Cyano, Hydroxy oder für einen der Reste -R⁶, -O-R⁶, -O-NR⁶R⁷, -S-R⁶, -S(O)-R⁶ oder -SO₂-R⁶ steht,
R² für Wasserstoff, Hydroxy, Amino, Cyano oder für einen der Reste -R⁶, -O-R⁶ oder -N=CR⁶R⁷ steht,
R³ für Wasserstoff, Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen - insbesondere Fluor, Chlor, Brom oder Iod - steht,
R⁴ für Wasserstoff, für einen der Reste -R⁶, -O-R⁶ oder -SO₂-R⁶, für ein Äquivalent eines Alkali- oder Erdalkalimetallkations oder für ein gegebenenfalls einfach oder mehrfach, gleich oder verschieden durch Alkyl mit 1 bis 16 Kohlenstoffatomen substituiertes Ammoniumkation steht und
R⁵ für Amino, Hydroxy oder für einen der Reste -R⁶ oder -NR⁶R⁷ steht, oder
R⁴ und R⁵ gemeinsam für einen zweifach verknüpften Alkandiylrest mit 2 bis 7 Kohlenstoffatomen stehen und
X für Sauerstoff oder Schwefel steht, wobei
R⁶ für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;
R⁶ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
R⁶ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
R⁶ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen und/oder 3-methylsubstituiertes 1,2,4-oxadiazol-2-yl substituiertes Phenyl;
R⁷ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;
R⁷ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
R⁷ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht, oder
R⁷ für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl.

3. Verfahren zur Herstellung der neuen substituierten Triazolinone der allgemeinen Formel (I), 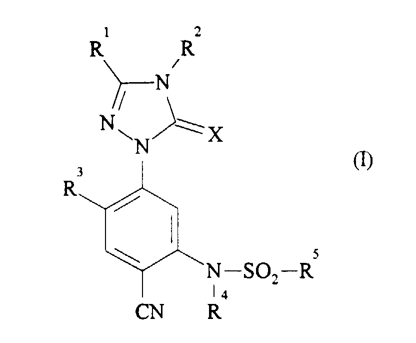 in welcher
R¹, R², R³, R⁴, R⁵ und X die in Anspruch 1 genannten Bedeutungen haben,
dadurch gekennzeichnet, daß man
a) 1H-Triazolinone der Formel (II), 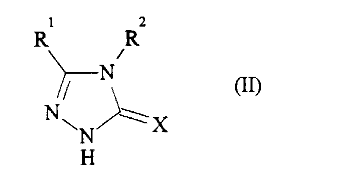 in welcher
R¹, R² und X die oben angegebenen Bedeutungen haben,
mit Halogenbenzol-Derivaten der Formel (III), 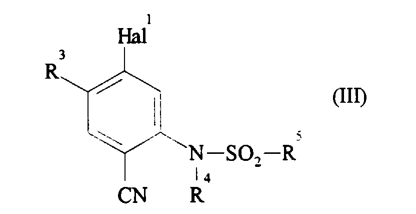 in welcher
R³, R⁴ und R⁵ die oben angegebenen Bedeutungen haben und
Hal¹ für Halogen, insbesondere Fluor, Chlor, Brom und Iod steht,
gegebenenfalls in Gegenwart eines Verdünnungsmittels und gegebebenfalls in Gegenwart eines Reaktionshilfsmittels umsetzt, oder daß man
b) substituierte Triazolinone der Formel (IV), 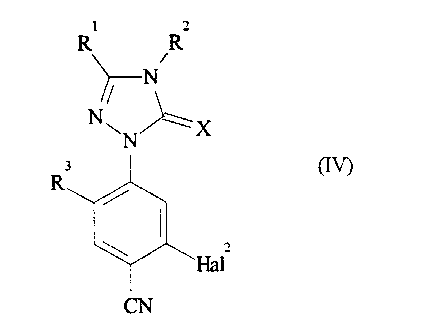 in welcher
R¹, R², R³ und X die oben angegebenen Bedeutungen haben und
Hal² für Halogen steht,
mit Sulfonamiden der Formel (V),
R⁴-NH-SO₂-R⁵ (V)
in welcher
R⁴ und R⁵ die oben angegebenen Bedeutungen haben, gegebenenfalls in Gegenwart eines Verdünnungsmittels und gegebenenfalls in Gegenwart eines Reaktionshilfsmittels umsetzt, oder daß man
c) substituierte Triazolinone der Formel (Ia), 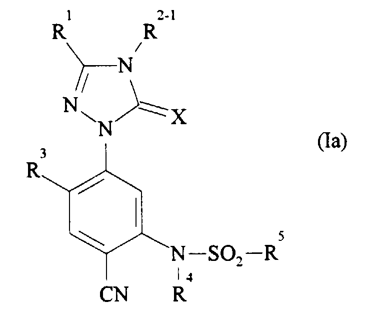 in welcher
R¹, R³, R⁴, R⁵ und X die oben angegebenen Bedeutungen haben und
R²⁻¹ für Amino steht,
mit Natriumnitrit in Gegenwart einer Säure und gegebenenfalls in Gegenwart eines Verdünnungsmittels umsetzt, oder daß man
d) substituierte Triazolinone der Formel (Ib), 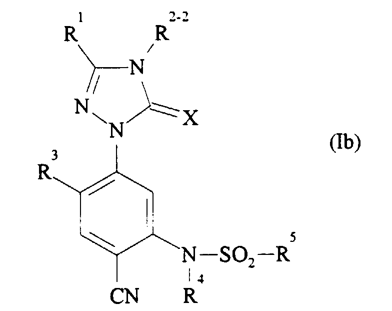 in welcher
R¹, R³, R⁴, R⁵ und X die oben angegebenen Bedeutungen haben und
R²⁻² für Wasserstoff steht,
mit Alkylierungsmitteln der Formel (VI),
R²⁻³-E¹ (VI)
in welcher
R²⁻³ für jeweils gegebenenfalls substituiertes Alkyl, Alkenyl, Alkinyl oder Cycloalkyl steht und
E¹ für eine elektronenanziehende Abgangsgruppe steht,
gegebenenfalls in Gegenwart eines Verdünnungsmittels und gegebenenfalls in Gegenwart eines Reaktionshilfsmittels umsetzt, oder daß man
e) substituierte Triazolinone der Formel (Ic), 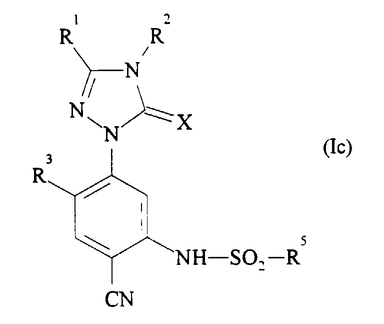 in welcher
R¹, R², R³, R⁵ und X die oben angegebenen Bedeutungen haben,
mit Alkylierungsmitteln der Formel (VII),
R⁴⁻¹-E² (VII)
in welcher
R⁴⁻¹ für Alkyl, Alkenyl, Alkinyl, Halogenalkyl, Halogenalkenyl, Halogenalkinyl, Alkoxyalkyl oder für gegebenenfalls substituiertes Cycloalkyl steht und
E² für eine elektronenanziehende Abgangsgruppe steht,
gegebenenfalls in Gegenwart eines Verdünnungsmittels und gegebenenfalls in Gegenwart eines Reaktionshilfsmittels umsetzt, oder wenn man
f) substituierte Triazolinone der Formel (VIII), 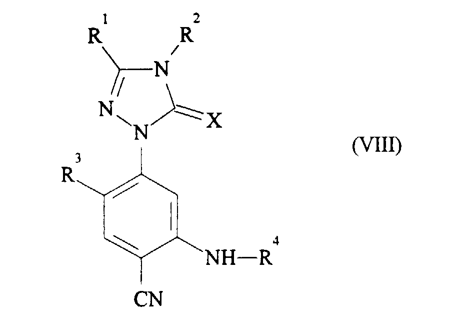 in welcher
R¹, R², R³, R⁴ und X die oben angegebenen Bedeutungen haben,
mit Sulfonsäurehalogeniden der Formel (IX),
R⁵-SO₂-Hal³ (IX)
in welcher
R⁵ die oben angegebene Bedeutung hat und
Hal³ für Halogen steht,
gegebenenfalls in Gegenwart eines Verdünnungsmittels und gegebenenfalls in Gegenwart eines Reaktionshilfsmittels umsetzt.

4. Herbizide Mittel, gekennzeichnet durch einen Gehalt an mindestens einem substituierten Triazolinon der Formel (I) gemäß den Ansprüchen 1 und 2.

5. Verfahren zur Bekämpfung von unerwünschten Pflanzen, dadurch gekennzeichnet, daß man substituierte Triazolinone der allgemeinen Formel (I) gemäß den Ansprüchen 1 und 2 auf die Pflanzen und/oder ihren Lebensraum einwirken läßt.

6. Verwendung von substituierten Triazolinonen der allgemeinen Formel (I) gemäß der Ansprüche 1 und 2 zur Bekämfpung von unerwünschten Pflanzen.

7. Verfahren zur Herstellung von herbiziden Mitteln, dadurch gekennzeichnet, daß man substituierte Triazolinone der allgemeinen Formel (I) gemäß der Ansprüche 1 und 2 mit Streckmitteln und/oder oberflächenaktiven Sustanzen vermischt.

8. Halogenbenzol-Derivate der Formel (III) 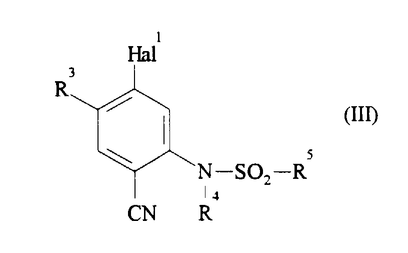 in welcher
R³ für Wasserstoff, Halogen, Alkyl oder Halogenalkyl steht,
R⁴ für Wasserstoff, für einen der Reste -R⁶, -O-R⁶ oder -SO₂-R⁶ oder für ein anorganisches oder organisches Kation steht und
R⁵ für Amino, Hydroxy oder für einen der Reste -R⁶ oder -NR⁶R⁷ steht, oder
R⁴ und R⁵ gemeinsam für einen zweifach verknüpften Alkandiylrest stehen und
R⁶ für jeweils gegebenenfalls substituiertes Alkyl, Alkenyl, Alkinyl, Cycloalkyl, Aryl oder Heterocyclyl steht und
R⁷ für Wasserstoff oder für jeweils gegebenenfalls substituiertes Alkyl, Alkenyl, Alkinyl, Cycloalkyl oder Aryl steht, und
Hal¹ für Halogen steht.

9. Substituierte Triazolinone der Formel (VIII), 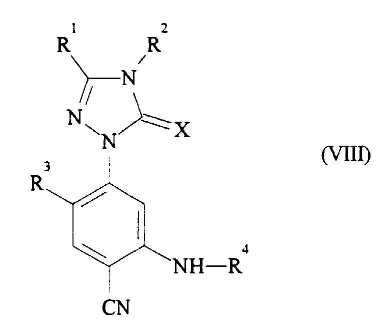
R¹ für Wasserstoff, Halogen, Cyano, Hydroxy oder für einen der Reste -R⁶, -O-R⁶, -O-NR⁶R⁷, -S-R⁶, -S(O)-R⁶ oder -SO₂-R⁶ steht, wobei R¹ jedoch nicht für Halogenalkyl steht,
R² für Wasserstoff, Hydroxy, Amino, Cyano oder für einen der Reste -R⁶, -O-R⁶ oder -N=CR⁶R⁷ steht,
R³ für Wasserstoff, Halogen, Alkyl oder Halogenalkyl steht,
R⁴ für Wasserstoff, für einen der Reste -R⁶, -O-R⁶ oder -SO₂-R⁶ oder für ein anorganisches oder organisches Kation steht und
X für Sauerstoff oder Schwefel steht, wobei
R⁶ für jeweils gegebenenfalls substituiertes Alkyl, Alkenyl, Alkinyl, Cycloalkyl, Aryl oder Heterocyclyl steht und
R⁷ für Wasserstoff oder für jeweils gegebenenfalls substituiertes Alkyl, Alkenyl, Alkinyl, Cycloalkyl oder Aryl steht.

## Claims

1. Substituted triazolinones of the general formula (I), 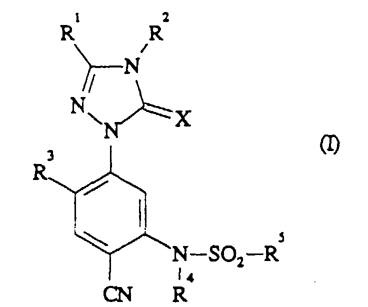 characterized in that
R¹ represents hydrogen, halogen, cyano, hydroxyl or one of the radicals -R⁶, -O-R⁶, -O-NR⁶R⁷, -S-R⁶, -S(O)-R⁶ or -SO₂-R⁶,
R² represents hydrogen, hydroxyl, amino, cyano or one of the radicals -R⁶, -O-R⁶ or -N=CR⁶R⁷,
R³ represents hydrogen, halogen, alkyl or halogenoalkyl,
R⁴ represents hydrogen, one of the radicals -R⁶, -O-R⁶ or -SO₂-R⁶ or an inorganic or organic cation and
R⁵ represents amino, hydroxyl or one of the radicals -R⁶ or -N⁶R⁷, or
R⁴ and R⁵ together represent a doubly attached alkanediyl radical and
X represents oxygen or sulphur, where
R⁶ represents alkyl, alkenyl, alkinyl, cycloalkyl, aryl or heterocyclyl, which may each optionally be substituted, and
R⁷ represents hydrogen or represents alkyl, alkenyl, alkinyl, cycloalkyl or aryl, which may each optionally be substituted.

2. Substituted triazolinones of the general formula (I) according to Claim 1, characterized in that
R¹ represents hydrogen, fluorine, chlorine, bromine, iodine, cyano, hydroxyl or one of the radicals -R⁶, -O-R⁶, -O-N⁶R⁷ *,* -S-R⁶, -S(O)-R⁶ or -SO₂-R⁶,
R² represents hydrogen, hydroxyl, amino, cyano or one of the radicals -R⁶, -O-R⁶ or -N=CR⁶R⁷,
R³ represents hydrogen, fluorine, chlorine, bromine, iodine, a straight chain or branched alkyl having from 1 to 8 carbon atoms or a straight chain or branched halogenoalkyl having from 1 to 8 carbon atoms and from 1 to 17 halogen atoms which may be identical or different - in particular fluorine, chlorine, bromine or iodine -,
R⁴ represents hydrogen, one of the radicals -R⁶, -O-R⁶ or -SO₂-R⁶, one equivalent of an alkali metal or alkaline earth metal cation, or an ammonium cation which may optionally be singly or multiply substituted by alkyl(s) having from 1 to 16 carbon atoms and which may be identical or different, and
R⁵ represents amino, hydroxyl or one of the radicals -R⁶ or -N⁶R⁷, or
R⁴ and R⁵ together represent a divalent alkanediyl radical having from 2 to 7 carbon atoms and
X represents oxygen or sulphur, where
R⁶ represents straight chain or branched alkyl having from 1 to 14 carbon atoms and which may optionally be singly or multiply substituted by substituents which may be identical or different, possible substituents being:
halogen - in particular fluorine, chlorine, bromine and/or iodine -, cyano, carboxyl, carbamoyl, or alkoxy, alkoxy-alkoxy, alkylthio, alkylsulphinyl, alkylsulphonyl, alkoxycarbonyl, N-alkylaminocarbonyl, N,N-dialkylaminocarbonyl, trialkylsilyl or alkylsulphonylaminocarbonyl which may each be straight chain or branched and each have from 1 to 8 carbon atoms in the individual alkyl moieties, or heterocyclyl, where the heterocyclyl radical is a from five- to seven-membered, optionally benzo-fused, saturated or unsaturated heterocycle having from 1 to 3 heteroatoms which may be identical or different - in particular nitrogen, oxygen and/or sulphur -;
R⁶ also represents alkenyl or alkinyl each having from 2 to 8 carbon atoms and which may each optionally be singly or multiply substituted by halogen which may be identical or different - in particular fluorine, chlorine, bromine and/or iodine-;
R⁶ also represents cycloalkyl having from 3 to 7 carbon atoms and which may optionally be singly or multiply substituted by halogen which may be identical or different - in particular fluorine, chlorine, bromine and/or iodine - and/or straight chain or branched alkyl having from 1 to 4 carbon atoms;
R⁶ also represents arylalkyl or aryl each having from 6 to 10 carbon atoms in the aryl part and optionally from 1 to 4 carbon atoms in the straight chain or branched alkyl part and which may each optionally be singly or multiply substituted in the aryl part by substituents which are identical or different, or a saturated or unsaturated, from five-to seven-membered heterocyclyl radical having from 1 to 3 heteroatoms which may be identical or different - in particular nitrogen, oxygen and/or sulphur - and which may optionally be singly or multiply substituted by substituents which may be identical or different, and/or be benzofused, possible aryl or heterocyclyl substituents in each case being:
halogen, cyano, nitro, amino, N-acetylamino, or alkyl, alkoxy, alkylthio, alkylsulphinyl or alkylsulphonyl which may each be straight chain or branched and each have from 1 to 6 carbon atoms, halogenoalkyl, halogenoalkoxy, halogenoalkylthio, halogenoalkylsulphinyl or halogenoalkylsulphonyl which may each be straight chain or branched and each have from 1 to 6 carbon atoms and from 1 to 13 halogen atoms which may be identical or different, alkoxycarbonyl or alkoximinoalkyl which may each be straight chain or branched and each have from 1 to 6 carbon atoms in the individual alkyl parts and phenyl which may optionally be singly or multiply substituted by substituents which may be identical or different and comprise halogen and/or straight chain or branched alkyl or alkoxy each having from 1 to 6 carbon atoms and/or straight chain or branched halogenoalkyl or halogenoalkoxy each having from 1 to 6 carbon atoms and from 1 to 13 halogen atoms which may be identical or different and/or 3-methyl-substituted 1,2,4-oxadiazol-2-yl;
R⁷ represents hydrogen or straight chain or branched alkyl having from 1 to 8 carbon atoms and which may optionally be singly or multiply substituted by substituents which may be identical or different, possible substituents being:
halogen - in particular fluorine, chlorine, bromine and/or iodine -, cyano, carboxyl, carbamoyl, or alkoxy, alkoxyalkoxy, alkylthio, alkylsulphinyl, alkylsulphonyl, alkoxycarbonyl, N-alkylaminocarbonyl, N,N-dialkylaminocarbonyl, trialkylsilyl or alkylsulphonylaminocarbonyl which may each be straight chain or branched and each have from 1 to 8 carbon atoms in the individual alkyl moieties, or heterocyclyl, where the heterocyclyl radical is a from five-to seven-membered, optionally benzo-fused, saturated or unsaturated heterocycle having from 1 to 3 heteroatoms which may be identical or different - in particular nitrogen, oxygen and/or sulphur -;
R⁷ also represents alkenyl or alkinyl each having from 2 to 8 carbon atoms and which may each optionally be singly or multiply substituted by halogen which may be identical or different - in particular fluorine, chlorine, bromine and/or iodine;
R⁷ also represents cycloalkyl having from 3 to 7 carbon atoms and which may optionally be singly or multiply substituted by halogen which may be identical or different - in particular fluorine, chlorine, bromine and/or iodine - and/or straight chain or branched alkyl having from 1 to 4 carbon atoms; or
R⁷ represents arylalkyl or aryl each having from 6 to 10 carbon atoms in the aryl part and optionally from 1 to 4 carbon atoms in the straight chain or branched alkyl part and which may each optionally be singly or multiply substituted in the aryl part by substituents which may be identical or different, possible aryl substituents in each case being:
halogen, cyano, nitro, or alkyl, alkoxy, alkylthio, alkylsulphinyl or alkylsulphonyl which may each be straight chain or branched and each have from 1 to 6 carbon atoms, halogenoalkyl, halogenoalkoxy, halogenoalkylthio, halogenoalkylsulphinyl or halogenoalkylsulphonyl which may each be straight chain or branched and each have from 1 to 6 carbon atoms and from 1 to 13 halogen atoms which may be identical or different, alkoxycarbonyl or alkoximinoalkyl which may each be straight chain or branched and each have from 1 to 6 carbon atoms in the individual alkyl parts and phenyl which may optionally be singly or multiply substituted by substituents which may be identical or different and comprise halogen and/or straight chain or branched alkyl or alkoxy each having from 1 to 6 carbon atoms and/or straight chain or branched halogenoalkyl or halogenoalkoxy each having from 1 to 6 carbon atoms and from 1 to 13 halogen atoms which may be identical or different.

3. Process for preparing the novel substituted triazolinones of the general formula (I), 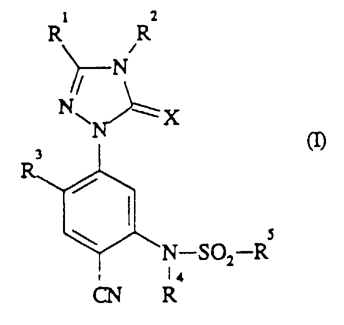 in which
R¹, R², R³, R⁴, R⁵ and X have the meanings given in Claim 1,
characterized in that
a) 1H-triazolinones of the formula (II), 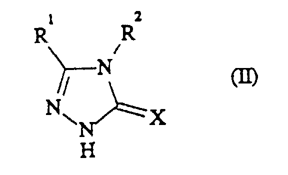 in which
R¹, R² and X have the above meanings,
are reacted with halogenobenzene derivatives of the formula (III), 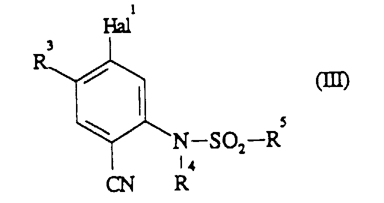 in which
R³, R⁴ and R⁵ have the above meanings and
Hal¹ represents halogen, in particular fluorine, chlorine, bromine and iodine,
optionally in the presence of a diluent and optionally in the presence of a reaction aid, or in that
b) substituted triazolinones of the formula (IV), 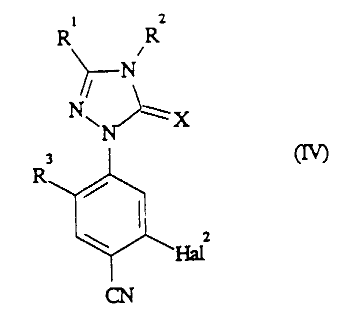 in which
R¹, R², R³ and X have the above meanings and
Hal² represents halogen,
are reacted with sulphonamides of the formula (V),
R⁴-NH-SO₂-R⁵ (V)
in which
R⁴ and R⁵ have the above meanings,
optionally in the presence of a diluent and optionally in the presence of a reaction aid, or in that
c) substituted triazolinones of the formula (Ia), 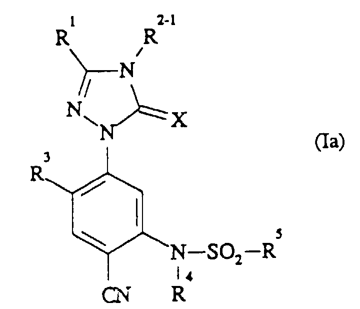 in which
R¹, R³, R⁴, R⁵ and X have the above meanings and
R²⁻¹ represents amino,
are reacted with sodium nitrite in the presence of an acid and optionally in the presence of a diluent, or in that
d) substituted triazolinones of the formula (Ib), 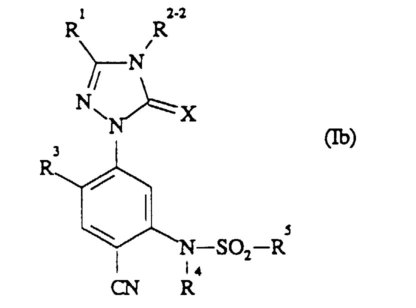 in which
R¹, R³, R⁴, R⁵ and X have the above meanings and
R²⁻² represents hydrogen,
are reacted with alkylating agents of the formula (VI),
R²⁻³-E¹ (VI)
in which
R²⁻³ represents alkyl, alkenyl, alkinyl or cycloalkyl, which may each optionally be substituted, and
E¹ represents an electron-attracting leaving group,
optionally in the presence of a diluent and optionally in the presence of a reaction aid, or in that
e) substituted triazolinones of the formula (Ic), 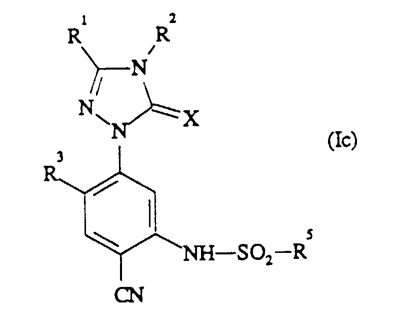 in which
R¹, R², R³, R⁵ and X have the above meanings,
are reacted with alkylating agents of the formula (VII),
R⁴⁻¹-E² (VII)
in which
R⁴⁻¹ represents alkyl, alkenyl, alkinyl, halogenoalkyl, halogenoalkenyl, halogenoalkinyl, alkoxyalkyl or optionally substituted cycloalkyl and
E² represents an electron-attracting leaving group,
optionally in the presence of a diluent and optionally in the presence of a reaction aid, or when
f) substituted triazolinones of the formula (VIII), 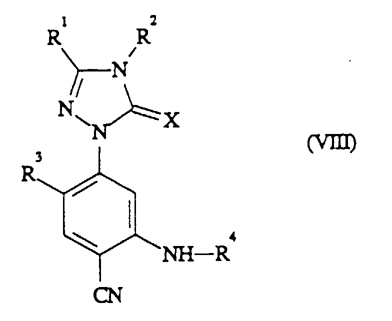 in which
R¹, R², R³, R⁴ and X have the above meanings,
are reacted with sulphonyl halides of the formula (IX),
R⁵-SO₂-Hal³ (IX)
in which
R⁵ has the above meaning and
Hal³ represents halogen,
optionally in the presence of a diluent and optionally in the presence of a reaction aid.

4. Herbicidal agents, characterized by a content of at least one substituted triazolinone of the formula (I) according to Claims 1 and 2.

5. Process for combating undesired plants, characterized in that substituted triazolinones of the general formula (I) according to Claims 1 and 2 are allowed to act on the plants and/or their habitat.

6. Use of substituted triazolinones of the general formula (I) according to Claims 1 and 2 for combating undesired plants.

7. Process for preparing herbicidal agents, characterized in that substituted triazolinones of the general formula (I) according to Claims 1 and 2 are mixed with extenders and/or surface-active substances.

8. Halogenobenzene derivatives of the formula (III) 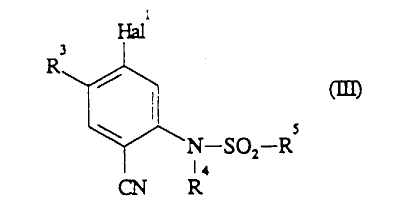 in which
R³ represents hydrogen, halogen, alkyl or halogenoalkyl,
R⁴ represents hydrogen, one of the radicals -R⁶, -O-R⁶ or -SO₂-R⁶ or an inorganic or organic cation and
R⁵ represents amino, hydroxyl or one of the radicals -R⁶ or -NR⁶R⁷, or
R⁴ and R⁵ together represent a divalent alkanediyl radical and
R⁶ represents alkyl, alkenyl, alkinyl, cycloalkyl, aryl or heterocyclyl, which may each optionally be substituted, and
R⁷ represents hydrogen or represents alkyl, alkenyl, alkinyl, cycloalkyl or aryl, which may each optionally be substituted, and
Hal¹ represents halogen.

9. Substituted triazolinones of the formula (VIII), 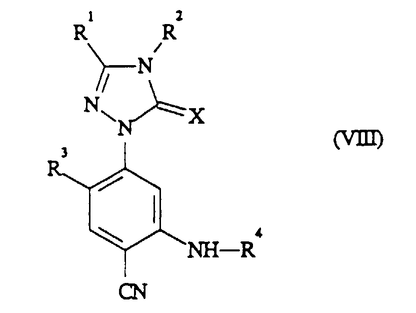 in which
R¹ represents hydrogen, halogen, cyano, hydroxyl or one of the radicals -R⁶, -O-R⁶, -O-NR⁶R⁷, -S-R⁶, -S(O)-R⁶ or -SO₂-R⁶,
but R¹ does not represent halogenoalkyl,
R² represents hydrogen, hydroxyl, amino, cyano or one of the radicals -R⁶, -O-R⁶ or -N=CR⁶R⁷,
R³ represents hydrogen, halogen, alkyl or halogenoalkyl,
R⁴ represents hydrogen, one of the radicals -R⁶, -O-R⁶ or -SO₂-R⁶ or an inorganic or organic cation and
X represents oxygen or sulphur, where
R⁶ represents alkyl, alkenyl, alkinyl, cycloalkyl, aryl or heterocyclyl, which may each optionally be substituted, and
R⁷ represents hydrogen or represents alkyl, alkenyl, alkinyl, cycloalkyl or aryl, which may each optionally be substituted.

## Revendications

1. Triazolinones substituées de formule générale (I) 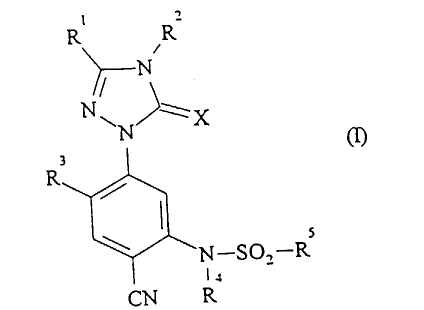 caractérisée en ce que
R¹ représente de l'hydrogène, un halogène, un groupe cyano, hydroxy ou l'un des restes -R⁶, -O-R⁶, -O-NR⁶R⁷, -S-R⁶, -S(O)-R⁶ ou -SO₂-R⁶,
R² est de l'hydrogène, un groupe hydroxy, amino, cyano ou l'un des restes -R⁶, -O-R⁶ ou -N=CR⁶R⁷,
R³ est de l'hydrogène, un halogène, un groupe alkyle ou halogénalkyle,
R⁴ est de l'hydrogène, l'un des restes -R⁶, -O-R⁶ ou -SO₂R⁶ ou représente un cation inorganique ou organique et
R⁵ est un groupe amino, hydroxy ou l'un des restes -R⁶ ou -NR⁶R⁷, ou bien
R⁴ et R⁵ forment ensemble un reste alcanediyle à deux liaisons et
X est de l'oxygène ou du soufre,
R⁶ représentant dans chaque cas un groupe alkyle, alcényle, alcynyle, cycloalkyle, aryle ou hétérocyclyle chacun éventuellement substitué et
R⁷ étant de l'hydrogène ou un groupe alkyle, alcényle, alcynyle, cycloalkyle ou aryle chacun éventuellement substitué.

2. Triazolinones substituées de formule générale (I) suivant la revendication 1, caractérisées en ce que
R¹ représente de l'hydrogène, du fluor, du chlore, du brome, de l'iode, un groupe cyano, hydroxy ou l'un des restes -R⁶, -O-R⁶, -O-NR⁶R⁷, -S-R⁶, -S(O)-R⁶ ou -SO₂-R⁶,
R² est de l'hydrogène, un groupe hydroxy, amino, cyano ou l'un des restes -R⁶, -O-R⁶ ou -N=CR⁶R⁷,
R³ représente de l'hydrogène, du fluor, du chlore, du brome, de l'iode, un groupe alkyle linéaire ou ramifié ayant 1 à 8 atomes de carbone ou un groupe halogénalkyle linéaire ou ramifié ayant 1 à 8 atomes de carbone et 1 à 17 atomes d'halogènes - notamment fluor, chlore, brome ou iode - identiques ou différents,
R⁴ représente de l'hydrogène, l'un des restes -R⁶, -O-R⁶ ou -SO₂R⁶, un équivalent d'un cation de métal alcalin ou alcalino-terreux ou un cation ammonium portant le cas échéant un ou plusieurs substituants, identiques ou différents, alkyle ayant 1 à 16 atomes de carbone et
R⁵ représente un groupe amino, hydroxy ou l'un des restes -R⁶ ou -NR⁶R⁷, ou bien
R⁴ et R⁵ forment ensemble un reste alcanediyle à deux liaisons ayant 2 à 7 atomes de carbone et
X représente de l'oxygène ou du soufre,
R⁶ représentant un groupe alkyle linéaire ou ramifié ayant 1 à 14 atomes de carbone, portant le cas échéant un ou plusieurs substituants identiques ou différents, en considérant comme substituants :
un halogène - notamment fluor, chlore, brome et/ou iode -, un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle, trialkylsilyle ou alkylsulfonylaminocarbonyle linéaire ou ramifié dans chaque cas, ayant chacun 1 à 8 atomes de carbone dans les parties alkyle individuelles, ou un reste hétérocyclyle, le reste hétérocyclyle étant un hétérocycle pentagonal à heptagonal saturé ou non saturé, éventuellement condensé au benzène, ayant 1 à 3 hétéroatomes - notamment azote, oxygène et/ou soufre - identiques ou différents ;
R⁶ représente en outre un groupe alcényle ou un groupe alcynyle ayant chacun 2 à 8 atomes de carbone et étant chacun éventuellement substitué une ou plusieurs fois, identiques ou différentes, par un halogène, notamment fluor, chlore, brome et/ou iode ;
R⁶ représente en outre un groupe cycloalkyle de 3 à 7 atomes de carbone portant le cas échéant un ou plusieurs substituants, identiques ou différents, halogéno - notamment fluoro, chloro, bromo et/ou iodo - et/ou alkyle linéaire ou ramifié ayant 1 à 4 atomes de carbone ;
R⁶ représente en outre un groupe arylalkyle ou un groupe aryle ayant chacun 6 à 10 atomes de carbone dans la partie aryle et le cas échéant 1 à 4 atomes de carbone dans la partie alkyle linéaire ou ramifiée, portant chacun le cas échéant dans la partie aryle un ou plusieurs substituants identiques ou différents, ou représente un reste hétérocyclyle pentagonal à heptagonal, saturé ou non saturé, éventuellement porteur d'un ou plusieurs substituants identiques ou différents et/ou condensé au benzène, ayant 1 à 3 hétéroatomes - notamment azote, oxygène et/ou soufre - identiques ou différents, en considérant comme substituants de chacun des restes aryle et hétérocyclyle : un halogène, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou un groupe alkoximinoalkyle étant chacun linéaire ou ramifié et ayant chacun 1 à 6 atomes de carbone dans les parties alkyle individuelles, ainsi qu'un groupe phényle à substituant 1,2,4-oxadiazole-2-yle portant le cas échéant un ou plusieurs substituants, identiques ou différents, halogéno et/ou alkyle ou alkoxy linéaire ou ramifié ayant chacun 1 à 6 atomes de carbone et/ou halogénalkyle ou halogénalkoxy linéaire ou ramifié ayant chacun 1 à 6 atomes de carbone et 1 à 13 atomes d'halogènes identiques ou différents et/ou portant un substituant 3-méthyle ;
R⁷ représente de l'hydrogène ou un groupe alkyle linéaire ou ramifié ayant 1 à 8 atomes de carbone, portant le cas échéant un ou plusieurs substituants identiques ou différents, en considérant comme substituants : un halogène - notamment fluor, chlore, brome et/ou iode -, un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle, trialkylsilyle ou alkylsulfonylaminocarbonyle étant chacun linéaire ou ramifié et ayant chacun 1 à 8 atomes de carbone dans les parties alkyle individuelles ou un reste hétérocyclyle, le reste hétérocyclyle étant un hétérocycle saturé ou non saturé pentagonal à heptagonal éventuellement condensé au benzène, ayant 1 à 3 hétéroatomes - notamment azote, oxygène et/ou soufre - identiques ou différents ;
R⁷ représente en outre un groupe alcényle ou un groupe alcynyle ayant chacun 2 à 8 atomes de carbone et étant substitués chacun une ou plusieurs fois identiques ou différentes par un halogène, notamment fluor, chlore, brome et/ou iode,
R⁷ représente en outre un groupe cycloalkyle de 3 à 7 atomes de carbone portant le cas échéant un ou plusieurs substituants, identiques ou différents, halogéno - notamment fluoro, chloro, bromo et/ou iodo - et/ou alkyle linéaire ou ramifié ayant 1 à 4 atomes de carbone, ou bien
R⁷ représente un groupe arylalkyle ou aryle portant chacun le cas échéant dans la partie aryle un ou plusieurs substituants identiques ou différents et ayant chacun 6 à 10 atomes de carbone dans la partie aryle et le cas échéant 1 à 4 atomes de carbone dans la partie alkyle linéaire ou ramifiée, en considérant dans chaque cas comme substituants de la partie aryle : un halogène, un groupe cyano, nitro, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou alkoximinoalkyle étant chacun linéaire ou ramifié et ayant chacun 1 à 6 atomes de carbone dans les parties alkyle individuelles ainsi qu'un groupe phényle portant le cas échéant un ou plusieurs substituants, identiques ou différents, halogéno et/ou alkyle ou alkoxy linéaire ou ramifié ayant chacun 1 à 6 atomes de carbone et/ou halogénalkyle ou halogénalkoxy linéaire ou ramifié ayant chacun 1 à 6 atomes de carbone et 1 à 13 atomes d'halogènes identiques ou différents.

3. Procédé de production des triazolinones substituées nouvelles de formule générale (I) 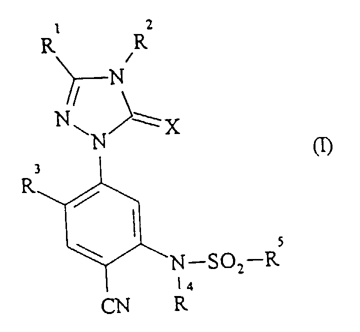 dans laquelle
R¹, R², R³, R⁴, R⁵ et X ont les définitions indiquées ci-dessus,
caractérisé en ce que
a) on fait réagir des lH-triazolinones de formule (II) 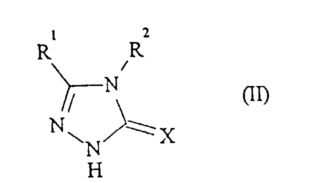 dans laquelle
R¹, R² et X ont les définitions indiquées ci-dessus, avec des dérivés halogénobenzéniques de formule (III) 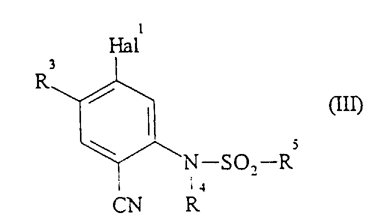 dans laquelle
R³, R⁴ et R⁵ ont les définitions indiquées ci-dessus et
Hal¹ représente un halogène, notamment le fluor, le chlore, le brome et l'iode,
le cas échéant en présence d'un diluant et en présence éventuelle d'une substance auxiliaire de réaction, ou bien
b) on fait réagir des triazolinones substituées de formule (IV) 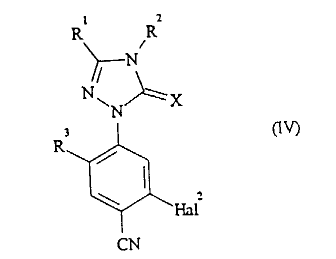 dans laquelle
R¹, R², R³ et X ont les définitions indiquées ci-dessus et
Hal² représente un halogène,
avec des sulfonamides de formule (V)
R⁴-NH-SO₂-R⁵ (V)
dans laquelle
R⁴ et R⁵ ont les définitions indiquées ci-dessus, le cas échéant en présence d'un diluant et en la présence éventuelle d'une substance auxiliaire de réaction, ou bien
c) on fait réagir des triazolinones substituées de formule (Ia), 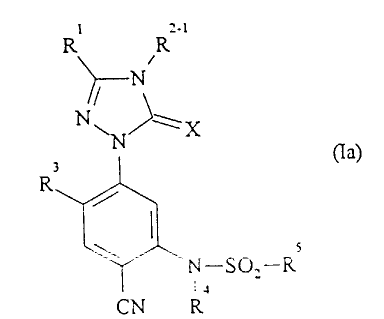 dans laquelle
R¹, R³, R⁴, R⁵ et X ont les définitions indiquées ci-dessus et
R²⁻¹ est un groupe amino,
avec le nitrite de sodium en présence d'acide et en la présence éventuelle d'un diluant, ou bien
d) on fait réagir des triazolinones substituées de formule (Ib) 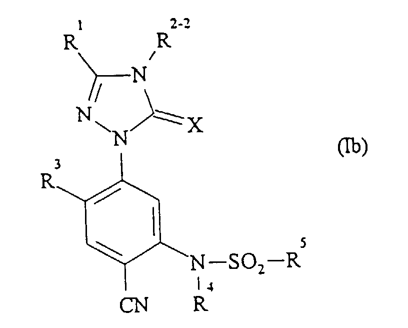 dans laquelle
R¹, R³, R⁴, R⁵ et X ont les définitions indiquées ci-dessus et
R²⁻² est de l'hydrogène,
avec des agents d'alkylation de formule (VI),
R²⁻³-E¹ (VI)
dans laquelle
R²⁻³ est un groupe alkyle, alcényle, alcynyle ou cycloalkyle dont chacun est éventuellement substitué et
E¹ est un groupe partant attirant les électrons,
le cas échéant en présence d'un diluant et en la présence éventuelle d'une substance auxiliaire de réaction, ou bien
e) on fait réagir des triazolinones substituées de formule (Ic) 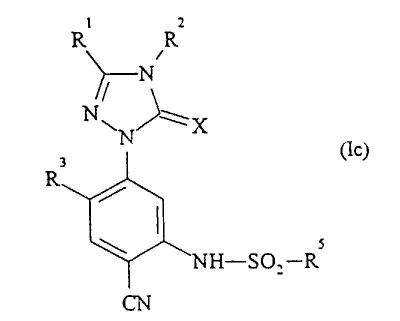 dans laquelle
R¹, R², R³, R⁵ et X ont les définitions indiquées ci-dessus, avec des agents d'alkylation de formule (VII)
R⁴⁻¹-E² (VII)
dans laquelle
R⁴⁻¹ est un groupe alkyle, alcényle, alcynyle, halogénalkyle, halogénalcényle, halogénalcynyle, alkoxyalkyle ou un groupe cycloalkyle éventuellement substitué et
E² est un groupe partant attirant les électrons,
le cas échéant en présence d'un diluant et la présence éventuelle d'une substance auxiliaire de réaction, ou bien
f) on fait réagir des triazolinones substituées de formule (VIII) 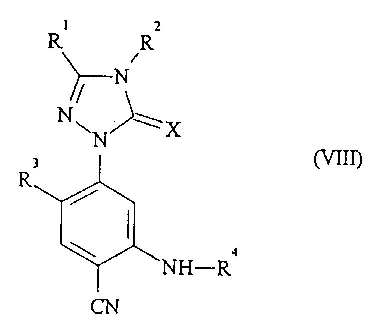 dans laquelle
R¹, R², R³, R⁴ et X ont les définitions indiquées ci-dessus, avec des halogénures d'acide sulfonique de formule (IX)
R⁵-SO₂-Hal³ (IX)
dans laquelle
R⁵ a la définition indiquée ci-dessus,
Hal³ est un halogène,
le cas échéant en présence d'un diluant et en la présence éventuelle d'une substance auxiliaire de réaction.

4. Composition herbicides, caractérisées par une teneur en au moins une triazolinone substituée de formule (I) suivant les revendications 1 et 2.

5. Procédé pour combattre des plantes indésirables, caractérisé en ce qu'on fait agir sur les plantes et/ou sur leur milieu des triazolinones substituées de formule générale (I) suivant les revendications 1 et 2.

6. Utilisation de triazolinones substituées de formule générale (I) suivant les revendications 1 et 2 pour combattre des plantes indésirables.

7. Procédé de préparation de compositions herbicides, caractérisé en ce qu'on mélange des triazolinones substituées de formule générale (I) suivant les revendications 1 et 2 avec des diluants et/ou des substances tensio-actives.

8. Dérivés halogénobenzéniques de formule (III) 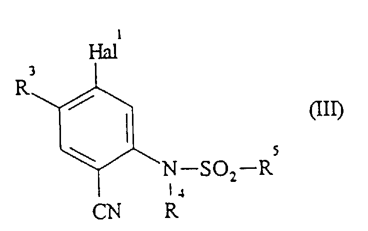 dans laquelle
R³ représente de l'hydrogène, un halogène, un groupe alkyle ou halogénalkyle,
R⁴ est de l'hydrogène, l'un des restes -R⁶, -O-R⁶ ou -SO₂-R⁶ ou un cation inorganique ou organique et
R⁵ est un groupe amino, hydroxy ou l'un des restes -R⁶ ou -NR⁶R⁷, ou bien
R⁴ et R⁵ forment ensemble un reste alcane-diyle à deux liaisons et
R⁶ est un groupe alkyle, alcényle, alcynyle, cycloalkyle, aryle ou hétérocyclyle dont chacun est éventuellement substitué
R⁷ représente de l'hydrogène ou un groupe alkyle, alcényle, alcynyle, cycloalkyle ou aryle dont chacun est éventuellement substitué et
Hal¹ est un halogène.

9. Triazolinones substituées de formule (VIII) 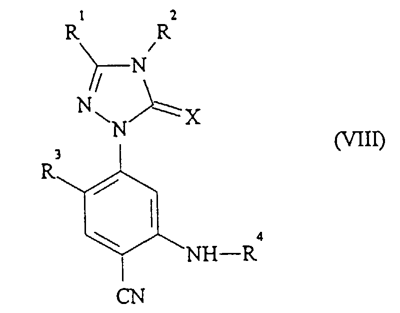 dans laquelle
R¹ représente de l'hydrogène, un halogène, un groupe cyano, hydroxy ou l'un des restes -R⁶, -O-R⁶, -O-NR⁶R⁷, -S-R⁶, -S(O)-R⁶ ou -SO₂-R⁶, R¹ ne pouvant toutefois pas représenter un groupe halogénalkyle,
R² est de l'hydrogène, un groupe hydroxy, amino, cyano ou l'un des restes -R⁶, -O-R⁶ ou -N=CR⁶R⁷,
R³ est de l'hydrogène, un halogène, un groupe alkyle ou halogénalkyle,
R⁴ est de l'hydrogène, l'un des restes -R⁶, -O-R⁶ ou -SO₂R⁶ ou un cation inorganique ou organique et
X est de l'oxygène ou du soufre,
R⁶ représentant un groupe alkyle, alcényle, alcynyle, cycloalkyle, aryle ou hétérocyclyle dont chacun est éventuellement substitué et
R⁷ représente de l'hydrogène ou un groupe alkyle, alcényle, alcynyle, cycloalkyle ou aryle dont chacun est éventuellement substitué.
